# EUROPÄISCHE PATENTSCHRIFT

(11) **EP 2 373 619 B1**
(45) Veröffentlichungstag und Bekanntmachung des Hinweises auf die Patenterteilung: **10.09.2014**
(21) Anmeldenummer: 09756441.3
(22) Anmeldetag: 20.11.2009
(51) Int. Cl.: C07D 209/54, A01N 43/38

(54) **GEMINAL ALKOXY/ALKYLSPIROCYCLISCH-SUBSTITUIERTE TETRAMSÄURE-DERIVATE**
GEMINAL ALKOXY/ALKYLSPIROCYCLIC SUBSTITUTED TETRAMIC ACID DERIVATES
DÉRIVÉS D'ACIDES TÉTRAMIQUES À SUBSTITUTION ALCOXY/ALKYLSPIROCYCLIQUE GÉMINALE

(30) Priorität: 02.12.2008 EP 08170489
(43) Veröffentlichungstag der Anmeldung: 12.10.2011
(73) Patentinhaber: BAYER CROPSCIENCE AG, 40789 Monheim (DE)
(72) Erfinder: FISCHER, Reiner, 40789 Monhein (DE); LEHR, Stefan, 65835 Liederbach (DE); DITTGEN, Jan, 60316 Frankfurt (DE); FEUCHT, Dieter, 65760 Eschborn (DE); GÖRGENS, Ulrich, 40882 Ratingen (DE); HÄUSER-HAHN, Isolde, 51375 Leverkusen (DE); ROSINGER, Christopher, Hugh, 65719 Hofheim (DE); VOERSTE, Arnd, 50674 Köln (DE); FRANKEN, Eva-Maria, 51381 Leverkusen (DE); MALSAM, Olga, 51503 Rösrath (DE)
(74) Vertreter: BIP Patents
(86) Internationale Anmeldenummer: PCT/EP2009/008260
(87) Internationale Veröffentlichungsnummer: WO 2010/063378

(56) Entgegenhaltungen:
- WO-A-2006/000355

## Beschreibung

Die vorliegende Erfindung betrifft neue geminal Alkoxy/Alkylspirocyclisch-substituierte Tetramsäure-Derivate, mehrere Verfahren zu ihrer Herstellung und ihre Verwendung als Schädlingsbekämpfungsmittel und/oder Herbizide. Auch Gegenstand der Erfindung sind selektiv herbizide Mittel, die geminal Alkoxy/Alkylspirocyclisch-substituierte Tetramsäure-Derivate einerseits und eine die Kulturpflanzenverträglichkeit verbessernde Verbindung andererseits enthalten.

Die vorliegende Erfindung betrifft weiterhin die Steigerung der Wirkung von Pflanzenschutzmitteln enthaltend insbesondere geminal Alkoxy/Alkylspirocyclisch-substituierte Tetramsäure-Derivate, durch die Zugabe von Ammonium- oder Phosphoniumsalzen und gegebenenfalls Penetrationsförderern, die entsprechenden Mittel, Verfahren zur ihrer Herstellung und ihre Anwendung im Pflanzenschutz als Insektizide und/oder Akarizide und/oder zur Verhinderung von unerwünschtem Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyr-rolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 95/01 971, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869 und WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049569, WO 05/066125, WO 05/092897, WO 06/000355, WO 06/029799, WO 06/056281, WO 06/056282, WO 06/089633, WO 07/048545, DEA 102 00505 9892, WO 07/073856, WO 07/096058, WO 07/121868, WO 07/140881, WO 08/067873, WO 08/067910, WO 08/067911, WO 08/138551, WO 09/015801, WO 09/039975, WO 09/049581). Außerdem sind ketalsubstituierte 1-H-Arylpyrrolidin-2,4-dione aus WO 99/16748 und (spiro)-ketalsubstituierte N-Alkoxy-alkoxy-substituierte Aryl-pyrrolidindione aus JP-A-14 205 984 und Ito M. et al.. Bioscience, Biotechnology and Biochemistry 67, 1230-1238, (2003) bekannt. Der Zusatz von Safenern zu Ketoenolen ist ebenfalls prinzipiell aus der WO 03/013249 bekannt. Außerdem sind aus WO 06/024411 herbizide Mittel enthaltend Ketoenole bekannt.

Die herbizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (**I**) 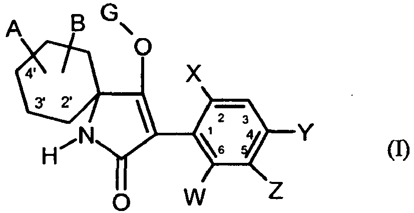 gefunden,
in welcher
- W: für Wasserstoff, Halogen, Alkyl, Alkenyl, Alkinyl, gegebenenfalls substituiertes Cycloalkyl, Alkoxy, Alkenyloxy, Halogenalkyl, Halogenalkoxy oder Cyano steht,
- X: für Halogen, Alkyl, Alkenyl, Alkinyl, gegebenenfalls substituiertes Cycloalkyl, Alkoxy, Alkenyloxy, Alkylthio, Alkylsulfonyl, Alkylsulfonyl, Halogenalkyl, Halogenalkoxy, Halogenalkenyloxy, Nitro oder Cyano steht,
- Y und Z: unabhängig voneinander für Wasserstoff, Alkyl, Alkenyl, Alkinyl, gegebenenfalls substituiertes Cycloalkyl, Alkoxy, Halogen, Halogenalkyl, Halogenalkoxy, Cyano, Nitro oder jeweils gegebenenfalls substituiertes Aryl oder Hetaryl stehen,
- A: für Alkoxy steht,
- B: für Alkyl steht, wobei
- A und B: an das selbe Kohlenstoffatom gebunden sind,
- G: für Wasserstoff (a) oder für eine der Gruppen 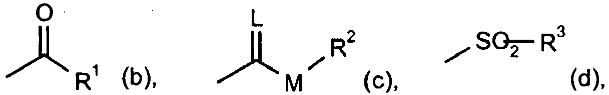 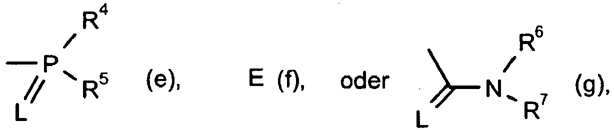
steht,
worin
- E: für ein Metallion oder ein Ammoniumion steht,
- L: für Sauerstoff oder Schwefel steht,
- M: für Sauerstoff oder Schwefel steht,
- R¹: für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der verschiedenen Bedeutungen (a), (b), (c), (d), (e), (f) und (g) der Gruppe G ergeben sich folgende hauptsächliche Strukturen (I-a) bis (I-g), 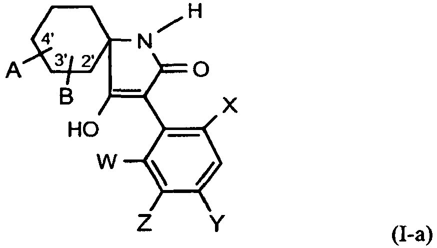 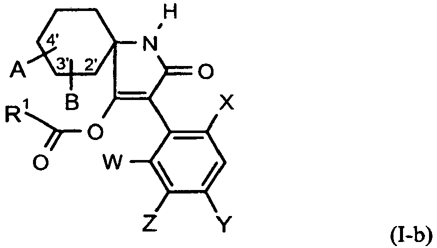 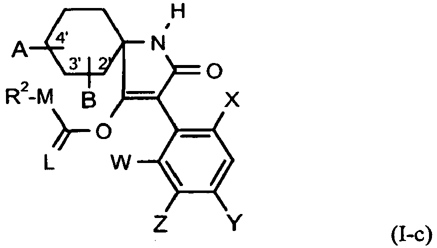 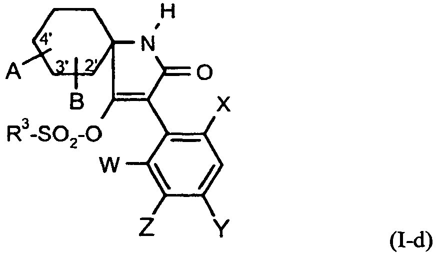 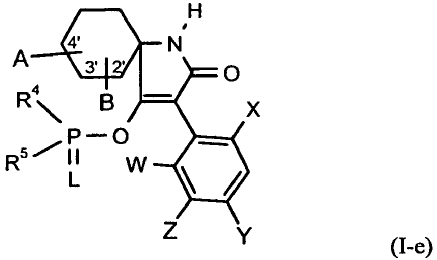 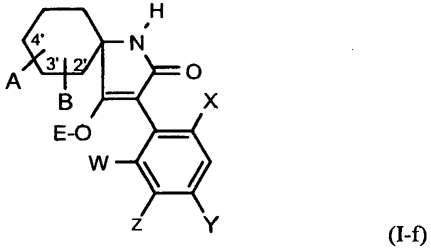 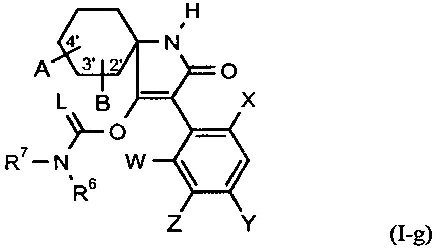 worin
A, B, E, L, M, W, X, Y, Z, R¹, R², R³, R⁴, R⁵, R⁶ und R⁷ die oben angegebenen Bedeutungen besitzen.

Weiterhin wurde gefunden, dass man die neuen Verbindungen der Formel (I) nach den im Folgenden beschriebenen Verfahren erhält:
(A) Man erhält Verbindungen der Formel (I-a) 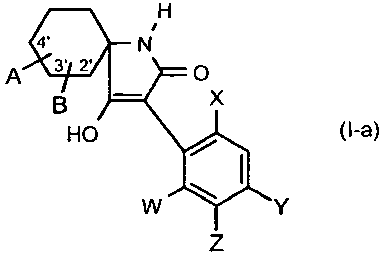 in welcher
   A, B, W, X, Y und Z die oben angegebenen Bedeutungen haben,
   wenn man
   Verbindungen der Formel (II) 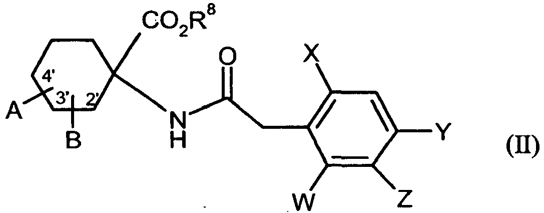 in welcher
   - A, B, W, X, Y und Z: die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert. Außerdem wurden gefunden
(B) dass man die Verbindungen der oben gezeigten Formeln (I-b), in welchen R¹, A, B, W, X, Y und Z die oben angegebenen Bedeutungen haben, erhält, wenn man Verbindungen der oben gezeigten Formeln (I-a), in welchen A, B, W, X, Y und Z die oben angegebenen Bedeutungen haben, jeweils
α) mit Verbindungen der Formel (III) 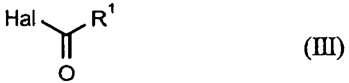 in welcher
   - R¹: die oben angegebene Bedeutung hat und
   - Hal: für Halogen (insbesondere Chlor oder Brom) steht
   oder
   - β): mit Carbonsäureanhydriden der Formel (IV)

   R¹-CO-O-CO-R¹ (IV)

   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(C) dass man die Verbindungen der oben gezeigten Formeln (I-c), in welchen R², A, B, M, W, X, Y und Z die oben angegebenen Bedeutungen haben und L für Sauerstoff steht, erhält, wenn man Verbindungen der oben gezeigten Formeln (I-a), in welchen A, B, W, X, Y und Z die oben angegebenen Bedeutungen haben, jeweils
   mit Chlorameisensäureestern oder Chlorameisensäurethioestern der Formel (V)

   R²-M-CO-Cl (V)

   in welcher
   R² und M die oben angegebenen Bedeutungen haben,
   gegebenenfalls in Gegenwart eines Verdünnungsmittels und gegebenenfalls in Gegenwart eines Säurebindemittels umsetzt;
(D) dass man Verbindungen der oben gezeigten Formeln (I-c), in welchen R², A, B, M, W, X, Y und Z die oben angegebenen Bedeutungen haben und L für Schwefel steht, erhält, wenn man Verbindungen der oben gezeigten Formeln (I-a), in welchen A, B, W, X, Y und Z die oben angegebenen Bedeutungen haben, jeweils
   mit Chlormonothioameisensäureestern oder Chlordithioameisensäureestern der Formel (VII) 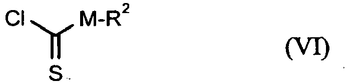 in welcher
   M und R² die oben angegebenen Bedeutungen haben,
   gegebenenfalls in Gegenwart eines Verdünnungsmittels und gegebenenfalls in Gegenwart eines Säurebindemittels umsetzt,
(E) dass man Verbindungen der oben gezeigten Formeln (I-d), in welchen R³, A, B, W, X, Y und Z die oben angegebenen Bedeutungen haben, erhält, wenn man Verbindungen der oben gezeigten Formeln (I-a), in welchen A, B, W, X, Y und Z die oben angegebenen Bedeutungen haben, jeweils
   mit Sulfonsäurechloriden der Formel (VII)

   R³-SO₂-Cl (VII)

   in welcher
   R³ die oben angegebene Bedeutung hat,
   gegebenenfalls in Gegenwart eines Verdünnungsmittels und gegebenenfalls in Gegenwart eines Säurebindemittels umsetzt,
(F) dass man Verbindungen der oben gezeigten Formeln (I-e), in welchen L, R⁴, R⁵, A, B, W, X, Y und Z die oben angegebenen Bedeutungen haben, erhält, wenn man Verbindungen der oben gezeigten Formeln (I-a), in welchen A, B, W, X, Y und Z die oben angegebenen Bedeutungen haben, jeweils mit Phosphorverbindungen der Formel (VIII) 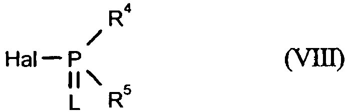 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(G) dass man Verbindungen der oben gezeigten Formeln (I-f), in welchen E, A, B, W, X, Y und Z die oben angegebenen Bedeutungen haben, erhält, wenn man Verbindungen der Formeln (I-a), in welchen A, B, W, X, Y und Z die oben angegebenen Bedeutungen haben, jeweils
   mit Metallverbindungen oder Aminen der Formeln (IX) oder (X)

   Me(OR¹⁰)ₜ (IX)

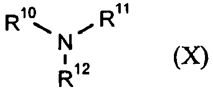

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(H) dass man Verbindungen der oben gezeigten Formeln (I-g), in welchen L, R⁶, R⁷, A, B, W, X, Y und Z die oben angegebenen Bedeutungen haben, erhält, wenn man Verbindungen der oben gezeigten Formeln (I-a), in welchen A, B, W, X, Y und Z die oben angegebenen Bedeutungen haben, jeweils
α) mit Isocyanaten oder Isothiocyanaten der Formel (XI)

   R⁶-N=C=L (XI)

   in welcher
   - R⁶ und L: die oben angegebenen Bedeutungen haben,
   gegebenenfalls in Gegenwart eines Verdünnungsmittels und gegebenenfalls in Gegenwart eines Katalysators umsetzt oder
β) mit Carbamidsäurechloriden oder Thiocarbamidsäurechloriden der Formel (XII) 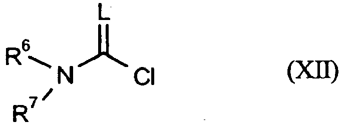 in welcher
   - L, R⁶ und R⁷: die oben angegebenen Bedeutungen haben,
   gegebenenfalls in Gegenwart eines Verdünnungsmittels und gegebenenfalls in Gegenwart eines Säurebindemittels, umsetzt,
(Iα) dass man Verbindungen der oben gezeigten Formeln (I-a) bis (I-g), in welchen A, B, G, W, X, Y und Z die oben angegebene Bedeutung haben, erhält, wenn man Verbindungen der Formeln (I-a') bis (I-g'), in welchen A, B, G, W, X und Y die oben genannte Bedeutung haben und Z' bevorzugt für Brom oder Iod steht 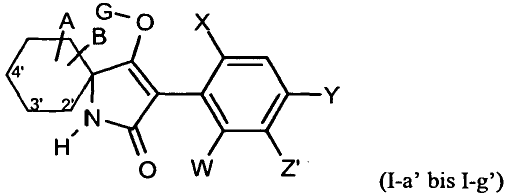 und
(Iβ) dass man Verbindungen der oben gezeigten Formeln (I-a) bis (I-g), in welchen A, B, G, W, X, Y und Z die oben angegebene Bedeutung haben, erhält, wenn man Verbindungen der Formeln (I-a") bis (I-g"), in welchen A, B, G, W, X und Z die oben genannte Bedeutung haben und Y' bevorzugt für Brom oder Iod steht 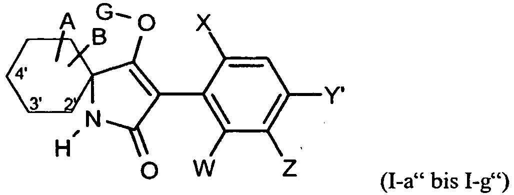 mit kupplungsfähigen (Het)-arylderivaten, z.B. Phenylboronsäuren der Formeln (XIIIα) und (XIIIβ) 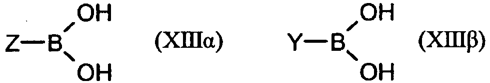 oder deren Ester in Gegenwart eines Lösungsmittels, in Gegenwart eines Katalysators (z. B. Pd-Komplexe) und in Gegenwart einer Base (z.B. Natriumcarbonat, Kaliumphosphat) kuppelt.

Weiterhin wurde gefunden, dass die neuen Verbindungen der Formel (I) eine sehr gute Wirksamkeit als Schädlingsbekämpfungsmittel, vorzugsweise als Insektizide, Akarizide und Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenem/Antidots) ausgesprochen gut die Schädigung der Kulturpflanzen verhindert und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
a') mindestens eine Verbindung der Formel (I), in welcher A, B, G, W, X, Y und Z die oben angegebene Bedeutung haben
   und
(b') zumindest eine die Kulturpflanzen-Verträglichkeit verbessernde Verbindung (Safener).

Die Safener sind vorzugsweise ausgewählt aus der Gruppe bestehend aus:
S1) Verbindungen der Formel (S1), 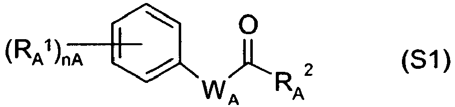 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴),

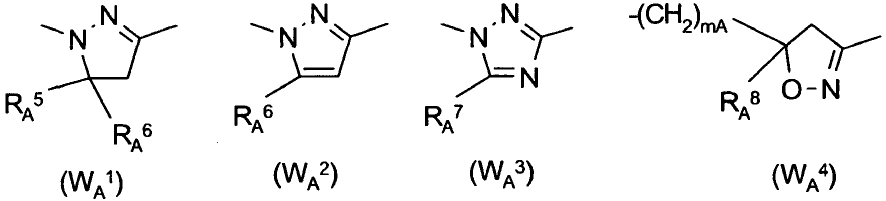

   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alk_{Y}1, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubstituiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1 -dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 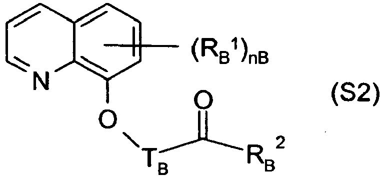 wobei die Symbole und Indizes folgende Bedeutungen haben:
   - R_{B}¹: ist Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: ist OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminum-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolin-oxy)malonsäure-methyl-ethylester und verwandte Verbindungen, wie sie in EP-A-0 582 198 beschrieben sind.
S3) Verbindungen der Formel (S3) 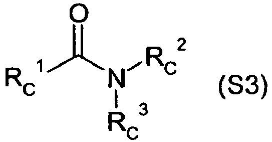 wobei die Symbole und Indizes folgende Bedeutungen haben:
   - R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{C}², R_{C}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin-oder Benzoxazinring;
   vorzugsweise:
   Wirkstoffe vom Typ der Dichloracetamide, die häufig als Vorauflaufsafener (bodenwirksame Safener) angewendet werden, wie z. B. "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9) (3-Dichloracetyl-2,5,5-trimethyl-1,3-diazabicyclo[4.3.0]nonan) der Firma BASF, "Furilazol" oder "MON 13900" ((RS)-3-Dichloracetyl-5-(2-furyl)-2,2-dimethyl-oxazolidin) (S3-10); sowie dessen (R)-Isomer (S3-11).
S4) N-Acylsulfonamide der Formel (S4) und ihre Salze, 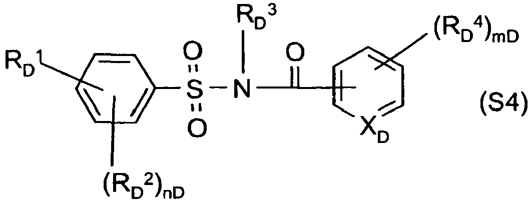 worin die Symbole und Indizes folgende Bedeutungen haben:
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   - R_{D}³: ist Wasserstoff, (C₁-C₄)Alkyl, (C₂-C₄)Alkenyl oder (C₂-C₄)Alkinyl;
   - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   - R_{d}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   - R_{D}⁶: ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   - R_{D}⁵ und R_{D}⁶: gemeinsam mit dem dem sie tragenden Stickstoffatom einen Pyrrolidinyl- oder Piperidinyl-Rest bilden;
   - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - n_{D}: ist 0, 1 oder 2;
   - m_{D}: ist 1 oder 2;
   - v_{D}: ist 0, 1, 2 oder 3;
   davon bevorzugt sind Verbindungen von Typ der N-Acylsulfonamide, z.B. der nachfolgenden Formel (S4^{a}), die z. B. bekannt sind aus WO-A-97/45016 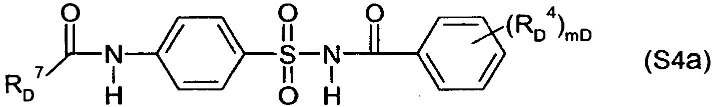 worin
   - R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - R_{D}⁴: Halogen, (C₁-C₄)Alkyl, (C₁-C₄)Alkoxy, CF₃;
   - m_{D}: 1 oder 2;
   - v_{D}: ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 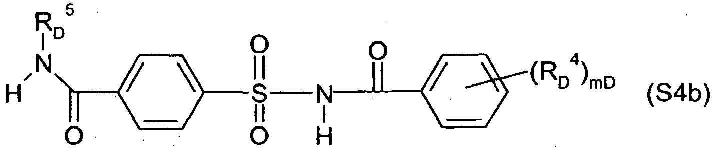 z.B. solche worin
   R_{D}⁵ = Cyclopropyl und (R_{D}⁴) = 2-OMe ist ("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5)
   sowie
   Verbindungen vom Typ der N-Acylsulfamoylphenylharnstoffe der Formel (S4^{c}), die z.B. bekannt sind aus der EP-A-365484, 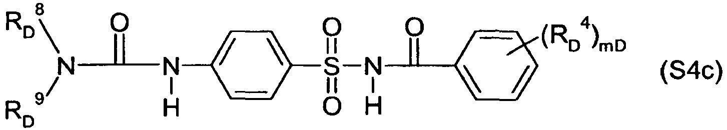 worin
   - R_{D}⁸ und R_{D}⁹: unabhängig voneinander Wasserstoff, (C₁-C₈)Alkyl, (C₃-C₈)Cycloalkyl, (C₃-C₆)Alkenyl, (C₃-C₆)Alkinyl,
   - R_{D}⁴: Halogen, (C₁-C₄)Alkyl, (C₁-C₄)Alkoxy, CF₃
   - m_{D}: 1 oder 2 bedeutet;
   beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 1,2-Dihydro-2-oxo-6-trifluoromethylpyridin-3-carboxamid, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-[2-{Diethylamino)ethyl]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 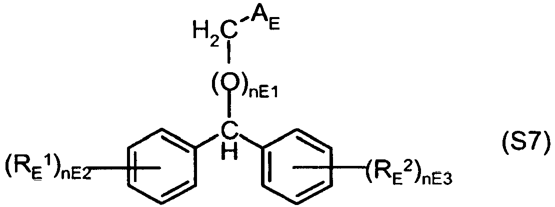 worin die Symbole und Indizes folgende Bedeutungen haben:
   - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{E}: ist COOR_{E}³ oder COSR_{E}⁴
   - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{E}¹: ist 0 oder 1
   - n_{E}², n_{E}³: sind unabhängig voneinander 0, 1 oder 2,
   vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1).
S8) Verbindungen der Formel (S8),wie sie in der WO-A-98/27049 beschrieben sind 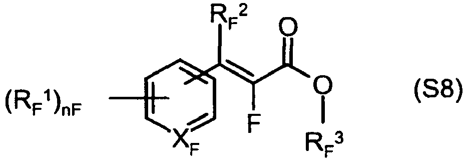 worin
   - X_{F}: CH oder N,
   - n_{F}: für den Fall, dass X_{F}=N ist, eine ganze Zahl von 0 bis 4 und für den Fall, dass X_{F}=CH ist, eine ganze Zahl von 0 bis 5 ,
   - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   - R_{F}²: Wasserstoff oder (C₁-C₄)Alkyl
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
   vorzugsweise Verbindungen worin
   - X_{F}: CH,
   - n_{F}: eine ganze Zahl von 0 bis 2 ,
   - R_{F}¹: Halogen, (C₁-C₄)Alkyl, (C₁-C₄)Haloalkyl, (C₁-C₄)Alkoxy, (C₁-C₄)Haloalkoxy,
   - R_{F}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 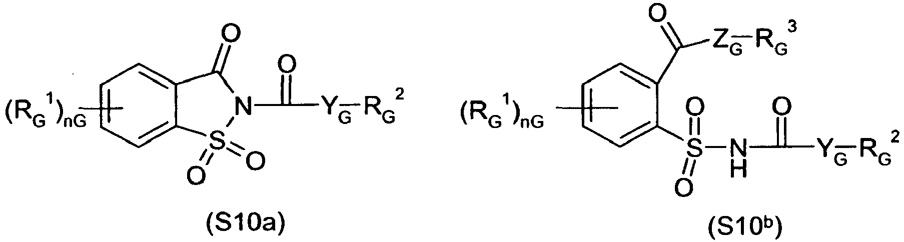 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S 11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S 12) Wirkstoffe aus der Klasse der Isothiochromanone (S 12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
S 13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S 13-5) der Firma Nitrokemia, das als Safener für Mais bekannt ist,
   "MG-838" (CAS-Reg.Nr. 133993-74-5) (2-propenyl 1-oxa-4-azaspiro[4.5]decane-4-carbodithioate) (S 13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorotioat) (S 13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (3-(2-Chlorphenylmethyl)-1(1methyl-1phenylethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Als die die Kulturpflanzen-Verträglichkeit verbessernde Verbindung [Komponente (b')] sind Cloquintocet-mexyl, Fenchlorazol-ethylester, Isoxadifen-ethyl, Mefenpyr-diethyl, Fenclorim, Cumyluron, S4-1 und S4-5 am meisten bevorzugt, wobei Cloquintocet-mexyl und Mefenpyr-diethyl besonders hervorgehoben seien.

Es wurde nun überraschend gefunden, dass die oben definierten Wirkstoffkombinationen aus Verbindungen der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von Verbindungen der Formel (I) auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
W steht bevorzugt für Wasserstoff, Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₄-Halogenalkyl, C₁-C₄-Halogenalkoxy oder Cyano,
X steht bevorzugt für Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Halogenalkyl, C₁-C₆-Alkoxy, C₃-C₆-Alkenyloxy, C₁-C₆-Alkyltlüo, C₁-C₆-Alkylsulfinyl, C₁-C₆-Alkylsulfonyl, C₁-C₆-Halogenalkoxy, C₃-C₆-Halogenalkenyloxy, Nitro oder Cyano,
Y und Z stehen bevorzugt unabhängig voneinander für Wasserstoff, Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₆-Halogenalkyl, C₁-C₆-Halogenalkoxy, Cyano, C₂-C₆-Alkenyl, C₂-C₆-Alkinyl oder für einen der (Het)-arylreste 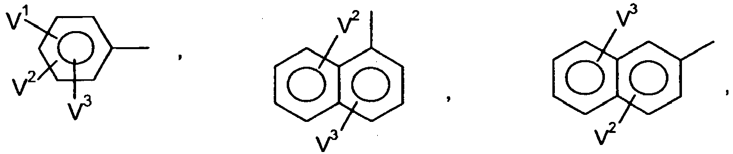 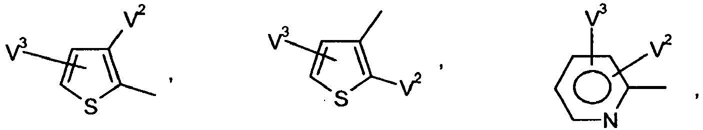 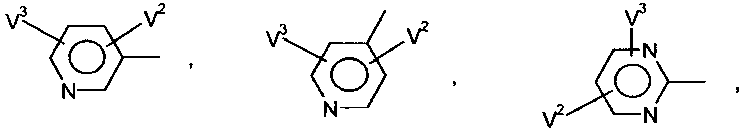 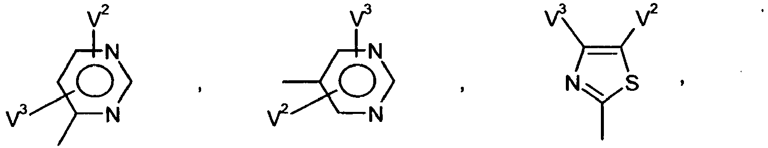 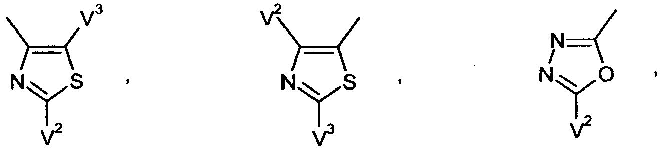 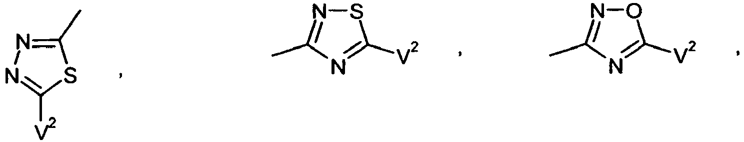 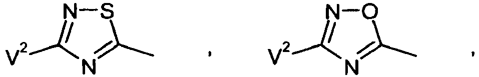 wobei im Falle von (Het)-aryl nur einer der Reste Y oder Z für (Het)-aryl stehen darf,
   - V¹: steht bevorzugt für Wasserstoff, Halogen, C₁-C₁₂-Alkyl, C₁-C₆-Alkoxy, C₁-C₆-Alkylthio, C₁-C₆-Alkylsulfmyl, C₁-C₆-Alkylsulfonyl, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio,
   - V² und V³: stehen bevorzugt unabhängig voneinander für Wasserstoff, Halogen, C₁-C₆-Alkyl, C₁-C₆-Alkoxy, C₁-C₄-Halogenalkyl oder C₁-C₄-Halogenalkoxy,
   - A: steht bevorzugt für C₁-C₆-Alkoxy,
   - B: steht bevorzugt für C₁-C₆-Alkyl,
   wobei A und B an das selbe Kohlenstoffatom gebunden sind,
   - G: steht bevorzugt für Wasserstoff (a) oder für eine der Gruppen 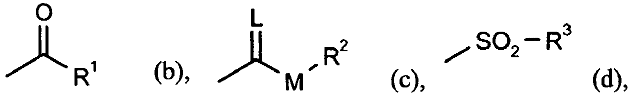 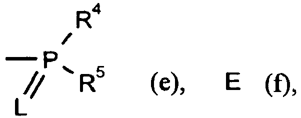 oder 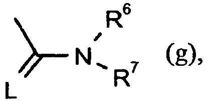 in welchen
   - E: für ein Metallion oder ein Ammoniumion steht,
   - L: für Sauerstoff oder Schwefel steht und
   - M: für Sauerstoff oder Schwefel steht,
   - R¹: steht bevorzugt für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
   für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
   für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
   für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
   für gegebenenfalls durch Halogen oder C₁-C₆-Alkyl substituiertes Phenoxy-C₁-C₆-alkyl oder
   für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff,
   - R²: steht bevorzugt für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
   für gegebenenfalls durch Halogen, C₁-C₆-Alkyl oder C₁-C₆-Alkoxy substituiertes C₃-C₈-Cycloalkyl oder
   für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl,
   - R³: steht bevorzugt für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
   - R⁴ und R⁵: stehen unabhängig voneinander bevorzugt für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogen-alkylthio, C₁-C₄-Alkyl oder C₁-C₄-Halogenalkyl substituiertes Phenyl, Phenoxy oder Phenylthio,
   - R⁶ und R⁷: stehen unabhängig voneinander bevorzugt für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest, in welchem gegebenenfalls eine Methylengruppe durch Sauerstoff oder Schwefel ersetzt ist.

In den als bevorzugt genannten Restedefinitionen steht Halogen für Fluor, Chlor, Brom und Iod, insbesondere für Fluor, Chlor und Brom.
- W: steht besonders bevorzugt für Wasserstoff, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₂-Halogenalkyl oder C₁-C₂-Halogenalkoxy,
- X: steht besonders bevorzugt für Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₄-Halogenalkyl, C₁-C₄-Halogenalkoxy oder Cyano,
- Y und Z: stehen besonders bevorzugt unabhängig voneinander für Wasserstoff, Fluor, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₆-Alkoxy, C₁-C₄-Halogenalkyl, C₁-C₄-Halogenalkoxy, Cyano, C₂-C₄-Alkenyl, C₂-C₄-Alkinyl oder für einen der (Het)-arylreste, 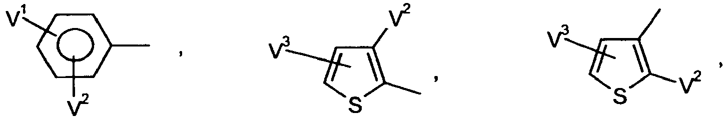 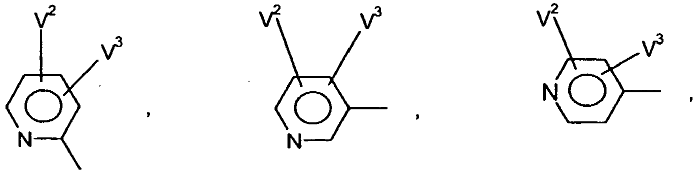 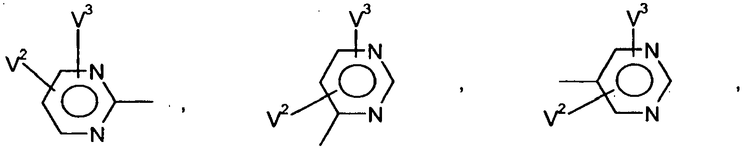
wobei im Falle von (Het)-aryl nur einer der Reste Y oder Z für (Het)-aryl stehen darf,
- V¹: steht besonders bevorzugt für Wasserstoff, Fluor, Chlor, Brom, C₁-C₆-Alkyl, C₁-C₄-Alkoxy, C₁-C₂- Halogenalkyl, C₁-C₂-Halogenalkoxy, Nitro, Cyano oder gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl,
- V² und V³: stehen besonders bevorzugt unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy,
- A: steht besonders bevorzugt für C₁-C₄-Alkoxy,
- B: steht besonders bevorzugt für C₁-C₄-Alkyl,
wobei A und B an das selbe Kohlenstoffatom gebunden sind,
- G: steht besonders bevorzugt für Wasserstoff (a) oder für eine der Gruppen 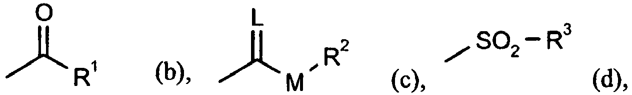 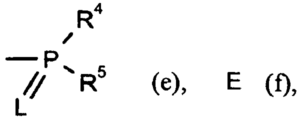
oder 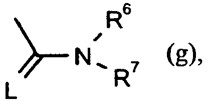 in welchen
- E: für ein Metallion oder ein Ammoniumion steht,
- L: für Sauerstoff oder Schwefel steht und
- M: für Sauerstoff oder Schwefel steht,
- R¹: steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl,
- R²: steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₅-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl,
- R³: steht besonders bevorzugt für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl,
- R⁴ und R⁵: stehen unabhängig voneinander besonders bevorzugt für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, oder C₁-C₃-Halogenalkyl substituiertes Phenyl, Phenoxy oder Phenylthio,
- R⁶ und R⁷: stehen unabhängig voneinander besonders bevorzugt für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor und Brom, insbesondere für Fluor und Chlor.
- W: steht ganz besonders bevorzugt für Wasserstoff, Chlor, Brom, Methyl, Ethyl, Vinyl, Ethinyl, Propinyl, Cyclopropyl, Methoxy, Ethoxy oder Trifluormethyl,
- X: steht ganz besonders bevorzugt für Chlor, Brom, Methyl, Ethyl, Propyl, iso-Propyl, Vinyl, Ethinyl, Propinyl, Cyclopropyl, Methoxy, Ethoxy, Trifluormethyl, Difluormethoxy, Trifluormethoxy oder Cyano,
- Y und Z: stehen ganz besonders bevorzugt unabhängig voneinander für Wasserstoff, Fluor, Chlor, Brom, Iod, Methyl, Ethyl, Vinyl, Ethinyl, Propinyl, Cyclopropyl, Methoxy, Trifluormethyl, Trifluormethoxy, Cyano oder einen Phenylrest, 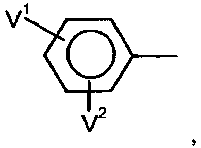 wobei im Falle von Phenyl nur einer der Reste Y oder Z für Phenyl stehen darf,
- V¹: steht ganz besonders bevorzugt für Wasserstoff, Fluor oder Chlor,
- V²: steht ganz besonders bevorzugt für Wasserstoff, Fluor, Chlor, Methyl, Ethyl, n-Propyl, iso-Propyl, Methoxy, Ethoxy oder Trifluormethyl,
- A: steht ganz besonders bevorzugt für Methoxy, Ethoxy oder Propoxy,
- B: steht ganz besonders bevorzugt für Methyl, Ethyl oder Propyl,
wobei A und B an das selbe Kohlenstoffatom gebunden sind, wobei die 3'-Position oder die 4'-Position bevorzugt sind,
- G: steht ganz besonders bevorzugt für Wasserstoff (a) oder für eine der Gruppen 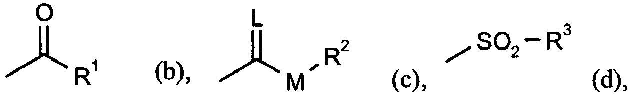 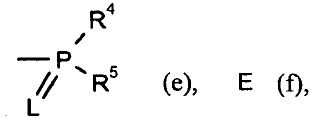 oder 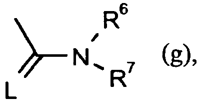
in welchen
- E: für ein Metallion oder ein Ammoniumion steht,
- L: für Sauerstoff oder Schwefel steht und
- M: für Sauerstoff oder Schwefel steht,
- R¹: steht ganz besonders bevorzugt für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl,
- R²: steht ganz besonders bevorzugt für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl,
- R³: steht ganz besonders bevorzugt für jeweils gegebenenfalls einfach bis dreifach durch Fluor oder Chlor substituiertes Methyl, Ethyl, Propyl oder iso-Propyl, oder jeweils gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl,
- R⁴ und R⁵: stehen unabhängig voneinander ganz besonders bevorzugt für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio,
- R⁶ und R⁷: stehen unabhängig voneinander ganz besonders bevorzugt für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für gegebenenfalls einfach bis zweifach durch Fluor, Chlor, Brom, Methyl, Methoxy oder Trifluormethyl substituiertes Phenyl, oder zusammen für einen C₅-C₆-Alkylenrest, in welchem gegebenenfalls eine Methylengruppe durch Sauerstoff oder Schwefel ersetzt ist.
- W: steht insbesondere bevorzugt für Wasserstoff, Chlor, Brom, Methyl, Ethyl oder Methoxy, (hervorgehoben für Wasserstoff, Methyl oder Ethyl),
- X: steht insbesondere bevorzugt für Chlor, Brom, Methyl, Ethyl, Methoxy oder Ethoxy,
- Y und Z: stehen insbesondere bevorzugt unabhängig voneinander für Wasserstoff, Chlor, Brom, Methyl oder für den Rest 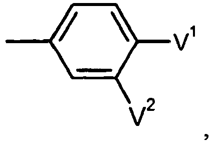 wobei in diesem Falle nur einer der Reste Y oder Z für 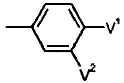 stehen darf,
- V¹: steht insbesondere bevorzugt für Fluor oder Chlor,
- V²: steht insbesondere bevorzugt für Wasserstoff, Fluor oder Chlor, (hervorgehoben für Wasserstoff),
- A: steht insbesondere bevorzugt für Methoxy oder Ethoxy,
- B: steht insbesondere bevorzugt für Methyl, Ethyl oder Propyl,
- wobei A und B: an das selbe Kohlenstoffatom in der 4'-Position gebunden sind,
- G: steht insbesondere bevorzugt für Wasserstoff (a) oder für eine der Gruppen in welchen 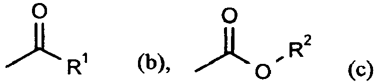 oder E(f)
- E: für ein Metallion oder ein Ammoniumion steht,
- R¹: steht insbesondere bevorzugt für C₁-C₁₀-Alkyl, C₁-C₄-Alkoxy-C₁-C₂-alkyl, C₃-C₆-Cycloalkyl,
für gegebenenfalls einfach durch Chlor substituiertes Phenyl oder für Thienyl, (hervorgehoben für C₁-C₁₀-Alkyl),
- R²: steht insbesondere bevorzugt für C₁-C₁₀-Alkyl, C₂-C₁₀-Alkenyl oder für Benzyl, (hervorgehoben für C₁-C₁₀-Alkyl).

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Hervorgehoben sind Verbindungen der Formel (I) in welchen G für Wasserstoff steht.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-a) genannt:

**Tabelle 1**

| | | | | | |
|---|---|---|---|---|---|
| 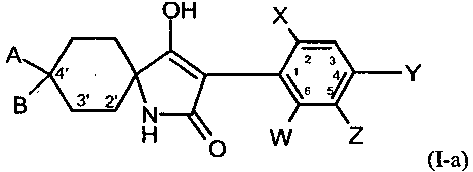 | | | | | |

| **A** | **B** | **X** | **W** | **Y** | **Z** |
|---|---|---|---|---|---|
| OCH₃ | CH₃ | CH₃ | H | H | H |
| OCH₃ | CH₃ | Br | H | H | H |
| OCH₃ | CH₃ | Cl | H | H | H |
| OCH₃ | CH₃ | CF₃ | H | H | H |
| OCH₃ | CH₃ | OCH₃ | H | H | H |
| OCH₃ | CH₃ | Br | H | Cl | H |
| OCH₃ | CH₃ | Cl | H | Br | H |
| OCH₃ | CH₃ | Cl | H | Cl | H |
| OCH₃ | CH₃ | Cl | H | CH₃ | H |
| OCH₃ | CH₃ | CH₃ | H | Cl | H |
| OCH₃ | CH₃ | Cl | Cl | H | H |
| OCH₃ | CH₃ | Cl | OCH₃ | H | H |
| OCH₃ | CH₃ | Cl | CH₃ | H | H |
| OCH₃ | CH₃ | Cl | OC₂H₅ | H | H |
| OCH₃ | CH₃ | OCH₃ | OCH₃ | H | H |
| OCH₃ | CH₃ | CH₃ | CH₃ | H | H |
| OCH₃ | CH₃ | C₂H₅ | CH₃ | H | H |
| OCH₃ | CH₃ | C₂H₅ | C₂H₅ | H | H |
| OCH₃ | CH₃ | Br | CH₃ | Br | H |
| OCH₃ | CH₃ | Cl | CH₃ | Cl | H |
| OCH₃ | CH₃ | CH₃ | Br | CH₃ | H |
| OCH₃ | CH₃ | CH₃ | Cl | CH₃ | H |
| OCH₃ | CH₃ | OCH₃ | CH₃ | CH₃ | H |
| OCH₃ | CH₃ | OC₂H₅ | CH₃ | CH₃ | H |
| OCH₃ | CH₃ | OC₃H₇ | CH₃ | CH₃ | H |
| OCH₃ | CH₃ | CH₃ | CH₃ | CH₃ | H |
| OCH₃ | CH₃ | Br | Br | CH₃ | H |
| OCH₃ | CH₃ | Cl | Cl | CH₃ | H |
| OCH₃ | CH₃ | CH₃ | CH₃ | Br | H |
| OCH₃ | CH₃ | OCH₃ | C₂H₅ | CH₃ | H |
| OCH₃ | CH₃ | OC₂H₅ | C₂H₅ | CH₃ | H |
| OCH₃ | CH₃ | CH₃ | CH₃ | OCH₃ | H |
| OCH₃ | CH₃ | Br | Cl | CH₃ | H |
| OCH₃ | CH₃ | Br | CH₃ | Cl | H |
| OCH₃ | CH₃ | Cl | CH₃ | Br | H |
| OCH₃ | CH₃ | CH₃ | CH₃ | Cl | H |
| OCH₃ | CH₃ | C₂H₅ | CH₃ | CH₃ | H |
| OCH₃ | CH₃ | C₂H₅ | C₂H₅ | CH₃ | H |
| OCH₃ | CH₃ | C₂H₅ | CH₃ | C₂H₅ | H |
| OCH₃ | CH₃ | C₂H₅ | C₂H₅ | C₂H₅ | H |
| OCH₃ | CH₃ | C₂H₅ | CH₃ | Cl | H |
| OCH₃ | CH₃ | C₂H₅ | C₂H₅ | Cl | H |
| OCH₃ | CH₃ | C₂H₅ | CH₃ | Br | H |
| OCH₃ | CH₃ | C₂H₅ | C₂H₅ | Br | H |
| OCH₃ | CH₃ | C₂H₅ | Cl | CH₃ | H |
| OCH₃ | CH₃ | C₂H₅ | Br | CH₃ | H |
| OCH₃ | CH₃ | C₂H₅ | Cl | Cl | H |
| OCH₃ | CH₃ | C₂H₅ | Br | Br | H |
| OCH₃ | CH₃ | C₂H₅ | Cl | Br | H |
| OCH₃ | CH₃ | C₂H₅ | Br | Cl | H |
| OCH₃ | CH₃ | OCH₃ | CH₃ | Cl | H |
| OCH₃ | CH₃ | OCH₃ | C₂H₅ | Cl | H |
| OCH₃ | CH₃ | OC₂H₅ | CH₃ | Cl | H |
| OCH₃ | CH₃ | OC₂H₅ | C₂H₅ | Cl | H |
| OCH₃ | CH₃ | Cl | OCH₃ | CH₃ | H |
| OCH₃ | CH₃ | Cl | OC₂H₅ | CH₃ | H |
| OCH₃ | CH₃ | CH₃ | CH₃ | Cl | H |
| OCH₃ | CH₃ | Cl | H | Cl | Cl |
| OCH₃ | CH₃ | CH₃ | H | CH₃ | CH₃ |
| OCH₃ | CH₃ | CH₃ | H | Cl | CH₃ |
| OCH₃ | CH₃ | Br | H | Cl | CH₃ |
| OCH₃ | CH₃ | Br | H | CH₃ | CH₃ |
| OCH₃ | CH₃ | Cl | H | Br | CH₃ |
| OCH₃ | CH₃ | Cl | H | Cl | CH₃ |
| OCH₃ | CH₃ | CH₃ | H | Br | CH₃ |
| OCH₃ | CH₃ | Cl | H | CH₃ | Cl |
| OCH₃ | CH₃ | CH₃ | H | H | CH₃ |
| OCH₃ | CH₃ | Cl | H | H | CH₃ |
| OCH₃ | CH₃ | Br | H | H | CH₃ |
| OCH₃ | CH₃ | CH₃ | H | H | Cl |
| OCH₃ | CH₃ | CH₃ | H | H | Br |
| OCH₃ | CH₃ | CH₃ | CH₃ | CH₃ | CH₃ |
| OCH₃ | CH₃ | CH₃ | CH₃ | CH₃ | F |
| OCH₃ | CH₃ | CH₃ | CH₃ | CH₃ | Cl |
| OCH₃ | CH₃ | CH₃ | CH₃ | CH₃ | Br |
| OCH₃ | CH₃ | CH₃ | CH₃ | H | Cl |
| OCH₃ | CH₃ | CH₃ | CH₃ | H | Br |
| OCH₃ | CH₃ | Cl | Cl | H | Br |
| OCH₃ | CH₃ | CH₃ | CH₃ | 4-Cl-C₆H₄ | H |
| OCH₃ | CH₃ | C₂H₅ | CH₃ | 4-Cl-C₆H₄ | H |
| OCH₃ | CH₃ | C₂H₅ | C₂H₅ | 4-Cl-C₆H₄ | H |
| OCH₃ | CH₃ | Cl | CH₃ | 4-Cl-C₆H₄ | H |
| OCH₃ | CH₃ | Cl | C₂H₅ | 4-Cl-C₆H₄ | H |
| OCH₃ | CH₃ | CH₃ | H | H | 4-Cl-C₆H₄ |
| OCH₃ | CH₃ | CH₃ | CH₃ | H | 4-Cl-C₆H₄ |
| OCH₃ | CH₃ | CH₃ | H | CH₃ | 4-Cl-C₆H₄ |
| OCH₃ | CH₃ | CH₃ | CH₃ | CH₃ | 4-Cl-C₆H₄ |
| OCH₃ | CH₃ | Cl | H | H | 4-Cl-C₆H₄ |
| OCH₃ | CH₃ | J | H | H | H |
| OCH₃ | CH₃ | J | H | CH₃ | H |
| OCH₃ | CH₃ | J | CH₃ | H | H |
| OCH₃ | CH₃ | J | C₂H₅ | H | H |
| OCH₃ | CH₃ | CH₃ | H | H | J |
| OCH₃ | CH₃ | CH₃ | H | CH₃ | J |
| OCH₃ | CH₃ | J | CH₃ | CH₃ | H |
| OCH₃ | CH₃ | J | C₂H₅ | CH₃ | H |
| OCH₃ | CH₃ | J | CH₃ | Cl | H |
| OCH₃ | CH₃ | J | C₂H₅ | Cl | H |
| OCH₃ | CH₃ | J | Cl | CH₃ | H |
| OCH₃ | CH₃ | J | H | CH₃ | CH₃ |
| OCH₃ | CH₃ | CH₃ | H | J | H |
| OCH₃ | CH₃ | C₂H₅ | H | J | H |
| OCH₃ | CH₃ | CH₃ | CH₃ | J | H |
| OCH₃ | CH₃ | C₂H₅ | CH₃ | J | H |
| OCH₃ | CH₃ | C₂H₅ | C₂H₅ | J | H |
| OCH₃ | CH₃ | Cl | CH₃ | J | H |
| OCH₃ | CH₃ | Cl | C₂H₅ | J | H |
| OCH₃ | CH₃ | CH₃ | H | J | CH₃ |
| OCH₃ | CH₃ | CH₃ | CH₃ | H | J |
| OCH₃ | CH₃ | J | H | H | CH₃ |
| OCH₃ | CH₃ | C₂H₅ | H | H | H |
| OCH₃ | CH₃ | 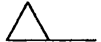 | H | H | H |
| OCH₃ | CH₃ | 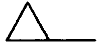 | CH₃ | H | H |
| OCH₃ | CH₃ | 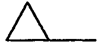 | H | CH₃ | H |
| OCH₃ | CH₃ | 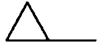 | C₂H₅ | H | H |
| OCH₃ | CH₃ | 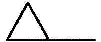 | CH₃ | CH₃ | H |
| OCH₃ | CH₃ | 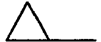 | C₂H₅ | CH₃ | H |
| OCH₃ | CH₃ | 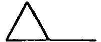 | CH₃ | Cl | H |
| OCH₃ | CH₃ | 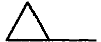 | C₂H₅ | Cl | H |
| OCH₃ | CH₃ | 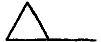 | Cl | CH₃ | H |
| OCH₃ | CH₃ | CH₃ | H | 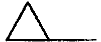 | H |
| OCH₃ | CH₃ | C₂H₅ | H | 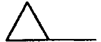 | H |
| OCH₃ | CH₃ | CH₃ | CH₃ | 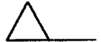 | H |
| OCH₃ | CH₃ | C₂H₅ | CH₃ | 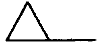 | H |
| OCH₃ | CH₃ | C₂H₅ | C₂H₅ | 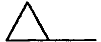 | H |
| OCH₃ | CH₃ | Cl | CH₃ | 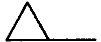 | H |
| OCH₃ | CH₃ | Cl | C₂H₅ | 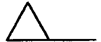 | H |

Weiterhin seien außer den bei den Beispielen genannten Verbindungen die folgenden Verbindungen der Formel (I) genannt:

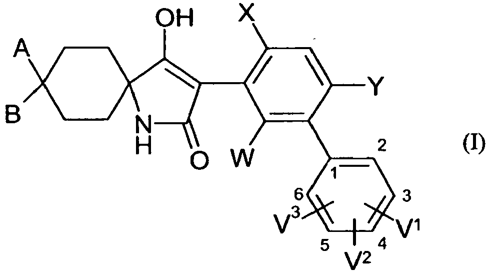

**Tabelle 2**

| **A** | **B** | **W** | **X** | **Y** | **V¹** | **V²** | **V³** |
|---|---|---|---|---|---|---|---|
| OCH₃ | CH₃ | H | Cl | H | 2-F | H | H |
| OCH₃ | CH₃ | H | Cl | H | 3-F | H | H |
| OCH₃ | CH₃ | H | Cl | H | 4-F | H | H |
| OCH₃ | CH₃ | H | Cl | H | 2-F | 4-F | H |
| OCH₃ | CH₃ | H | Cl | H | 2-F | 4-Cl | H |
| OCH₃ | CH₃ | H | Cl | H | 2-F | 4-CH₃ | H |
| OCH₃ | CH₃ | H | Cl | H | 2-F | 4-OCH₃ | H |
| OCH₃ | CH₃ | H | Cl | H | 3-F | 4-F | H |
| OCH₃ | CH₃ | H | Cl | H | 3-F | 4-Cl | H |
| OCH₃ | CH₃ | H | Cl | H | 3-F | 4-CH₃ | H |
| OCH₃ | CH₃ | H | Cl | H | 3-F | 4-OCH₃ | H |
| OCH₃ | CH₃ | H | Cl | H | 4-F | 3-Cl | H |
| OCH₃ | CH₃ | H | Cl | H | 4-F | 3-CH₃ | H |
| OCH₃ | CH₃ | H | Cl | H | 4-F | 3-OCH₃ | H |
| OCH₃ | CH₃ | H | Cl | H | 2-F | 4-F | 5-F |
| OCH₃ | CH₃ | H | Cl | H | 2-F | 4-F | 6-F |
| OCH₃ | CH₃ | H | Cl | H | 2-F | 4-Cl | 5-F |
| OCH₃ | CH₃ | H | Cl | H | 2-F | 5-Cl | 4-F |
| OCH₃ | CH₃ | H | Cl | H | 3-F | 4-F | 5-F |
| OCH₃ | CH₃ | H | Cl | H | 3-Cl | 4-Cl | H |
| OCH₃ | CH₃ | H | Cl | H | 4-CF₃ | 3-F | H |
| OCH₃ | CH₃ | H | Cl | H | 4-CN | H | H |
| OCH₃ | CH₃ | H | Cl | H | 3-CF₃ | 4-F | H |
| OCH₃ | CH₃ | H | CH₃ | H | 2-F | H | H |
| OCH₃ | CH₃ | H | CH₃ | H | 3-F | H | H |
| OCH₃ | CH₃ | H | CH₃ | H | 4-F | H | H |
| OCH₃ | CH₃ | H | CH₃ | H | 2-F | 4-F | H |
| OCH₃ | CH₃ | H | CH₃ | H | 2-F | 4-Cl | H |
| OCH₃ | CH₃ | H | CH₃ | H | 2-F | 4-CH₃ | H |
| OCH₃ | CH₃ | H | CH₃ | H | 2-F | 4-OCH₃ | H |
| OCH₃ | CH₃ | H | CH₃ | H | 3-F | 4-F | H |
| OCH₃ | CH₃ | H | CH₃ | H | 3-F | 4-Cl | H |
| OCH₃ | CH₃ | H | CH₃ | H | 3-F | 4-CH₃ | H |
| OCH₃ | CH₃ | H | CH₃ | H | 3-F | 4-OCH₃ | H |
| OCH₃ | CH₃ | H | CH₃ | H | 4-F | 3-Cl | H |
| OCH₃ | CH₃ | H | CH₃ | H | 4-F | 3-CH₃ | H |
| OCH₃ | CH₃ | H | CH₃ | H | 4-F | 3-OCH₃ | H |
| OCH₃ | CH₃ | H | CH₃ | H | 2-F | 4-F | 5-F |
| OCH₃ | CH₃ | H | CH₃ | H | 2-F | 4-F | 6-F |
| OCH₃ | CH₃ | H | CH₃ | H | 2-F | 4-Cl | 5-F |
| OCH₃ | CH₃ | H | CH₃ | H | 2-F | 5-Cl | 4-F |
| OCH₃ | CH₃ | H | CH₃ | H | 3-F | 4-F | 5-F |
| OCH₃ | CH₃ | H | CH₃ | H | 3-Cl | 4-Cl | H |
| OCH₃ | CH₃ | H | CH₃ | H | 4-CF₃ | 3-F | H |
| OCH₃ | CH₃ | H | CH₃ | H | 4-CN | H | H |
| OCH₃ | CH₃ | H | CH₃ | H | 3-CF3 | 4-F | H |
| OCH₃ | CH₃ | CH₃ | CH₃ | H | 2-F | H | H |
| OCH₃ | CH₃ | CH₃ | CH₃ | H | 3-F | H | H |
| OCH₃ | CH₃ | CH₃ | CH₃ | H | 4-F | H | H |
| OCH₃ | CH₃ | CH₃ | CH₃ | H | 2-F | 4-F | H |
| OCH₃ | CH₃ | CH₃ | CH₃ | H | 2-F | 4-Cl | H |
| OCH₃ | CH₃ | CH₃ | CH₃ | H | 2-F | 4-CH₃ | H |
| OCH₃ | CH₃ | CH₃ | CH₃ | H | 2-F | 4-OCH₃ | H |
| OCH₃ | CH₃ | CH₃ | CH₃ | H | 3-F | 4-F | H |
| OCH₃ | CH₃ | CH₃ | CH₃ | H | 3-F | 4-Cl | H |
| OCH₃ | CH₃ | CH₃ | CH₃ | H | 3-F | 4-CH₃ | H |
| OCH₃ | CH₃ | CH₃ | CH₃ | H | 3-F | 4-OCH₃ | H |
| OCH₃ | CH₃ | CH₃ | CH₃ | H | 4-F | 3-Cl | H |
| OCH₃ | CH₃ | CH₃ | CH₃ | H | 4-F | 3-CH₃ | H |
| OCH₃ | CH₃ | CH₃ | CH₃ | H | 4-F | 3-OCH₃ | H |
| OCH₃ | CH₃ | CH₃ | CH₃ | H | 2-F | 4-F | 5-F |
| OCH₃ | CH₃ | CH₃ | CH₃ | H | 2-F | 4-F | 6-F |
| OCH₃ | CH₃ | CH₃ | CH₃ | H | 2-F | 4-Cl | 5-F |
| OCH₃ | CH₃ | CH₃ | CH₃ | H | 2-F | 5-Cl | 4-F |
| OCH₃ | CH₃ | CH₃ | CH₃ | H | 3-F | 4-F | 5-F |
| OCH₃ | CH₃ | CH₃ | CH₃ | H | 3-CF₃ | 4-F | H |
| OCH₃ | CH₃ | CH₃ | CH₃ | H | 3-Cl | 4-Cl | H |
| OCH₃ | CH₃ | CH₃ | CH₃ | H | 4-CF₃ | 3-F | H |
| OCH₃ | CH₃ | CH₃ | CH₃ | H | 4-CN | H | H |
| OCH₃ | CH₃ | H | CH₃ | CH₃ | 2-F | H | H |
| OCH₃ | CH₃ | H | CH₃ | CH₃ | 3-F | H | H |
| OCH₃ | CH₃ | H | CH₃ | CH₃ | 4-F | H | H |
| OCH₃ | CH₃ | H | CH₃ | CH₃ | 2-F | 4-F | H |
| OCH₃ | CH₃ | H | CH₃ | CH₃ | 2-F | 4-Cl | H |
| OCH₃ | CH₃ | H | CH₃ | CH₃ | 2-F | 4-CH₃ | H |
| OCH₃ | CH₃ | H | CH₃ | CH₃ | 2-F | 4-OCH₃ | H |
| OCH₃ | CH₃ | H | CH₃ | CH₃ | 3-F | 4-F | H |
| OCH₃ | CH₃ | H | CH₃ | CH₃ | 3-F | 4-Cl | H |
| OCH₃ | CH₃ | H | CH₃ | CH₃ | 3-F | 4-CH₃ | H |
| OCH₃ | CH₃ | H | CH₃ | CH₃ | 3-F | 4-OCH₃ | H |
| OCH₃ | CH₃ | H | CH₃ | CH₃ | 4-F | 3-Cl | H |
| OCH₃ | CH₃ | H | CH₃ | CH₃ | 4-F | 3-CH₃ | H |
| OCH₃ | CH₃ | H | CH₃ | CH₃ | 4-F | 3-OCH₃ | H |
| OCH₃ | CH₃ | H | CH₃ | CH₃ | 2-F | 4-F | 5-F |
| OCH₃ | CH₃ | H | CH₃ | CH₃ | 2-F | 4-F | 6-F |
| OCH₃ | CH₃ | H | CH₃ | CH₃ | 2-F | 4-Cl | 5-F |
| OCH₃ | CH₃ | H | CH₃ | CH₃ | 2-F | 5-Cl | 4-F |
| OCH₃ | CH₃ | H | CH₃ | CH₃ | 3-F | 4-F | 5-F |
| OCH₃ | CH₃ | CH₃ | CH₃ | H | 3-Cl | 4-Cl | H |
| OCH₃ | CH₃ | CH₃ | CH₃ | H | 4-CF3 | 3-F | H |
| OCH₃ | CH₃ | CH₃ | CH₃ | H | 4-CN | H | H |
| OCH₃ | CH₃ | CH₃ | CH₃ | H | 3-CF3 | 4-F | H |
| OCH₃ | CH₃ | CH₃ | CH₃ | CH₃ | 2-F | H | H |
| OCH₃ | CH₃ | CH₃ | CH₃ | CH₃ | 3-F | H | H |
| OCH₃ | CH₃ | CH₃ | CH₃ | CH₃ | 4-F | H | H |
| OCH₃ | CH₃ | CH₃ | CH₃ | CH₃ | 2-F | 4-F | H |
| OCH₃ | CH₃ | CH₃ | CH₃ | CH₃ | 2-F | 4-Cl | H |
| OCH₃ | CH₃ | CH₃ | CH₃ | CH₃ | 2-F | 4-CH₃ | H |
| OCH₃ | CH₃ | CH₃ | CH₃ | CH₃ | 2-F | 4-OCH₃ | H |
| OCH₃ | CH₃ | CH₃ | CH₃ | CH₃ | 3-F | 4-F | H |
| OCH₃ | CH₃ | CH₃ | CH₃ | CH₃ | 3-F | 4-Cl | H |
| OCH₃ | CH₃ | CH₃ | CH₃ | CH₃ | 3-F | 4-CH₃ | H |
| OCH₃ | CH₃ | CH₃ | CH₃ | CH₃ | 3-F | 4-OCH₃ | H |
| OCH₃ | CH₃ | CH₃ | CH₃ | CH₃ | 4-F | 3-Cl | H |
| OCH₃ | CH₃ | CH₃ | CH₃ | CH₃ | 4-F | 3-CH₃ | H |
| OCH₃ | CH₃ | CH₃ | CH₃ | CH₃ | 4-F | 3-OCH₃ | H |
| OCH₃ | CH₃ | CH₃ | CH₃ | CH₃ | 2-F | 4-F | 5-F |
| OCH₃ | CH₃ | CH₃ | CH₃ | CH₃ | 2-F | 4-F | 6-F |
| OCH₃ | CH₃ | CH₃ | CH₃ | CH₃ | 2-F | 4-Cl | 5-F |
| OCH₃ | CH₃ | CH₃ | CH₃ | CH₃ | 2-F | 5-Cl | 4-F |
| OCH₃ | CH₃ | CH₃ | CH₃ | CH₃ | 3-F | 4-F | 5-F |
| OCH₃ | CH₃ | CH₃ | CH₃ | H | 3-Cl | 4-Cl | H |
| OCH₃ | CH₃ | CH₃ | CH₃ | H | 4-CF₃ | 3-F | H |
| OCH₃ | CH₃ | CH₃ | CH₃ | H | 4-CN | H | H |
| OCH₃ | CH₃ | CH₃ | CH₃ | H | 3-CF₃ | 4-F | H |

Tabelle 3 X, W, Y und Z wie in Tabelle 1 und 2 angegeben
   A = OC₂H₅; B = CH₃
Tabelle 4 X, W, Y und Z wie in Tabelle 1 und 2 angegeben
   A = OCH₃; B = C₂H₅

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat, Phosphinothricin und für phenylsubstituierte Cyclische Ketoenole beschrieben (US 6 645 914, EP-A2 0 036 106, WO 07/068427). Eine entsprechende Wirkungssteigerung bei Insektiziden wurde bereits durch WO 07/068428 beschrieben.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun ebenfalls überraschend gefunden, dass sich die Wirkung von Insektiziden und/oder Akariziden und/oder Herbiziden aus der Klasse der geminal Alkoxy/Alkylspirocyclisch-substituierte Tetramsäure-Derivate der Formel (I) durch den Zusatz von Ammonium- oder Phosphoniumsalzen zur Anwendungslösung oder durch den Einbau dieser Salze in eine Formulierung enthaltend geminal Alkoxy/Alkylspirocyclisch-substituierte Tetramsäure-Derivate der Formel (I) deutlich steigern lässt. Gegenstand der vorliegenden Erfindung ist also die Verwendung von Ammonium- oder Phosphoniumsalzen zur Wirkungssteigerung von Pflanzenschutzmitteln, die herbizid und/oder insektizid und/oder akarizid wirksame geminal Alkoxy/Alkylspirocyclisch-substituierte Tetramsäure-Derivate der Formel (I) als Wirkstoff enthalten. Gegenstand der Erfindung sind ebenfalls Mittel, die herbizid und/oder akarizid und/oder insektizid wirksame geminal Alkoxy/Alkylspirocyclisch-substituierte Tetramsäure-Derivate der Formel (I) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Die Verbindungen der Formel (I) besitzen eine breite insektizide und/oder akarizide und/oder herbizide Wirkung, die Wirkung und/oder Pflanzenverträglichkeit lässt im Einzelnen aber zu wünschen übrig.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Fettsäure-Biosynthese-Inhibitoren steigern, werden durch Formel (III') definiert 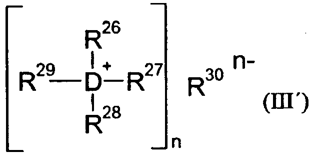 in welcher
- D: für Stickstoff oder Phosphor steht,
- D: bevorzugt für Stickstoff steht,
- R²⁶, R²⁷, R²⁸ und R²⁹: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R²⁶, R²⁷, R²⁸ und R²⁹: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R²⁶, R²⁷, R²⁸ und R²⁹: besonders bevorzugt unabhängig voneinander für Wasserstoff, Methyl, Ethyl, n-Propyl, i-Propyl, n-Butyl, i-Butyl, s-Butyl oder t-Butyl stehen,
- R²⁶, R²⁷, R²⁸ und R²⁹: ganz besonders bevorzugt für Wasserstoff stehen,
- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
- R³⁰: besonders bevorzugt für Laktat, Sulfat, Nitrat, Thiosulfat, Thiocyanat, Oxalat oder Formiat steht,
- R³⁰: ganz besonders bevorzugt für Sulfat steht.

Erfindungsgemäß hervorgehobene Kombinationen von Wirkstoff, Salz und Penetrationsförderer sind in folgender Tabelle aufgeführt. "Penetrationsförderer gemäß Test" bedeutet dabei, dass jede Verbindung geeignet ist, die in dem Test für die Kutikelpenetration (Baur et al., 1997, Pesticide Science 51, 131-152) als Penetrationsförderer wirkt.

Die Ammonium- und Phosphoniumsalze der Formel (III') können in einem breiten Konzentrationsbereich zur Steigerung der Wirkung von Pflanzenschutzmitteln enthaltend Ketoenole eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid und/oder akarizid und/oder herbizid wirksame, geminal Alkoxy/Alkylspirocyclisch-substituierte Tetramsäure-Derivate der Formel (I) als Wirkstoff enthalten. Gegenstand der Erfindung sind ebenfalls Mittel, die herbizid und/oder akarizid und/oder insektizid wirksame geminal Alkoxy/Alkylspirocyclisch-substituierte Tetramsäure-Derivate der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässrigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV')

R-O-(-AO)ᵥ-R'(IV')

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht,
- PO: für 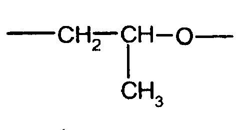 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-CEO-)ₛ-R' (IV'-c)

in welcher
- R: die oben angegebene Bedeutung hat,
- R': die oben angegebene Bedeutung hat,
- EO: für -CH₂-CH₂-O- steht,
- PO: für 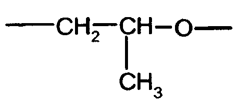 steht
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher
- R und R': die oben angegebenen Bedeutungen haben,
- EO: für -CH₂-CH₂-O- steht,
- BO: für 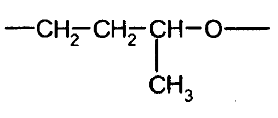 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher

R und R' die oben angegebenen Bedeutungen haben,
- BO: für 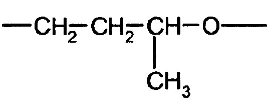 steht,
- EO: für -CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (IV'-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV-c) sei 2-Ethyl-hexyl-alkoxylat der Formel 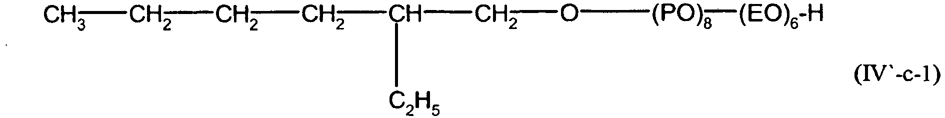 in welcher
- EO: für -CH₂-CH₂-O- steht,
- PO: für 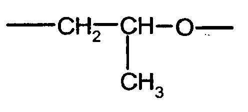 steht und
die Zahlen 8 und 6 Durchschnittswerte darstellen, genannt.

Als Beispiel für ein Alkanol-Alkoxylat der Formel (IV-d) sei die Formel

CH3-(CH2)₁₀-O-(-EO-)₆-(-BO-)₂-CH₃ (IV'-d-1)

in welcher
- EO: für -CH₂-CH₂-O- steht,
- BO: für 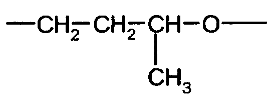 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-O-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäwen, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Verwendet man beispielsweise gemäß Verfahren (A) N-[(2,4,6-Trimethyl)-phenylacetyl]-1-amino-4- methoxy-4'-methyl-cyclohexancarbonsäureethylester als Ausgangsstoff, so kann der Verlauf des erfindungsgemäßen Verfahrens durch folgendes Reaktionsschema wiedergegeben werden:

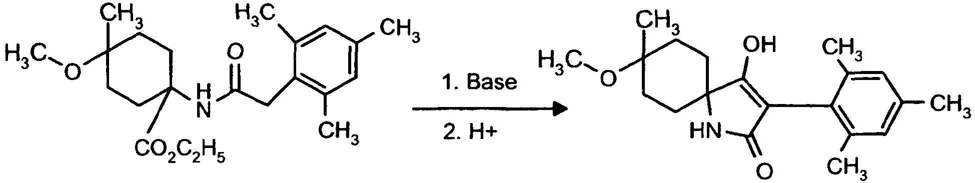

Verwendet man beispielsweise gemäß Verfahren (Bα) 8-Ethoxy-8'-methyl-3-[(4-chlor-2,6-dimethyl)-phenyl]-1-azaspiro[4,5]decan-2,4-dion und Pivaloylchlorid als Ausgangsstoffe, so kann der Verlauf des erfindungsgemäßen Verfahrens durch folgendes Reaktionsschema wiedergegeben werden:

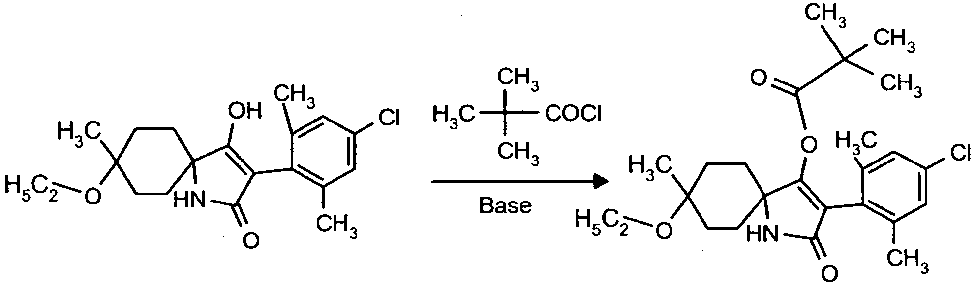

Verwendet man beispielsweise gemäß Verfahren (B) (Variante ß) 8-Methoxy-8'-methyl-3-[(2,4-dichlor)-phenyl]-1-azaspiro-[4,5]-decan-2,4-dion und Acetanhydrid als Ausgangsverbindungen, so kann der Verlauf des erfindungsgemäßen Verfahrens durch folgendes Reaktionsschema wiedergegeben werden:

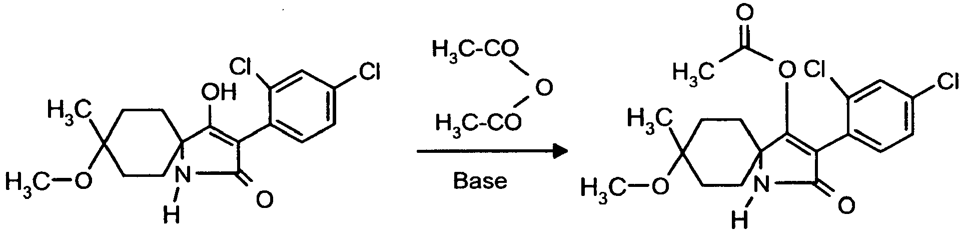

Verwendet man beispielsweise gemäß Verfahren (C) 8-Methoxy-8'-ethyl-3-[(2,4-dichlor-6-methyl)-phenyl]-1-azaspiro[4,5]decan-2,4-dion und Chlorameisensäureethylester als Ausgangsverbindungen, so kann der Verlauf des erfindungsgemäßen Verfahrens durch folgendes Reaktionsschema wiedergegeben werden:

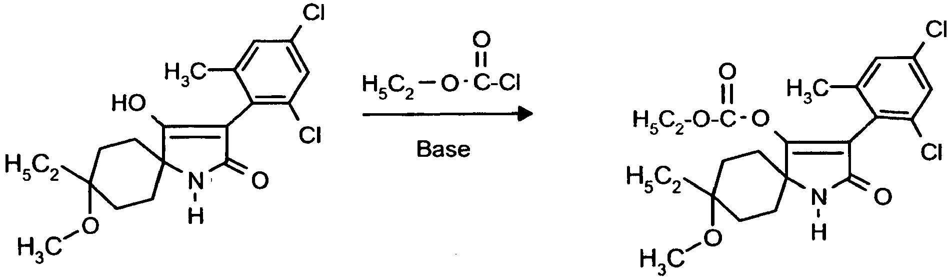

Verwendet man beispielsweise gemäß Verfahren (D) 8-Ethoxy-8'-methyl-3-[(2,4,6-trimethyl)-phenyl]-1-azaspiro[4,5]decan-2,4-dion und Chlormonothioameisensäuremethylester als Ausgangsprodukte, so kann der Reaktionsverlauf folgendermaßen wiedergegeben werden:

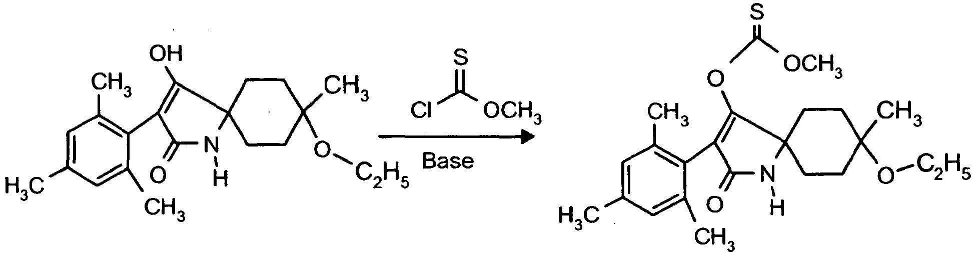

Verwendet man beispielsweise gemäß Verfahren (E) 8-Methoxy-8'-methyl-3-[(2,4,6-trimethyl)-phenyl]-1-azaspiro[4,5]decan-2,4-dion und Methansulfonsäurechlorid als Ausgangsprodukte, so kann der Reaktionsverlauf durch folgendes Reaktionsschema wiedergegeben werden:

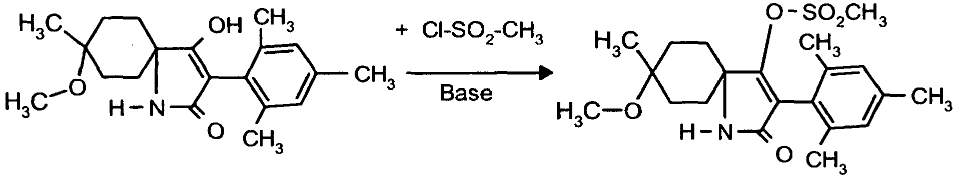

Verwendet man beispielsweise gemäß Verfahren (F) 8-Ethoxy-8'-methyl-3-[(2,4-dichlor-6-methyl)-phenyl]-1-azaspiro[4,5]decan-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

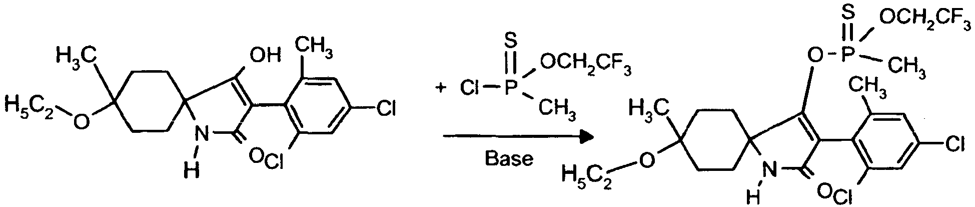

Verwendet man beispielsweise gemäß Verfahren (G) 8-Methoxy-8'-methyl-3-[(2,3,4,6-tetra-methylphenyl]-1-azaspiro[4,5]decan-2,4-dion und NaOH als Komponenten, so kann der Verlauf des erfindungsgemäßen Verfahrens durch folgendes Reaktionsschema wiedergegeben werden:

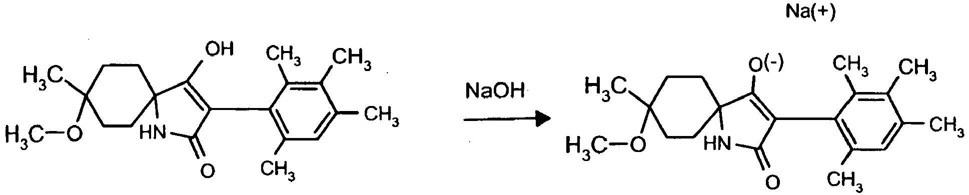

Verwendet man beispielsweise gemäß Verfahren (H) (Variante α) 8-Methoxy-8'-methyl-3-[(2,4,5-trimethyl)-phenyl]-1-azaspiro[4,5]decan-2,4-dion und Ethylisocyanat als Ausgangsprodukte, so kann der Reaktionsverlauf durch folgendes Reaktionsschema wiedergegeben werden:

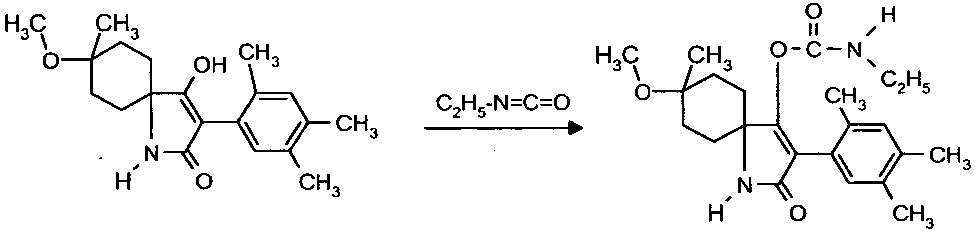

Verwendet man beispielsweise gemäß Verfahren (H) (Variante ß) 8-Methoxy-8'-methyl-3-[(2,4,6-trimethyl)-phenyl]-1-azaspiro[4,5]decan-2,4-dion und Dimethylcarbamidsäurechlorid als Ausgangsprodukte, so kann der Reaktionsverlauf durch folgendes Schema wiedergegeben werden:

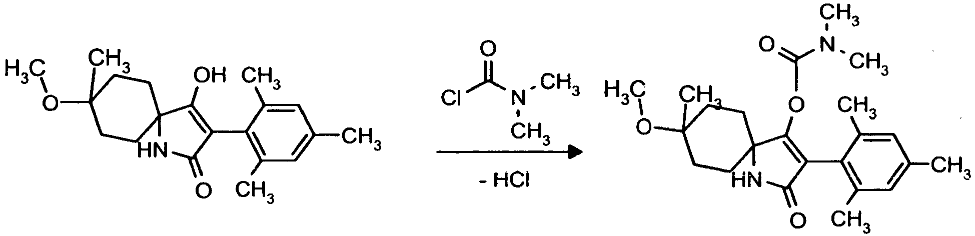

Verwendet man beispielsweise gemäß Verfahren (Iß) 8-Methoxy-8'-methyl-3-[(4-brom-2,6-dimethyl-phenyl)]-1-azaspiro[4,5]decan-2,4-dion und 4-Chlorphenyl-boronsäure als Ausgangsstoffe, so kann der Reaktionsverlauf durch folgendes Schema wiedergegeben werden:

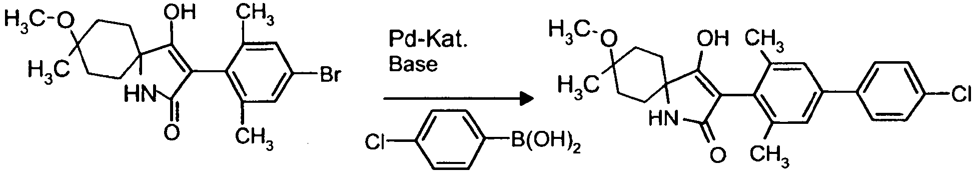

Die beim erfindungsgemäßen Verfahren (A) als Ausgangsstoffe benötigten Verbindungen der Formel (II) 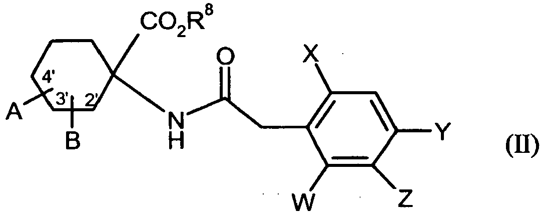 in welcher
A, B, W, X, Y, Z und R⁸ die oben angegebenen Bedeutungen haben,
sind neu.
Man erhält die Acylaminosäureester der Formel (II) beispielsweise, wenn man Aminosäurederivate der Formel (XVI) 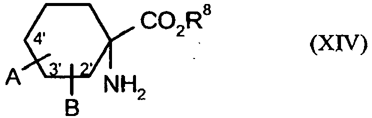 in welcher
A, B und R⁸ die oben angegebene Bedeutung haben,
mit substituierten Phenylessigsäurederivaten der Formel (XV) 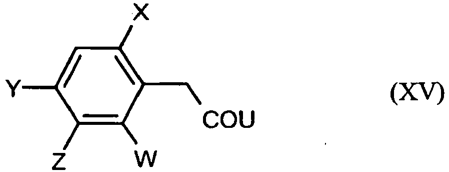 in welcher
W, X, Y und Z die oben angegebenen Bedeutungen haben und
U für eine durch Carbonsäureaktivierungsreagenzien wie Carbonyldiimidazol, Carbonyldiimide (wie z.B. Dicyclohexylcarbondiimid), Phosphorylierungsreagenzien (wie z.B. POCl₃, BOP-Cl), Halogenierungsmittel wie z.B. Thionylchlorid, Oxalylchlorid, Phosgen oder Chlorameisensäureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XVI) 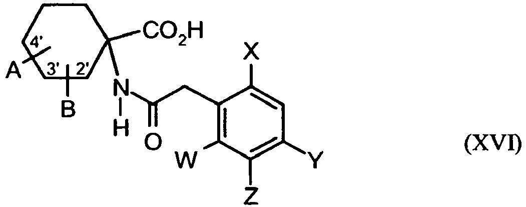 in welcher
A, B, W, X, Y und Z die oben angegebenen Bedeutungen haben,
verestert (Chem. Ind. (London) 1568 (1968)).

Die Verbindungen der Formel (XVI) 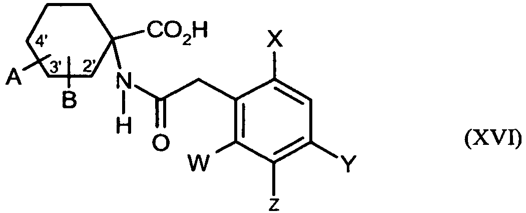 in welcher
A, B, W, X, Y und Z die oben angegebenen Bedeutungen haben,
sind neu.

Man erhält die Verbindungen der Formel (XVI) beispielsweise, wenn man 1-Amino-cyclohexancarbonsäuren der Formel (XVII) 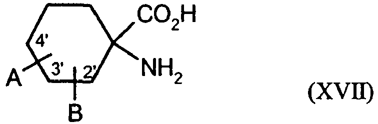 in welcher
A und B die oben angegebenen Bedeutungen haben
mit substituierten Phenylessigsäurederivaten der Formel (XV) 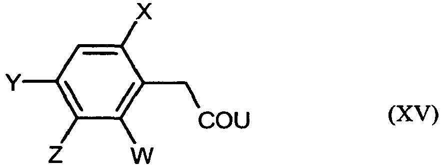 in welcher
U, W, X, Y und Z die oben angegebenen Bedeutungen haben
z.B. nach Schotten-Baumann acyliert (Organikum, VEB Deutscher Verlag der Wissenschaften, Berlin 1977, S. 505).

Die Verbindungen der Formel (XV) sind teilweise bekannt und/oder lassen sich nach den bekannten Verfahren in den eingangs zitierten Offenlegungsschriften herstellen.

Die Verbindungen der Formel (XIV) und (XVII) sind neu und lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975).

Die neuen 1-Amino-cyclohexan-carbonsäuren (XVII) sind im Allgemeinen nach der Bucherer Bergs-Synthese oder nach der Strecker-Synthese erhältlich und fallen dabei jeweils in unterschiedlichen Isomerenformen an. Im Folgenden werden der Einfachheit halber die Isomeren als ß bezeichnet, in welchem das Sauerstoffatom in der 4-Position und die Aminogruppe äquatorial/axial oder axial/äquatorial stehen. Im Folgenden werden der Einfachheit halber die Isomeren als a bezeichnet, in welchen die Aminogruppe und das Sauerstoffatom in der 4-Position äquatorial/äquatorial oder axial/axial stehen.

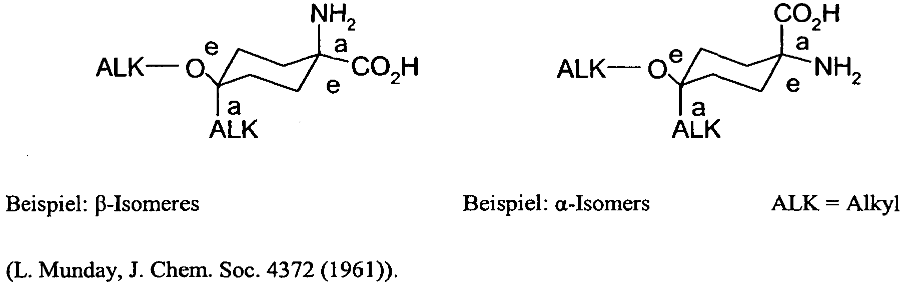

Die Verbindungen der Formel (XVII) sind erhältlich ausgehend von Verbindungen der Formel (XVIII) 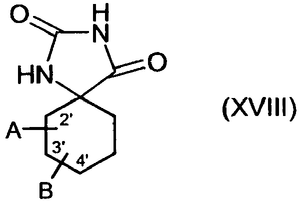 in welcher A und B die oben angegebenen Bedeutungen haben.

Die Verbindungen der Formel (XVIII) sind neu und lassen sich nach literaturbekannten Methoden (z.Bsp. Bucherer-Bergs-Reaktion, s.a. Beispiele) herstellen.

Weiterhin lassen sich die bei dem obigen Verfahren (A) verwendeten Ausgangsstoffe der Formel (II) 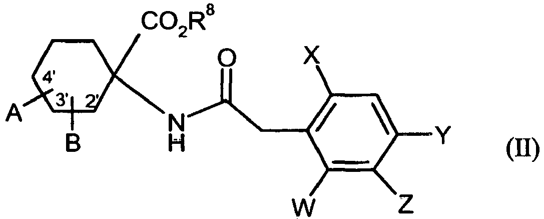 in welcher
A, B, W, X, Y, Z und R⁸ die oben angegebenen Bedeutungen haben,
herstellen, wenn man 1-Amino-cyclohexan-carbonsäurenitrile der Formel (XIX) 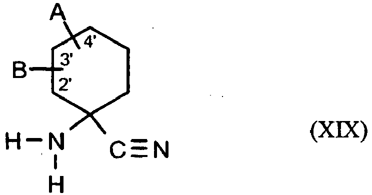 in welcher
A und B die oben angegebenen Bedeutungen haben,
mit substituierten Phenylessigsäurederivaten der Formel (XV) 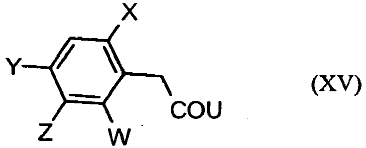 in welcher
U, W, X, Y und Z die oben angegebenen Bedeutungen haben,
zu Verbindungen der Formel (XX) 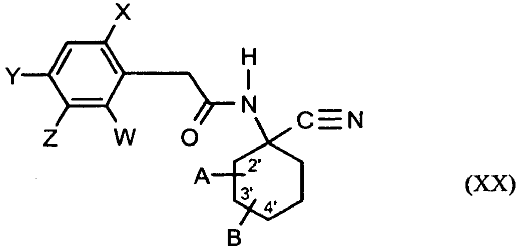 in welcher
A, B, W, X, Y und Z die oben angegebenen Bedeutungen haben, umsetzt,
und diese anschließend einer sauren Alkoholyse unterwirft.

Die Verbindungen der Formel (XX) sind ebenfalls neu und lassen sich nach bekannten Verfahren die in der eingangs zitierten Literatur beschrieben sind herstellen. Die Verbindungen der Formel (XIX) sind auch neu und lassen sich z.B. wie in EP-A-595 130 beschrieben herstellen.

Die zur Durchführung der erfindungsgemäßen Verfahren (B), (C), (D), (E), (F), (G), (H) und (I) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Sulfonsäure-chloride der Formel (VII), Phosphorverbindungen der Formel (VIII) und Metallhydroxide, Metallalkoxide oder Amine der Formel (IX) und (X) und Isocyanate der Formel (XI) und Carbamidsäurechloride der Formel (XII) und Boronsäuren der Formel (XIII) sind allgemein bekannte Verbindungen der organischen bzw. anorganischen Chemie.

Die Verbindungen der Formeln (XV) sind darüber hinaus aus den eingangs zitierten Patentanmeldungen bekannt und/oder lassen sich nach den dort angegebenen Methoden herstellen.

Die Verbindungen der Formeln (I-a' - I-g') und (I-a" - I-g") lassen sich nach den beschriebenen Verfahren A bis H herstellen.

Das Verfahren (A) ist dadurch gekennzeichnet, dass man Verbindungen der Formel (II), in welcher A, B, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (=Methyltrialkyl(C₈-C₁₀)ammoniumchlorid) oder TDA 1 (=Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -75°C und 200°C, vorzugsweise zwischen -50°C und 150°C. Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppelt-äquimolaren Mengen ein. Es ist jedoch auch möglich, die eine oder andere Komponente in einem größeren Überschuss (bis zu 3 Mol) zu verwenden.

Das Verfahren (B_{α}) ist dadurch gekennzeichnet, dass man Verbindungen der Formel (I-a) jeweils mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (B_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (B_{α}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (I-a) und das Carbonsäurehalogenid der Formel (III) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (B_{ß}) ist dadurch gekennzeichnet, dass man Verbindungen der Formel (I-a) jeweils mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B_{ß}) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (B_{ß}) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (B_{ß}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (B_{ß}) werden die Ausgangsstoffe der Formel (I-a) und das Carbonsäureanhydrid der Formel (IV) im allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, dass man Verbindungen der Formel (I-a) jeweils mit Chlorameisensäureestern oder Chlorameisensäwethiolestern der Formel (V) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber den Chlorameisensäureestern bzw. Chlorameisensäwethiolestem inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (C) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formeln (I-a) und der entsprechende Chlorameisensäureester bzw. Chlorameisensäurethiolester der Formel (V) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Das erfindungsgemäße Verfahren (D) ist dadurch gekennzeichnet, dass man Verbindungen der Formel (I-a) jeweils mit Verbindungen der Formel (VI) in Gegenwart eines Verdünnungsmittels und gegebenenfalls in Gegenwart eines Säurebindemittels umsetzt.

Beim Herstellungsverfahren (D) setzt man pro Mol Ausgangsverbindung der Formel (I-a) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid, Essigsäureethylester oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-a) dar, kann auf den weiteren Zusatz von Säurebindemitteln verzichtet werden.

Als Basen können beim Verfahren (D) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (E) ist dadurch gekennzeichnet, dass man Verbindungen der Formel (I-a) jeweils mit Sulfonsäurechloriden der Formel (VII) gegebenenfalls in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Sulfonsäurechlorid der Formel (VII) bei -20 bis 150°C, vorzugsweise bei 0 bis 70°C um.

Das Verfahren (E) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Essigsäureethylester, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formel (I-a) jeweils mit Phosphorverbindungen der Formel (VIII) gegebenenfalls in Gegenwart eines Verdünnungsmittels und gegebenenfalls in Gegenwart eines Säurebindemittels umsetzt.

Beim Herstellungsverfahren (F) setzt man zum Erhalt von Verbindungen der Formel (I-e) auf 1 Mol der Verbindungen (I-a) 1 bis 2, vorzugsweise 1 bis 1,3 Mol der Phosphorverbindung der Formel (VIII) bei Temperaturen zwischen -40°C und 150°C, vorzugsweise zwischen -10 und 110°C um.

Das Verfahren (F) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (G) ist dadurch gekennzeichnet, dass man Verbindungen der Formel (I-a) jeweils mit Metallhydroxiden bzw. Metallalkoxiden der Formel (IX) oder Aminen der Formel (X), gegebenenfalls in Gegenwart eines Verdünnungsmittels, umsetzt.

Als Verdünnungsmittel können bei dem erfindungsgemäßen Verfahren (G) vorzugsweise Ether wie Tetrahydrofuran, Dioxan, Diethylether oder aber Alkohole wie Methanol, Ethanol, Iso-propanol, aber auch Wasser eingesetzt werden. Das erfindungsgemäße Verfahren (G) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (H) ist dadurch gekennzeichnet, dass man Verbindungen der Formel (I-a) jeweils mit (Hα) Verbindungen der Formel (XI) gegebenenfalls in Gegenwart eines Verdünnungsmittels und gegebenenfalls in Gegenwart eines Katalysators oder (Hß) mit Verbindungen der Formel (XII) gegebenenfalls in Gegenwart eines Verdünnungsmittels und gegebenenfalls in Gegenwart eines Säurebindemittels umsetzt.

Bei Herstellungsverfahren (Hα) setzt man pro Mol Ausgangsverbindung der Formel (I-a) ca. 1 Mol Isocyanat der Formel (XI) bei 0 bis 100°C, vorzugsweise bei 20 bis 50°C um.

Das Verfahren (Hα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Hß) setzt man pro Mol Ausgangsverbindung der Formel (I-a) ca. 1 Mol Carbamidsäurechlorid der Formel (XII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylen-chlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Zur Durchführung des erfmdungsgemäßen Verfahrens (Iα) und (Iβ) sind Palladium(0)-Komplexe als Katalysator geeignet. Bevorzugt wird beispielsweise Tetrakis-(triphenylphosphin)palladium. Gegebenenfalls können auch Palladium(II)-Verbindungen eingesetzt werden, beispielsweise PdCl₂, Pd(OAc)₂. Bei der Verwendung von Palladium(II)-Verbindungen werden in der Regel Phosphine als Komplexbildner wie beispielsweise Tricyclohexylphosphin eingesetzt.

Als Säureakzeptoren zur Durchführung des erfindungsgemäßen Verfahrens (Iα) und (Iβ) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium-, Cäsium- oder Ammoniumcarbonat, Natriumhydrogen- oder Kaliumhydrogencarbonat, Alkalifluoride, wie beispielsweise Cäsiumfluorid, Alkaliphosphate wie z.B. Kaliumdihydrogenphosphat, Kaliumphosphat sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (Iα) und (Iβ) kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Dicalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuren, 1,2-Dimethoxyethan, 1,2-Diethoxyethan, Diethylenglykoldimethylether oder Anisol; Alkohole, wie Methanol, Ethanol, n-oder i-Propanol, n-, iso-, sek.- oder tert.-Butanol, Ethandiol, Propan-1,2-diol, Ethoxyethanol, Methoxyethanol, Diethylenglykolmonomethylether, Diethylglykolmonomethylether; Wasser.

Die Reaktionstemperatur kann bei dem erfindungsgemäßen Verfahren (Iα) und (Iβ) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (Iα) und (Iβ) werden die Boronsäuren der Formeln (XIIIα) und (XIIIβ), in welchen Y und Z die oben angegebene Bedeutung haben und Verbindungen der Formeln (I-a') bis (I-g'), in welchen A, B, G, W, X, Y, und Z' bzw. (I-a") bis (I-g"), in welchen A, B, G, W, X, Z und Y' die oben angegebene Bedeutung haben, im molaren Verhältnis 1:1 bis 3:1, vorzugsweise 1:1 bis 2:1 eingesetzt. Vom Katalysator setzt man im allgemeinen 0,005 bis 0,5 Mol, vorzugsweise 0,01 Mol bis 0,1 Mol pro Mol der Verbindungen der Formeln (I-a') bis (I-g') bzw. (I-a") bis (I-g") ein. Die Base setzt man im Allgemeinen in einem Überschuss ein. Die Aufarbeitung geschieht nach üblichen Methoden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoffimprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Erfindungsgemäß bedeutet Trägerstoff eine natürliche oder synthetische, organische oder anorganische Substanz, welcher fest oder flüssig sein kann, mit welchen die Wirkstoffe zur besseren Anwendbarkeit, insbesondere zum Aufbringen auf Pflanzen oder Pflanzenteile, gemischt oder verbunden sind. Der feste oder flüssige Trägerstoff ist im Allgemeinen inert und sollte in der Landwirtschaft verwendbar sein.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfmdungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIIA, CryIBB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirexjuvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propeller Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Verbindungen der Formel (I) (Wirkstoffe) weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Mit den erfindungsgemäßen Wirkstoffen können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der Formel (I) in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten. Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühle und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischem und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Die Bezeichnung "Wirkstoffe" bzw. "Verbindungen" schließt immer auch die hier genannten Wirkstoffkombinationen mit ein.

Die Herstellung und die Verwendung der erfindungsgemäßen Wirkstoffe geht aus den nachfolgenden Beispielen hervor.

### Herstellungsbeispiele:

### Beispiel I-a-1, I-a-2

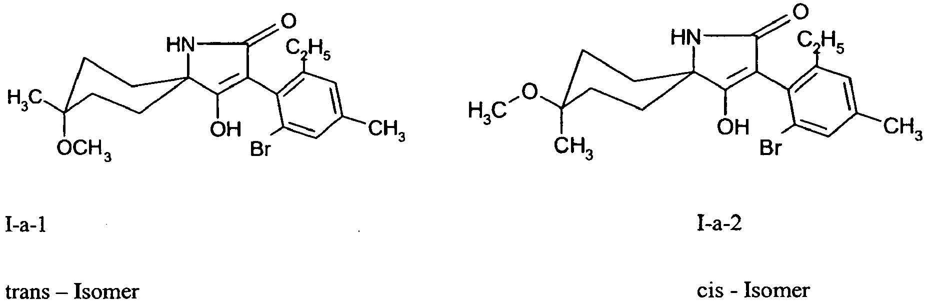

0,46 g (4,1 mmol) Kalium-tert.-butylat werden in 4 ml N,N-Dimethylacetamid (DMA) vorgelegt. Bei 20°C tropft man 0,6 g (1,36 mmol) der Verbindung gemäß Beispiel II-7 in 10 ml DMA zu und rührt 2 h nach. Das Reaktionsgemisch wird auf Eiswasser gegossen, mit verdünnter Salzsäure angesäuert, mit Methylenchlorid extrahiert, getrocknet und eingedampft. Der Rückstand wird an Kieselgel mit Methylenchlorid/Essigester (3 : 1) chromatographiert. Man erhält 0,09 g (15 % d. Theorie) der Verbindung I-a-1 vom Fp. 219°C und 0,1 g (16 % d. Theorie) der Verbindung I-a-2 vom Fp. 178°C.
¹H-NMR (400 MHz, d₆-DMSO): Verschiebung δ in ppm
trans Isomer
1.03 (t, 3H, CH₂-CH₃), 1.09 (s, 3H, CH₃), 1.05-1.24 (m, 2H, CH₂), 1.60-1.68 (m, 2H, CH₂), 1.74-1.78 (m, 2H, CH₂), 2.04-2.12 (m, 2H, CH₂), 2.28 (s, 3H, Ar-CH₃), 2.40-2.46 (m, 2H, Ar-CH₂-CH₃), 3.10 (s, 3H, OCH₃), 7.02 (s, 1H, Ar-H), 7.26 (s, 1H, Ar-H), 7.82 (s, br, 1H, NH), 10.52 (s, 1H, OH).
cis-Isomer
1.03 (t, 3H, CH₂-CH₃), 1.18 (s, 3H, CH₃), 1.47-1.58 (m, 2H, CH₂), 1.66-1.93 (m, 6H, CH₂), 2.28 (s, 3H, Ar-CH₃), 2.40-2.46 (m, 2H, Ar-CH₂CH₃), 3.15 (s, 3H, OCH₃), 7.02 (s, 1H, Ar-H), 7.26 (s, 1H, Ar-H), 7.72 (s, br, 1H, NH), 10.53 (s, 1H, OH).

In Analogie zu den Beispielen (I-a-1) und (I-a-2) und gemäß der allgemeinen Angaben zur Herstellung erhält man folgende Verbindungen der Formal (I-a)

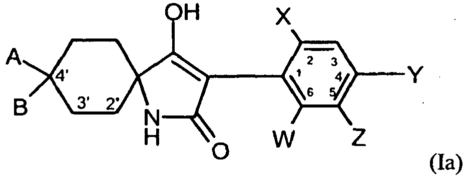

| **Bsp-Nr.** | **W** | **X** | **Y** | **Z** | **A** | **B** | **Fp. °C** | **Isomer** |
|---|---|---|---|---|---|---|---|---|
| I-a-3 | CH₃ | CH₃ | Cl | H | OCH₃ | CH₃ | 286 | cis/trans ca. 1:5 |
| I-a-4 | CH₃ | CH₃ | Br | H | OCH₃ | CH₃ | 308 | trans |
| I-a-5 | CH₃ | Cl | Cl | H | OCH₃ | CH₃ | 312 | trans |
| I-a-6 | H | CH₃ | H | CH₃ | OCH₃ | CH₃ | 100 | trans |
| I-a-7 | CH₃ | OCH₃ | Cl | H | OCH₃ | CH₃ | 239 | cis/trans ca. 1:4 |
| I-a-8 | C₂H₅ | OCH₃ | Cl | H | OCH₃ | CH₃ | 237 | trans |
| I-a-9 | C₂H₅ | OCH₃ | Cl | H | OCH₃ | CH₃ | 239 | cis/trans ca. 4:1 |
| I-a-10 | CH₃ | CH₃ | H | 4-Cl-Ph | OCH₃ | CH₃ | 254 | trans |
| I-a-11 | CH₃ | CH₃ | H | 4-F-Ph | OCH₃ | CH₃ | 265 | trans |
| I-a-12 | CH₃ | CH₃ | CH₃ | H | OCH₃ | CH₃ | 259 | trans |
| I-a-13 | CH₃ | OCH₃ | Cl | H | OC₂H₅ | CH₃ | 216 | trans |
| I-a-14 | CH₃ | OCH₃ | Cl | H | OC₂H₅ | CH₃ | 108 | cis |
| I-a-15 | H | CH₃ | H | 4-F-Ph | OCH₃ | CH₃ | 143 | trans |
| I-a-16 | CH₃ | CH₃ | H | Br | OCH₃ | CH₃ | 241 | trans |
| I-a-17 | CH₃ | CH₃ | Cl | H | OC₂Hs | CH₃ | 260 | trans |
| I-a-18 | CH₃ | CH₃ | H | 4-F-Ph | OC₂H₅ | CH₃ | 142 | cis |
| I-a-19 | CH₃ | CH₃ | H | 4-F-Ph | OC₂H₅ | CH₃ | 276 | trans |
| I-a-20 | CH₃ | CH₃ | Cl | H | OCH₃ | CH₃ | 273 | tranx |
| I-a-21 | CH₃ | CH₃ | Cl | H | OCH₃ | CH₃ | 258 | cis |
| I-a-22 | CH₃ | CH₃ | CH₃ | H | OC₂H₅ | CH₃ | 140 | trans |
| I-a-23 | CH₃ | CH₃ | CH₃ | H | OC₂H₅ | CH₃ | ¹⁾ | cis |
| I-a-24 | C₂H₅ | OCH₃ | Cl | H | OCH₃ | C₂H₅ | ²⁾ | cis |
| I-a-25 | C₂H₅ | OCH₃ | Cl | H | OCH₃ | C₂H₅ | 218 | trans |
| I-a-26 | CH₃ | CH₃ | CH₃ | H | OCH₃ | C₂H₅ | 251 | trans |
| I-a-27 | CH₃ | CH₃ | Br | H | OCH₃ | C₂H₅ | 185 | trans |
| I-a-28 | CH₃ | CH₃ | Cl | H | OCH₃ | C₂H₅ | 230 | cis |
| I-a-29 | CH₃ | CH₃ | Cl | H | OCH₃ | C₂H₅ | Zersetzung | trans |
| I-a-30 | CH₃ | CH₃ | H | 4-F-Ph | OCH₃ | C₂H₅ | Zersetzung | cis |
| I-a-31 | CH₃ | CH₃ | H | 4-F-Ph | OCH₃ | C₂H₅ | Zersetzung | trans |
| I-a-32 | C₂H₅ | OCH₃ | Cl | H | OCH₃ | C₂H₅ | 92 | cis |
| I-a-33 | C₂H₅ | OCH₃ | Cl | H | OCH₃ | C₂H₅ | 96 | trans |
| I-a-34 | CH₃ | CH₃ | H | 4-Cl-Ph | OCH₃ | C₂H₅ | 159 | trans |
| I-a-35 | CH₃ | OCH₃ | Cl | H | OCH₃ | C₂H₅ | 243 | trans |
| I-a-36 | CH₃ | OCH₃ | Cl | H | OCH₃ | C₂H₅ | Wachs | cis |
| I-a-37 | C₂Hs | Br | CH₃ | H | OCH₃ | C₂H₅ | ³⁾ | trans |
| I-a-38 | CH₃ | OCH₃ | CH₃ | H | OCH₃ | CH₃ | 212 | trans/cis 18 : 1 |
| I-a-39 | CH₃ | OCH₃ | CH₃ | H | OCH₃ | CH₃ | 300 | trans/cis 1: 2 |
| I-a-40 | CH₃ | Br | Cl | H | OCH₃ | CH₃ | 294 | cis |
| I-a-41 | CH₃ | Cl | Br | H | OCH₃ | CH₃ | 319 | cis |
| I-a-42 | C₂H₅ | OC₂H₅ | Cl | H | OCH₃ | CH₃ | 178 | trans |
| I-a-43 | H | CH₃ | Cl | H | OCH₃ | CH₃ | 219 | cis |
| I-a-44 | H | Cl | Cl | H | OCH₃ | CH₃ | 312 | cis |
| I-a-45 | C₂H₅ | C₂H₅ | CH₃ | H | OCH₃ | CH₃ | 254 | trans |
| I-a-46 | CH₃ | Br | Cl | H | OCH₃ | CH₃ | 304 | trans |
| I-a-47 | CH₃ | Cl | Br | H | OCH₃ | CH₃ | 324 | trans |
| I-a-48 | CH₃ | C₂H₅ | Br | H | OCH₃ | CH₃ | 274 | trans |
| I-a-49 | CH₃ | C₂H₅ | Br | H | OCH₃ | CH₃ | 281 | cis |
| I-a-50 | H | CH₃ | Cl | H | OCH₃ | CH₃ | 245 | trans |
| I-a-51 | H | Cl | Cl | H | OCH₃ | CH₃ | 238 | trans |
| I-a-52 | CH₃ | Cl | 4-Cl-Ph | H | OCH₃ | CH₃ | 293 | trans |
| I-a-53 | CH₃ | Cl | 4-Cl-Ph | H | OCH₃ | CH₃ | 302 | cis |
| I-a-54 | CH₃ | CH₃ | 4-Cl-Ph | H | OCH₃ | CH₃ | 237 | trans |
| I-a-55 | CH₃ | OC₂H₅ | Cl | H | OCH₃ | CH₃ | ⁴⁾ | trans |
| I-a-56 | CH₃ | CH₃ | Br | H | OC₂H₅ | CH₃ | ⁵⁾ | cis/trans ca. 7 : 1 |
| I-a-57 | CH₃ | CH₃ | Br | H | OC₂H₅ | CH₃ | ⁶⁾ | trans |
| I-a-58 | C₂H₅ | OCH₃ | Cl | H | OC₂H₅ | CH₃ | 91 | cis/trans Gemisch |
| I-a-59 | C₂H₅ | Br | CH₃ | H | OC₂H₅ | CH₃ | 115 | cis/trans Gemisch |
| I-a-60 | CH₃ | CH₃ | 4-Cl-Ph | H | OC₂H₅ | CH₃ | 251 | trans/cis ca. |
| I-a-61 | CH₃ | CH₃ | 4-Cl-Ph | H | OC₂H₅ | CH₃ | 185-195 | cis/trans ca. |
| I-a-62 | C₂H₅ | Br | CH₃ | H | OCH₃ | C₂H₅ | 122 | trans |
| I-a-63 | CH₃ | C₂H₅ | CH₃ | H | OCH₃ | C₂H₅ | 254-255 | trans |
| I-a-64 | CH₃ | C₂H₅ | CH₃ | H | OCH₃ | C₂H₅ | 81 | cis/trans ca. 2 :1 |
| I-a-65 | CH₃ | C₂H₅ | Br | H | OCH₃ | C₂H₅ | 250-252 | trans |
| I-a-66 | CH₃ | C₂H₅ | Br | H | OCH₃ | C₂H₅ | 76 | cis |
| I-a-67 | H | CH₃ | H | 4-F-Ph | OCH₃ | C₂H₅ | 115-117 | trans |
| I-a-68 | CH₃ | OCH₃ | CH₃ | H | OCH₃ | C₂H₅ | 216-219 | trans |
| I-a-69 | C₂H₅ | C₂H₅ | CH₃ | H | OCH₃ | C₂H₅ | ⁷⁾ | cis |
| I-a-70 | CH₃ | CH₃ | H | 4-F-Ph | OCH₃ | C₃H₇ | ⁸⁾ | cis |
| I-a-71 | CH₃ | CH₃ | H | 4-F-Ph | OCH₃ | C₃H₇ | ⁹⁾ | trans |

| | | | | | | | | |
|---|---|---|---|---|---|---|---|---|
| Ph = Phenyl ¹H-NMR (400 MHz, d₆-DMSO): Verschiebung δ in ppm 1) 1.09 (tr, 3H, CH₂CH₃), 1.46-1.50 (cm, 2H), 2.04 (s, 6H, 2xArCH₃), 2.22 (s, 3H, Ar-4-CH₃), 3.44 (q, 2H, O CH₂CH₃) 2) 0.77 (t, 3H, CH₂CH₃), 1.00 (tr, 3H, CH₂CH₃), 2.43 (dq, 2H, Ar-CH₂CH₃), 3.66 (s 3H, ArOCH₃) 3) 0.85 (t, 3H, CH₂CH₃), 1.03 (t, 3H, Ar-CH₂-CH₃), 1.11-1.14 (dm, 1H, CH₂), 1.21-1.24 (dm, 1H, CH₂), 1.41-1.47 (q, 2H, CH₂-CH₃), 2.28 (s, 3H, Ar-CH₃), 2.40-2.46 (dq, 2H, Ar-CH₂CH₃), 7.02 (s, 1H, Ar-H), 7.26 (s, 1H, Ar-H), 7.84 (s, br, 1H, NH), 10.52 (s, 1H, OH) 4) 1.07 (s,3H,CH₃), 1.20 (t, 3H, OCH₂CH₃), 2.09 (s, 3H, Ar-CH₃), 3.87-3.94 (m, 2H, O-CH₂-CH₃) 5) 1.09 (t, 3H, CH₂-CH₃), 1.20 (s, 3H, CH₃), 2.08 (s, 6H, Ar-CH₃), 3.41-3.46 (q, 2H, OCH₂-CH₃), 7.21 (s, 2H, Ar-H), 7.8 (s, br, 1 H, NH) 6) 1.10 (s, 3H, CH₃), 1.10 (t, 3H, CH₂CH₃), 2.08 (s, 6H, Ar-CH₃), 3.3-3.35 (q, 2H, OCH₂CH₃), 7.2 (s, 2H, Ar-H), 7.88 (s, br, 1H- NH) 7) 0.79 (t, 3H, CH₂-CH₃), 1.02 (t, 6H, Ar-CH₂-CH₃), 1.56-1.62 (q, 2H, CH₂-CH₃), 2.26 (s, 3H, Ar-CH₃), 2.33-2.37 (q, 4H, Ar-CH₂-CH₃), 3.10 (s, 3H, OCH₃), 6.84 (s, 2H, Ar-H), 7.72 (s, br, 1H, NH) 8) 0.92 (t, 3H, CH₂-CH₃), 1.97, 2.12 (2s, 6H, Ar-CH₃), 3.10 (s, 3H, OCH₃), 7.03-7.05 (d, 1H, Ar-H), 7.10-7.12 (d, 1H, Ar-H), 7.25-7.32 (m, 4H, Ar-H), 8.03 (s, br, 1H, NH) 9) 0.90 (t, 3H, CH₂-CH₃), 1.97, 2.12 (2s, 6H, Ar-CH₃), 3.05 (s, 3H, OCH₃), 7.03-7.05 (d, 1 H, Ar-H), 7.10-7.12 (d, 1 H, Ar-H), 7.12-7.32 (m, 4H, Ar-H), 8.11 (s, br, 1 H, NH). | | | | | | | | |

### Beispiel I-b-1

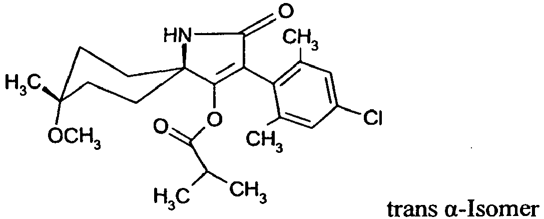

0,525g (1.5 mmol) der Verbindung gemäß Beispiel (I-a-3) werden in 20ml Essigsäure-ethylester (EE) vorgelegt, mit 0,21ml (1.5 mmol) Triethylamin und 10mg 4-N,N-Dimethyl-aminopyridin versetzt. Unter Rückfluss tropft man 0,16ml (1.5 mmol) Isobuttersäurechlorid in 1,5ml EE hinzu und rührt 2h nach. Nach dem Abkühlen wird eingeengt und der Rückstand an Kieselgel mit Methylenchlorid/Essigester 10:1 chromatographiert. Man erhält 0,45g (71% d. Theorie) vom Schmelzpunkt 236°C.
¹H-NMR (400 MHz, d₆-DMSO), Verschiebung δ in ppm
0.94-0.96 (d, 6H, CH(CH₃)₂), 1.09 (s, 3H, CH₃), 1.21-1.23 (dm, 2H, CH₂), 1.62-1.63 (tm, 2H, CH₂), 1.77-1.79 (dm, 2H, CH₂), 1.91-1.97 (tm, 2H, CH₂), 2.11 (s, 6H, ArCH₃), 2.62-2.65 (m, 1H, CH(CH₃)₂), 3.07 (s, 3H, OCH₃), 7.13 (s, 2H, Ar-H), 9.04 (s, br, 1H, NH).

In Analogie zu Beispiel (I-b-1) und gemäß den allgemeinen Angaben zur Herstellung erhält man folgende Verbindungen der Formel (I-b)

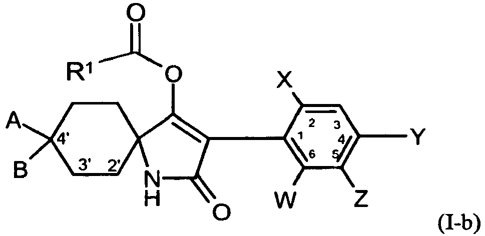

| **Bsp-Nr.** | **W** | **X** | **Y** | **Z** | **A** | **B** | **R¹** | **Fp. °C** | **Isomer** |
|---|---|---|---|---|---|---|---|---|---|
| I-b-2 | CH₃ | CH₃ | Br | H | OCH₃ | C₂H₅ | i-C₃H₇ | 228 | trans |

### Beispiel I-c-1

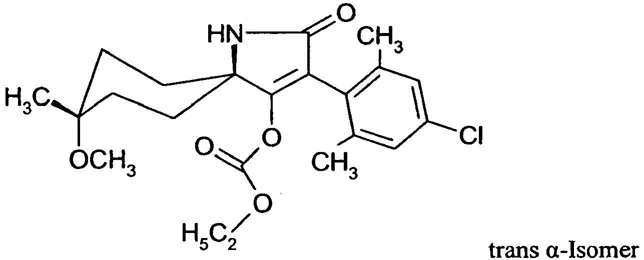

525mg (1.5 mmol) der Verbindung gemäß Beispiel (I-a-3) werden in 20ml Dichlormethan vorgelegt, mit 0,21ml (1.5 mmol) Triethylamin und 10 mg 4-N,N-Dimethylaminopyridin versetzt. Bei Raumtemperatur tropft man 0,14ml (1.5 mmol) Chlorameisensäure-ethylester in 1ml Dichlormethan zu und rührt 1h nach. Das Lösungsmittel wird abgedampft und der Rückstand an Kieselgel mit Methylenchlorid/Essigsäureethylester 10:1 chromatogrphiert.
Ausbeute: 0,35g (54% d. Theorie) vom Schmelzpunkt 238°C.
¹H-NMR (400 MHz, CD₃CN); Verschiebung δ in ppm:
1.06 (t, 3H, CH₂CH₃), 1.15 (s, 3H, CH₃), 1.34-1.39 (d, m, 2H, CH₂), 1.50-1.58 (dt, 2H, CH₂), 2.05-2.12 (dt, 2H, CH₂), 3.15 (s, 3H, OCH₃), 3.98-4.03 (q, 2H, OCH₂CH₃), 7.10 (s, 2H, Ar-H), 7.3 (s, br, 1H, NH).

In Analogie zu Beispiel (I-c-1) und gemäß den allgemeinen Angaben zur Herstellung erhält man folgende Verbindungen der Formel (I-c):

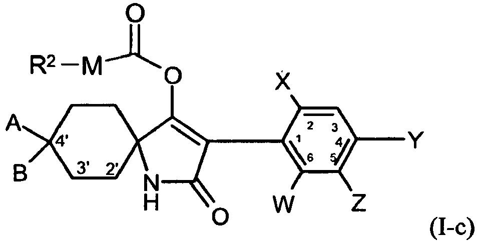

| **Bsp-Nr.** | **W** | **X** | **Y** | **Z** | **A** | **B** | **M** | **R²** | **Fp. °C** | **Isomer** |
|---|---|---|---|---|---|---|---|---|---|---|
| I-1-c-2 | CH₃ | CH₃ | CH₃ | H | OCH₃ | CH₃ | O | C₂H₅ | 178-182 | trans |
| I-1-c-3 | CH₃ | CH₃ | CH₃ | H | OCH₃ | CH₃ | O | C₂H₅ | 205-211 | cis |
| I-1-c-4 | CH₃ | CH₃ | Br | H | OCH₃ | C₂H₅ | O | C₂H₅ | 234 | trans |

### Beispiel II-1

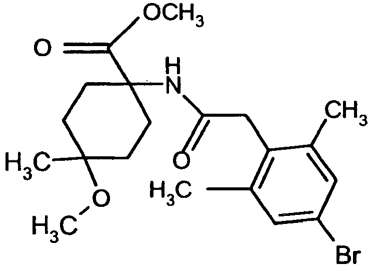

5,23 g (22 mmol) der Verbindung gemäß Beispiel (XIV-1) wird in 80 ml Tetrahydrofuran (THF) vorgelegt und mit 6.1 ml (44 mmol) Triethylamin versetzt. Anschließend gibt man unter schnellem Rühren bei 20°C 5,23 g (20 mmol) 4-Brom-2,6-dimethyl-phenyl-essigsäurechlorid gelöst in 10 ml THF zu. Nach 4 h Rühren bei 40°C wird eingeengt und der Rückstand an Kieselgel mit einem Gradienten Methylchlorid + 0→10 % Essigsäureethylester chromatographiert.
Ausbeute: 4.3 g (36 % der Theorie), Fp 158°C
¹H-NMR (400 MHz, CD₃CN): δ = 1.08, 1.10 (2s,3H,CH₃), 2.25, 2.27 (2s, 6H, ArCH₃), 3.11, 3.13 (2s, 3H, OCH₃), 3.50-3.57 (ms, 5H, CO₂CH₃, COCH₂), 7.20, 7.21 (2s, 2H, ArH) ppm.

In Analogie zu Beispiel (II-1) und gemäß den allgemeinen Angaben zur Herstellung erhält man folgende Verbindungen der Formel (II). Anreicherung der Isomeren erfolgt durch chromatographische Methoden bevorzugt an Kieselgel.

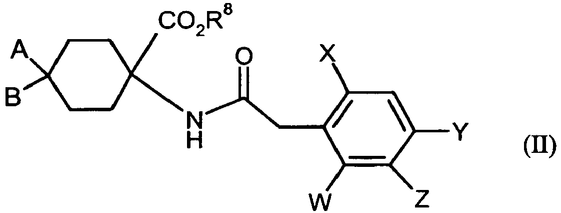

| **Bsp-Nr.** | **W** | **X** | **Y** | **Z** | **A** | **B** | **R⁸** | **Fp. °C** | **Isomer** |
|---|---|---|---|---|---|---|---|---|---|
| II-2 | CH₃ | CH₃ | Cl | H | OCH₃ | CH₃ | CH₃ | 150 | Gemisch |
| II-3 | H | CH₃ | H | CH₃ | OCH₃ | CH₃ | CH₃ | 119 | Gemisch |
| II-4 | C₂H₅ | Br | CH₃ | H | OCH₃ | CH₃ | CH₃ | 152 | cis/trans ca 1:4 |
| II-5 | CH₃ | Cl | Cl | H | OCH₃ | CH₃ | CH₃ | 144 | Gemisch |
| II-6 | CH₃ | CH₃ | CH₃ | H | OCH₃ | CH₃ | CH₃ | 135 | Gemisch |
| II-7 | C₂H₅ | Br | CH₃ | H | OCH₃ | CH₃ | CH₃ | Wachs | cis/trans ca 13:5 |
| II-8 | CH₃ | CH₃ | Cl | H | OCH₃ | CH₃ | CH₃ | 147 | cis |
| II-9 | CH₃ | CH₃ | H | 4-Cl-Ph | OCH3 | CH₃ | CH₃ | Wachs | cis/trans ca 1:4 |
| II-10 | H | CH₃ | H | 4-F-Ph | OCH₃ | CH₃ | CH₃ | 148 | Gemisch |
| II-11 | CH₃ | CH₃ | H | 4-F-Ph | OCH₃ | CH₃ | CH₃ | Wachs | Gemisch |
| II-12 | CH₃ | OCH₃ | Cl | H | OC₂H₅ | CH₃ | CH₃ | 137 | Gemisch |
| II-13 | CH₃ | OCH₃ | Cl | H | OCH₃ | CH₃ | CH₃ | Wachs | cis/trans ca 1:2 |
| II-14 | C₂H₅ | OCH₃ | Cl | H | OCH₃ | CH₃ | CH₃ | 163 | Gemisch |
| II-15 | CH₃ | CH₃ | H | 4-F-Ph | OC₂H₅ | CH₃ | CH₃ | Wachs | Gemisch |
| II-16 | CH₃ | CH₃ | CH₃ | H | OC₂H₅ | CH₃ | CH₃ | 121 | cis/trans ca 4:9 |
| II-17 | CH₃ | CH₃ | Cl | H | OC₂H₅ | CH₃ | CH₃ | 145 | Gemisch |
| II-18 | C₂H₅ | OCH₃ | Cl | H | OCH₃ | C₂H₅ | CH₃ | 122 | cis |
| II-19 | C₂H₅ | OCH₃ | Cl | H | OCH₃ | C₂H₅ | CH₃ | Wachs | trans |
| II-20 | CH₃ | OCH₃ | Cl | H | OCH₃ | C₂H₅ | CH₃ | 171 | Gemisch |
| II-21 | CH₃ | CH₃ | Cl | H | OCH₃ | C₂H₅ | CH₃ | 188 | Gemisch |
| II-22 | C₂H₅ | OC₂H₅ | Cl | H | OCH₃ | C₂H₅ | CH₃ | 170 | Gemisch |
| II-23 | CH₃ | CH₃ | CH₃ | H | OCH₃ | C₂H₅ | CH₃ | 164 | Gemisch |
| II-24 | CH₃ | CH₃ | Br | H | OCH₃ | C₂H₅ | CH₃ | 196 | Gemisch |
| II-25 | CH₃ | CH₃ | H | 4-Cl-Ph | OCH₃ | C₂H₅ | CH₃ | 109 | cis/trans ca 1:4 |
| II-26 | CH₃ | CH₃ | H | 4-F-Ph | OCH₃ | C₂H₅ | CH₃ | Wachs | cis/trans ca 1:10 |
| II-27 | C₂H₅ | Br | CH₃ | H | OCH₃ | C₂H₅ | CH₃ | 181 | trans |
| II-28 | CH₃ | CH₃ | Cl | H | OCH₃ | CH₃ | CH₃ | ¹⁾ | trans |
| II-29 | CH₃ | OCH₃ | CH₃ | H | OCH₃ | CH₃ | CH₃ | 160 | Gemisch |
| II-30 | CH₃ | Cl | Br | H | OCH₃ | CH₃ | CH₃ | 156 | Gemisch |
| II-31 | CH₃ | Br | Cl | H | OCH₃ | CH₃ | CH₃ | 166 | Gemisch |
| II-32 | CH₃ | C₂H₅ | Br | H | OCH₃ | CH₃ | CH₃ | 150 | Gemisch |
| II-33 | C₂H₅ | C₂H₅ | CH₃ | H | OCH₃ | CH₃ | CH₃ | 136 | Gemisch |
| II-34 | C₂H₅ | OC₂H₅ | Cl | H | OCH₃ | CH₃ | CH₃ | 167 | Gemisch |
| II-35 | H | CH₃ | Cl | H | OCH₃ | CH₃ | CH₃ | 134 | Gemisch |
| II-36 | H | Cl | Cl | H | OCH₃ | CH₃ | CH₃ | 149 | Gemisch |
| II-37 | C₂H₅ | Br | CH₃ | H | OC₂H₅ | CH₃ | CH₃ | 129-130 | Gemisch cis/trans ca. 3 : 5 |
| II-38 | CH₃ | CH₃ | H | 4-Cl-Ph | OC₂H₅ | CH₃ | CH₃ | Wachs | Gemisch cis/trans ca. 3 : 4 |
| II-39 | CH₃ | CH₃ | Br | H | OC₂H₅ | CH₃ | CH₃ | 154-155 | Gemisch cis/trans ca. 1 : 3 |
| II-40 | CH₃ | OC₂H₅ | Cl | H | OCH₃ | CH₃ | CH₃ | Wachs | Gemisch |
| II-42 | CH₃ | OCH₃ | Cl | H | OC₂H₅ | CH₃ | CH₃ | Wachs | Gemisch |
| II-43 | C₂H₅ | Br | CH₃ | H | OCH₃ | C₂H₅ | CH₃ | Wachs | trans |
| II-44 | CH₃ | OCH₃ | CH₃ | H | OCH₃ | C₂H₅ | CH₃ | 149-151 | Gemisch |
| II-45 | H | CH₃ | H | 4-F-Ph | OCH₃ | C₂H₅ | CH₃ | 60-62 | Gemisch |
| II-46 | CH₃ | C₂H₅ | Br | H | OCH₃ | C₂H₅ | CH₃ | 183 | Gemisch |
| II-47 | CH₃ | C₂H₅ | CH₃ | H | OCH₃ | C₂H₅ | CH₃ | Wachs | Gemisch |
| II-48 | C₂H₅ | C₂H₅ | CH₃ | H | OCH₃ | C₂H₅ | CH₃ | 166-168 | Gemisch |
| II-49 | CH₃ | CH₃ | H | 4-F-Ph | OCH₃ | C₃H₇ | CH₃ | Wachs | Gemisch |

| | | | | | | | | | |
|---|---|---|---|---|---|---|---|---|---|
| ^{1) 1}H-NMR (400 MHz, CD3CN): Verschiebung δ in ppm: 1.09 (s, 3H, CH₃), 1.34-1.42 (tm, 2H, CH₂), 2.27 (s, 6H, AR-CH₃), 3.11 (s, 3H, OCH₃), 3.56 (s, 5H, 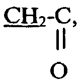 CO₂CH₃), 6.56 (s, br, 1H, NH), 7.07 (s, 2H, Ar-H) | | | | | | | | | |

### Beispiel XIV-1

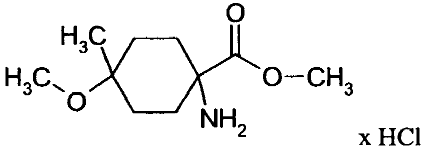

7.2 g (32 mmol) der Verbindung gemäß Bsp. XVII-1 werden unter Argon in 150 ml Methanol bei 0 bis 5°C vorgelegt. Es wird 10 ml Thionylchlorid zugetropft und 30 Minuten bei 0°C gerührt und 24 h bei 40°C, bis eine klare Lösung entsteht. Anschließend kühlt man auf 5°C und saugt den Niederschlag ab. Die Lösung wird einrotiert und der Rückstand mit Methylenchlorid/Hexan zur Kristallisation gebracht.
Ausbeute: 5.9 g (76 % d. Theorie)
¹H-NMR (400 MHz, d₆-DMSO): δ = 1.07, 1.10 (2s, 3H, CH₃), 3.08, 3.09 (2s, 3H, OCH₃), 3.75, 3.76 (s, 3H, CO₂CH₃) ppm.

### Beispiel XVII-1

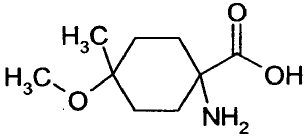

Es werden 6.85 g der Verbindung gemäß Beispiel XVIII-1 in 80 ml 30 %ige KOH und Argon suspendiert und am Rückfluß über Nacht gerührt.

Es wird auf ca 25 % des Volumens einrotiert; bei 0 - 10°C mit HCI-konz. auf pH 2 gestellt. Die Lösung wird einrotiert und getrocknet. Der Rückstand wird direkt in die Veresterung zu XIV-I eingesetzt.

### Beispiel XVIII-1

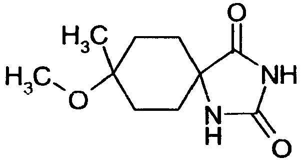

In 120 ml Wasser werden Ammoniumcarbonat (27 g) und Natriumcyanid (2.92 g) vorgelegt. Bei Raumtemperatur beginnend wird 7.7 g 4-Methoxy-4-methyl-cyclohexanon zugetropft und die Reaktionsmischung über vier Stunden bei 55°C bis 60°C gerührt, auf 50 ml eingeengt, dann bei 0° bis 5°C zwei Stunden gerührt, bei ca. -2°C abgesaugt und mit wenig Eiswasser nachgewaschen, getrocknet.

Ausbeute: 6.9 g (52 % d. Theorie) cis/trans Isomerengemisch ca 1:2 nach NMR, ¹H-NMR (400 MHz, d₆-DMSO): δ = 1.06, 1.11 (2s, 3H, CH₃), 3.08, 3.10 (2s, 3H, OCH₃), 7.86, 8.21 (s, br, 1H, NH).

### Herstellung von 4-Methoxy-4-methyl-cyclohexanon (gemäß Wulff, D et.al, Synthesis 1999, 415-422)

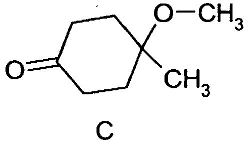

Im 600 ml Becherglas werden 8.9 g der Verbindung B in 50 ml THF und 70 ml Wasser mit 9 ml HCl-konz. versetzt. Man rührt 2 Std. bei 20°C und neutralisiert mit 20%iger NaOH auf pH 7. Man rotiert ein und extrahiert mit MtB-Ether/Wasser; die Organische Phase wird mit gesättigter NaCl-Lösung gewaschen, getrocknet und einrotiert.

Man erhält 7.7 g (= 82.7 % d. Theorie) 4-Methoxy-4-methyl-cyclohexanon in einer Reinheit von 62 % nach GC/MS, welches man ohne weitere Aufreinigung direkt in die Synthese von (XXI-1) einsetzt.

### Herstellung von B

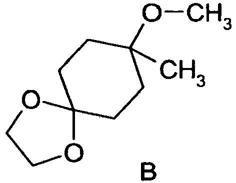

In einem 100 ml Dreihalskolben werden unter Argon 3.5 g Natriumhydrid 3 x mit 20 ml Hexan pa. verrührt (Hexan mit Pipette abziehen). Es wird mit 20 ml THF versetzt. Bei 20°C tropft man 7.6 g der Verbindung A in 80 ml THF zu, rührt 30 Min. bei 20°C nach und anschließend gibt man 11 ml Methyljodid und 1.65 g Tetramethylammoniumbromid schnell zu.

Man rührt über Nacht bei 20°C. Bei 0°C tropft man langsam 10 ml Isopropanol zu. Es wird mit MtB-Ether/Wasser extrahiert; die Organische Phase wird mit gesättigter NaCl-Lösung gewaschen, getrocknet, einrotiert. Man erhält 9 g (= 80 % der Theorie) in einer Reinheit von 73 % nach GC/MS. Das Produkt wird ohne weitere Aufreinigung mit Salzsäure zu C deketalisiert.

### Herstellung von A

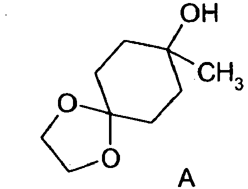

In einem 4000 ml Dreihalskolben werden unter Argon 1000 ml Toluol und 1000 ml 1 Molare Methylmagnesiumbromid-Lösung in THF vorgelegt. Bei 0 bis 5°C tropft man 156.2 g 1.4-Cyclohexandion-monoethylenglykol-ketal in 130 ml THF innerhalb 2 Std. zu. Man rührt 4 Std. bei 0 bis 5°C nach und versetzt dann mit 200 ml NH₄Cl-Lösung. Die Phasen werden getrennt; die Wasserphase mit CH₂Cl₂ extrahiert; die Organische Phasen mit MgSO₄ getrocknet.

Bei Normaldruck wird das Lösemittel abdestilliert und der Rückstand an einer 10 cm Vigreux-Kolonne bei 1 mbar/110-115°C destilliert.
Man erhält 152.3 g (= 88 % der Theorie).

### Beispiel 1

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 l/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

Folgende Verbindungen zeigen neben den zuvor genannten Verbindungen im Vorauflauf mit 320 g/ha a.i. gegen Echinocloa crus-qalli, Lolium multiflorum und Setaria viridis eine Wirkung von ≥ 80 %: I-a-1, I-a-2, I-a-7, I-a-8, I-a-9, I-a-10, I-a-24, I-a-25, I-a-32, I-a-33, I-a-37, I-a-41, I-a-48, I-a-49, I-a-52, I-a-53, I-a-54, I-a-58, I-a-59, I-a-63, I-a-64, I-a-66, I-a-69

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

Neben den zuvor genannten Verbindungen zeigen folgende Verbindungen im Nachauflauf mit 80 g/ha gegen Alopecurus myosuroides, Echinocloa crus-galli, Lolium multiflorum und Setaria viridis eine Wirkung von ≥ 80%: I-a-1, I-a-2, I-a-9, I-a-22, I-a-25, I-a-32, I-a-33, I-a-37, I-a-38, I-a-41, I-a-42, I-a-45, I-a-48, I-a-49, I-a-58, I-a-59, I-a-62, I-a-63, I-a-64, I-a-69.

### Verwendung von Safenern:

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

### Gefäßversuche mit Getreide im Gewächshaus

### Mefenpyr 1 Tag vor Herbizidapplikation

**Tabelle 1**

| | | 10 Tage nach Applikation | | 10 Tage nach Applikation |
|---|---|---|---|---|
| | Aufwandmenge g a.i./ha | Sommergerste beobachtet (%) | Aufwandmeng e g a.i./ha | Sommerweizen beobachtet (%) |
| (I-a-2) | 100 | 30 | 100 | 60 |
| | 50 | 10 | 50 | 60 |
| | | | 25 | 50 |
| | | | 12,5 | 30 |
| (I-a-2) + Mefenpyr | 100 + 50 | 2 | 100 + 50 | 30 |
| | 50 + 50 | 2 | 50 + 50 | 10 |
| | | | 25 + 50 | 5 |
| | | | 12,5 + 50 | 3 |

**Tabelle 2**

| | | 28 Tage nach Applikation | | 10 Tage nach Applikation |
|---|---|---|---|---|
| | Aufwandmenge ga.i./ha | Sommergerste beobachtet (%) | Aufwandmeng e g a.i./ha | Sommerweizen beobachtet (%) |
| (I-a-9) | 100 | 50 | 100 | 30 |
| | 50 | 20 | 50 | 30 |
| | | | 25 | 20 |
| (I-a-9) + Mefenpyr | 100 + 50 | 8 | 100 + 50 | 10 |
| | 50 + 50 | 5 | 50 + 50 | 10 |
| | | | 25 + 50 | 5 |

**Tabelle 3**

| | | 10 Tage nach Applikation | 28 Tage nach Applikation |
|---|---|---|---|
| | Aufwandmenge g a.i./ha | Sommerweizen beobachtet (%) | Sommerweizen beobachtet (%) |
| (I-a-2) | 100 | 50 | 60 |
| | 50 | 50 | 60 |
| | 25 | 40 | 30 |
| | 12,5 | 40 | 10 |
| (I-a-2) + Mefenpyr | 100 + 50 | 20 | 5 |
| | 50 + 50 | 10 | 2 |
| | 25 + 50 | 8 | 0 |
| | 12,5 + 50 | 5 | 0 |

**Tabelle 4**

| | | 10 Tage nach Applikation | 10 Tage nach Applikation |
|---|---|---|---|
| | Aufwandmenge g a.i./ha | Sommergerste beobachtet (%) | Sommerweizen beobachtet (%) |
| (I-a-49) | 100 | 70 | 50 |
| | 50 | 50 | 50 |
| | 25 | 50 | 50 |
| | 12,5 | 40 | 40 |
| (I-a-49) + Mefenpyr | 100 + 50 | 40 | 30 |
| | 50 + 50 | 20 | 30 |
| | 25 + 50 | 20 | 20 |
| | 12,5 + 50 | 5 | 20 |

**Tabelle 5**

| | | 28 Tage nach Applikation |
|---|---|---|
| | Aufwandmenge g a.i./ha | Sommerweizen beobachtet (%) |
| (I-a-64) | 50 | 70 |
| | 25 | 60 |
| | 12,5 | 20 |
| (I-a-64) + Mefenpyr | 50 + 50 | 20 |
| | 25 + 50 | 10 |
| | 12,5 + 50 | 0 |

**Tabelle 6**

| | | 28 Tage nach Applikation | 10 Tage nach Applikation |
|---|---|---|---|
| | Aufwandmenge g a.i./ha | Sommergerste beobachtet (%) | Sommerweizen beobachtet (%) |
| (I-a-69) | 100 | 15 | 40 |
| | 50 | 10 | 40 |
| | 25 | 10 | 30 |
| | 12,5 | | 20 |
| (I-a-69) + Mefenpyr | 100 + 50 | 5 | 15 |
| | 50 + 50 | 3 | 15 |
| | 25 + 50 | 0 | 10 |
| | 12,5 + 50 | | 10 |

### Beispiel 2

### Phaedon-Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp Nr : I-a-3, I-a-4, I-a-5, I-a-10, I-a-15, I-a-12, I-a-14, I-a-13, I-a-17, I-a-19, I-a-18, I-a-26, I-a-20, I-a-22, I-a-27, I-a-28, I-a-29, I-a-34, I-a-36, I-a-38, I-a-39, I-a-47, I-a-49, I-a-50, I-a-51, I-a-63, I-a-64

### Beispiel 3

### Spodoptera frugiperda-Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele eine Wirkung von ≥ 80 % bei einer Aufwandmenge von 500 g/ha:
Bsp Nr : I-a-10, I-a-15, I-a-34, I-a-61, I-a-71, I-c-2

### Beispiel 4

### Myzus persicae-Test (MYZUPE Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp Nr : I-a-3, I-a-4, I-a-5, I-a-6, I-a-7, I-a-8, I-a-9, I-a-2, I-a-1, I-a-10, I-a-11, I-a-15, I-a-12, I-a-14, I-a-13, I-a-16, I-a-17, I-a-19, I-a-18, I-a-24, I-a-20, I-a-21, I-a-22, I-a-23, I-a-26, I-a-27, I-a-29, I-a-30, I-a-31, I-a-33, I-a-34, I-a-35, I-a-36, I-a-37, I-a-38, I-a-39, I-a-40, I-a-41, I-a-42, I-a-43, I-a-44, 1-a-45, I-a-46, I-a-47, I-a-48, I-a-49, I-a-50, I-a-51, I-a-52, I-a-53, I-a-54, I-a-55, I-a-57, I-a-58, I-a-59, I-a-60, I-a-61, I-a-62, I-a-63, I-a-64, I-a-65, I-a-66, I-a-67, I-a-69, I-a-70, I-a-71, 1-b-1, I-c-1, I-c-2, I-c-3

### Beispiel 5

### Tetranychus- Test, OP-resistent (TETRUR Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

### RPI 2008-65.doc

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 100 g/ha :
Bsp Nr : I-a-3, I-a-4, I-a-5, I-a-2, I-a-10, I-a-11, I-a-15, I-a-12, I-a-13, I-a-19, I-a-18, I-a-26, I-a-22, I-a-23, I-a-27, I-a-30, I-a-31, I-a-35, I-a-36, I-a-38, I-a-39, I-a-40, I-a-41, I-a-43, I-a-44, I-a-47, 1-a-51, I-a-52, I-a-53, I-a-60, I-a-61, I-a-68, I-a-71 I-b-1, I-c-1, I-c-2, I-c-3

### Beispiel 6

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm :
Bsp Nr: I-a-3

### Beispiel 7

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach der gewünschten Zeit wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele gute Wirksamkeit bei einer Aufwandmenge von 20µg / Tier : siehe Tabelle
Bsp Nr : I-a-3, I-c-3

### Beispiel 8

### Heliothis virescens - Test - Behandlung transgener Pflanzen

| | |
|---|---|
| Lösungsmittel: | 7 Gewichtsteile Aceton |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) einer transgenen Sorte werden durch Besprühen mit der Wirkstoffzubereitung in der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

### Beispiel 9

### Grenzkonzentrations-Test / Bodeninsekten - Behandlung transgener Pflanzen

| | |
|---|---|
| Testinsekt: | Diabrotica balteata - Larven im Boden |
| Lösungsmittel: | 7 Gewichtsteile Aceton |
| | |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und lässt diese bei 20°C stehen.

Sofort nach dem Ansatz werden je Topf 5 vorgekeimte Maiskörner einer transgenen Sorte gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

## Patentansprüche

1. Verbindungen der Formel (I) 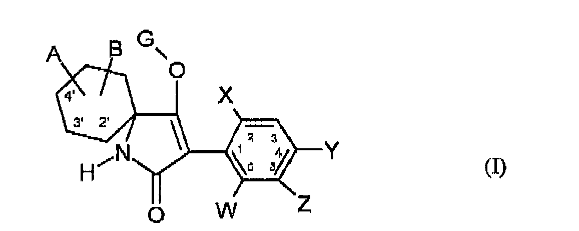 in welcher
W für Wasserstoff, Halogen, Alkyl, Alkenyl, Alkinyl, gegebenenfalls substituiertes Cycloalkyl, Alkoxy, Alkenyloxy, Halogenalkyl, Halogenalkoxy oder Cyano steht,
X für Halogen, Alkyl, Alkenyl, Alkinyl, gegebenenfalls substituiertes Cycloalkyl, Alkoxy, Alkenyloxy, Alkylthio, Alkylsulfinyl, Alkylsulfonyl, Halogenalkyl, Halogenalkoxy, Halogenalkenyloxy, Nitro oder Cyano steht,
Y und Z unabhängig voneinander für Wasserstoff, Alkyl, Alkenyl, Alkinyl, gegebenenfalls substituiertes Cycloalkyl, Alkoxy, Halogen, Halogenalkyl, Halogenalkoxy, Cyano, Nitro oder jeweils gegebenenfalls substituiertes Aryl oder Hetaryl stehen,
A für Alkoxy steht,
B für Alkyl steht, wobei
A und B an das selbe Kohlenstoffatom gebunden sind,
G für Wasserstoff (a) oder für eine der Gruppen 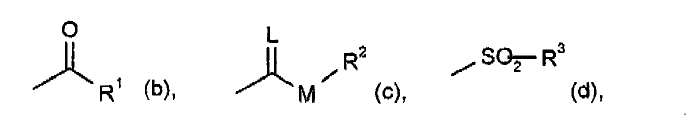 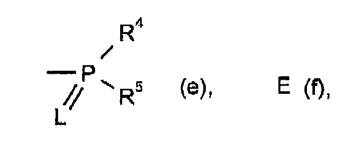
oder 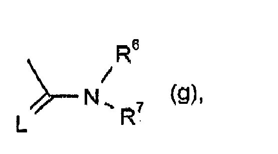 steht, worin
E für ein Metallion oder ein Ammoniumion steht,
L für Sauerstoff oder Schwefel steht,
M für Sauerstoff oder Schwefel steht,
R¹ für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxy-alkyl steht,
R² für jeweils gegebenenfalls durch Halogen oder Cyano substituiertes Alkyl, Alkenyl, Alk-oxyalkyl oder Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
R⁶ und R⁷ unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzoyl stehen, oder gemeinsam mit dem N-Atom, an das sie gebunden sind, einen gegebenenfalls Sauerstoff oder Schwefel enthaltenden und gegebenenfalls substituierten Cyclus bilden.

2. Verbindungen der Formel (I) gemäß Anspruch 1, in welcher
W für Wasserstoff, Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₄-Halogen-alkyl, C₁-C₄-Halogenalkoxy oder Cyano steht,
X für Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Halogenalkyl, C₁-C₆-Alkoxy, C₃-C₆-Alkenyloxy, C₁-C₆-Alkylthio, C₃-C₆-Alkylsulfinyl, C₁-C₆-Alkylsulfonyl, C₁-C₆-Halo-genalkoxy, C₃-C₆-Halogenalkenyloxy, Nitro oder Cyano steht,
Y und Z unabhängig voneinander für Wasserstoff, Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₆-Halogenalkyl, C₁-C₆-Halogenalkoxy, Cyano, C₂-C₆-Alkenyl, C₂-C₆-Alkinyl oder für einen der (Het)-arylreste stehen 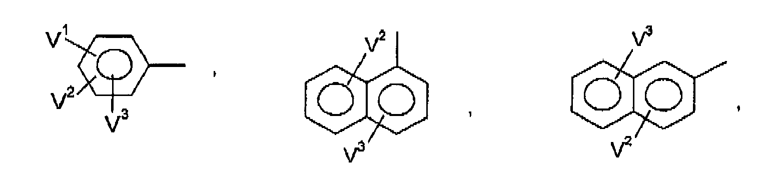 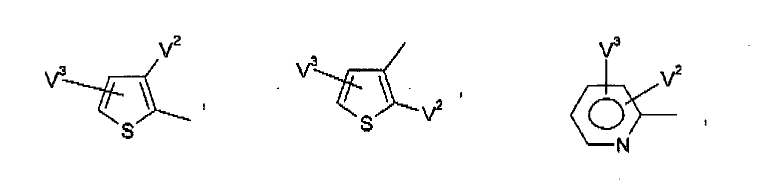 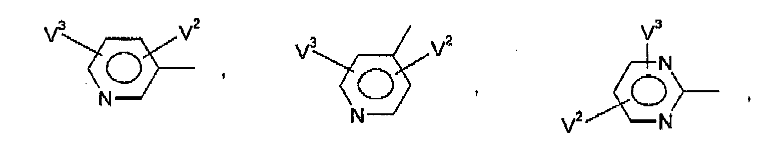 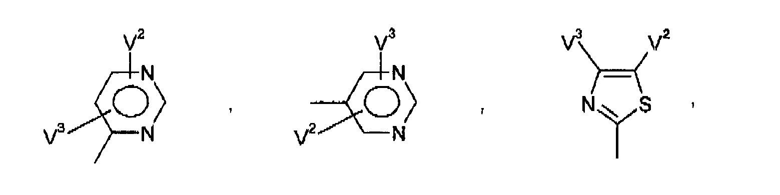 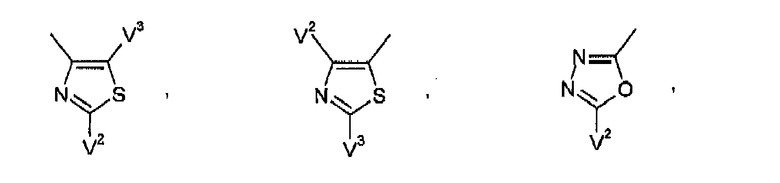 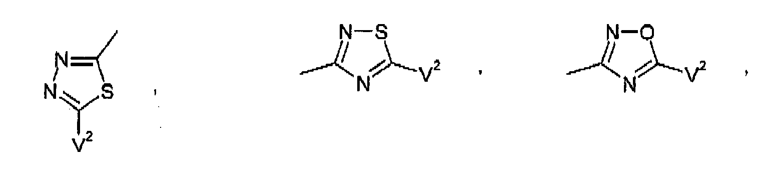 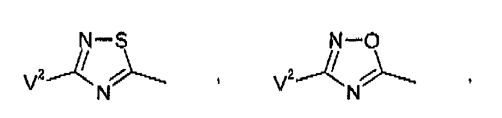 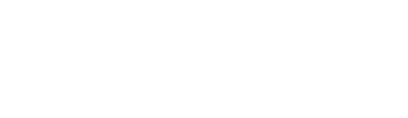
wobei im Falle von (Het)-aryl nur einer der Reste Y oder Z für (Hetaryl stehen darf,
V¹ für Wasserstoff, Halogen, C₁-C₁₂-Alkyl, C₁-C₆-Alkoxy, C₁-C₆-Atkylthio, C₁-C₆-Alkylsulfonyl, C₁-C₆-Alkylsulfonyl, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio steht,
V² und V³ unabhängig voneinander für Wasserstoff, Halogen, C₁-C₆-Alkyl, C₁-C₆-Alkoxy, C₁-C₄-Halogenalkyl oder C₁-C₄-Halogenalkoxy stehen,
A für C₁-C₆-Alkoxy steht,
B für C₁-C₆-Alkyl steht
wobei A und B an das selbe Kohlenstoffatom gebunden sind,
G für Wasserstoff (a) oder für eine der Gruppen 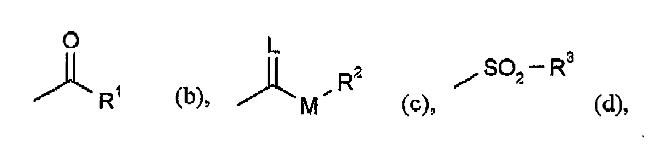 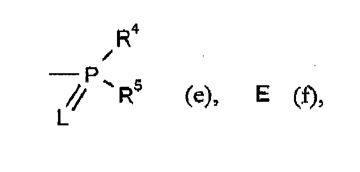
oder 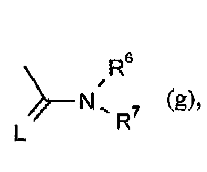 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls durch Halogen oder Cyano substituiertes C₁-C₂₀-Alkyl, C₂-C₂₀-.Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nacht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Allkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen, oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
R² für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist.

3. Verbindungen der Formel (I) gemäß Anspruch 1, in welcher
W für Wasserstoff, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₂-Halogenalkyl oder C₁-C₂-Halogenalkoxy steht,
X für Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₄-Halogenalkyl, C₁-C₄-Halogenalkoxy oder Cyano steht,
Y und Z unabhängig voneinander für Wasserstoff, Fluor, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₆-Alkoxy, C₁-C₄-Halogenalkyl, C₁-C₄-Halogenalkoxy, Cyano, C₂-C₄-Alkenyl, C₂-C₄-Alkinyl oder für einen der (Het)-arylreste stehen, 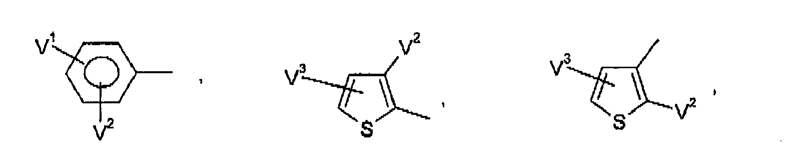 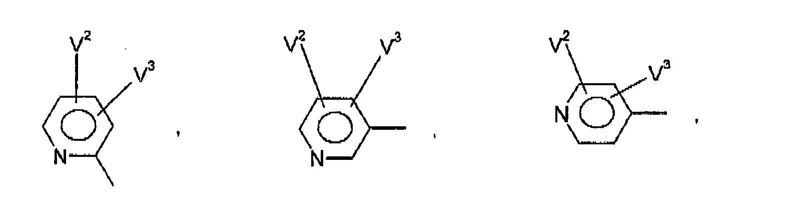 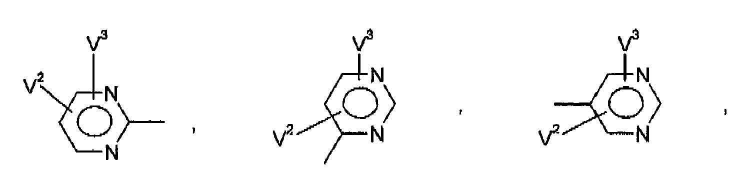
wobei im Falle von (Het)-aryl nur einer der Reste Y oder Z für (Het)-aryl stehen darf,
V¹ für Wasserstoff, Fluor, Chlor, Brom, C₁-C₆-Allcyl, C₁-C₄-Alkoxy, C₁-C₂- Halogenalkyl, C₁-C₂-Halogenalkoxy, Nitro, Cyano oder gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl steht,
V² und V³ unabhängig voneinander für Wasserstoff, Fluor, Chlor, Brom, C₁-C₄-Alkyl, C₁-C₄-Alkoxy, C₁-C₂~Halogenalkyl oder C₁-C₂-Halogenalkoxy stehen,
A für C₁-C₄-Alkoxy steht,
B für C₁-C₄-Alkyl steht,
wobei A und B an das selbe Kohlenstoffatom gebunden sind,
G für Wasserstoff (a) oder für eine der Gruppen 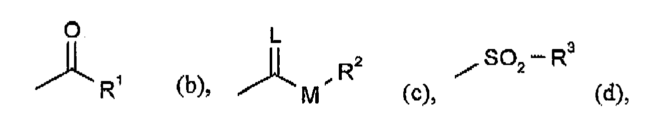 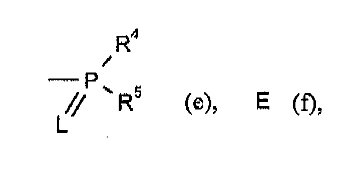
oder 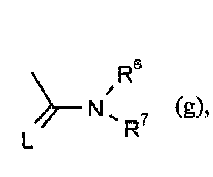 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht,
R⁴ und R⁵ unabhängig voneinander für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, oder C₁-C₃-Halogenalkyl, substituiertes Phenyl, Phenoxy oder Phenylthio stehen,
R⁶ und R⁷ unabhängig voneinander für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist.

4. Verbindungen der Formel (I) gemäß Anspruch 1, in welcher
W für Wasserstoff, Chlor, Brom, Methyl, Ethyl, Vinyl, Ethinyl, Propinyl, Cyclopropyl, Methoxy, Ethoxy oder Trifluormethyl steht,
X für Chlor, Brom, Methyl, Ethyl, Propyl, iso-Propyl, Vinyl, Ethinyl, Propinyl, Cyclopropyl, Methoxy, Ethoxy, Trifluormethyl, Difluormethoxy, Trifluormethoxy oder Cyano steht,
Y und Z unabhängig voneinander für Wasserstoff, Fluor, Chlor, Brom, Iod, Methyl, Ethyl, Vinyl, Ethinyl, Propinyl, Cyclopropyl, Methoxy, Trifluormethyl, Trifluormethoxy, Cyano oder einen Phenylrest stehen 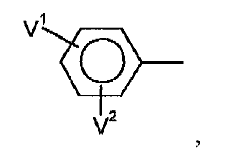
wobei im Falle von Phenyl nur einer der Reste Y oder Z für Phenyl stehen darf,
V¹ für Wasserstoff, Fluor oder Chlor steht,
V² für Wasserstoff, Fluor, Chlor, Methyl, Ethyl, n-Propyl, iso-Propyl, Methoxy, Ethoxy oder Trifluormethyl steht,
A für Methoxy, Ethoxy oder Propoxy steht,
B für Methyl, Ethyl oder Propyl steht,
wobei A und B an das selbe Kohlenstoffatom gebunden sind,
G für Wasserstoff (a) oder für eine der Gruppen 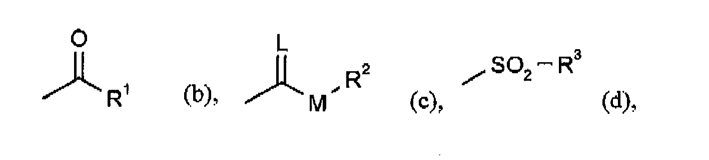 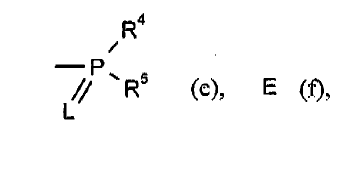
oder 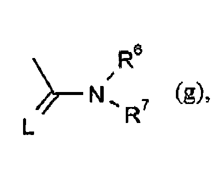 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl, oder Pyridyl steht,
R² für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl steht,
R³ für jeweils gegebenenfalls einfach bis dreifach durch Fluor oder Chlor substituiertes Methyl, Ethyl, Propyl oder iso-Propyl, oder jeweils gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl steht,
R⁴ und R⁵ unabhängig voneinander für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl Phenoxy oder Phenylthio stehen,
R⁶ und R⁷ unabhängig voneinander für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für gegebenenfalls einfach bis zweifach durch Fluor, Chlor, Brom, Methyl, Methoxy oder Trifluormethyl substituiertes Phenyl, oder zusammen für einen C₅-C₆-Alkylenrest stehen, in welchem gegebenenfalls eine Methylengruppe durch Sauerstoff oder Schwefel ersetzt ist.

5. Verbindungen der Formel (I) gemäß Anspruch 1, in welcher
W für Wasserstoff, Chlor, Brom, Methyl, Ethyl oder Methoxy steht,
X für Chlor, Brom, Methyl, Ethyl,Methoxy oder Ethoxy steht,
Y und Z unabhängig voneinander für Wasserstoff, Chlor, Brom, Methyl oder für den Rest 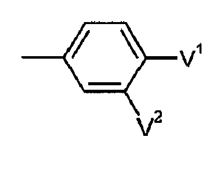
stehen,
wobei in diesem Falle nur einer der Reste Y oder Z für 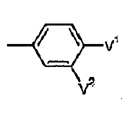 stehen darf,
V¹ für Fluor oder Chlor steht,
V² für Wasserstoff, Fluor oder Chlor steht,
A for Methoxy oder Ethoxy steht,
B für Methyl, Ethyl oder Propyl steht,
wobei A und B an das selbe Kohlenstoffatom in der 4'-Position gebunden sind,
G für Wasserstoff (a) oder für eine der Gruppen 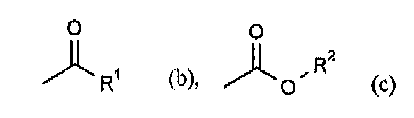
oder E (f) steht
in welchen
E für ein Metallion oder ein Ammoniumion steht,
R¹ für C₁-C₁₀-Alkyl, C₁-C₄-Alkoxy-C₁-C₂-alkyl, C₃-C₆-Cycloalkyl, für gegebenenfalls einfach durch Chlor substituiertes Phenyl oder für Thienyl, steht,
R² für C₁-C₁₀-Alkyl, C₂-C₁₀-Alkenyl oder für Benzyl steht.

6. Verfahren zur Herstellung von Verbindungen der Formel (I) gemäß Anspruch 1, **dadurch gekennzeichnet, dass** man zum Erhalt von
(A) Verbindungen der Formel (I-a) 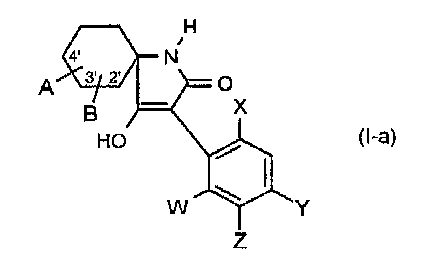 in welcher
A, B, W, X, Y und Z die oben angegebenen Bedeutungen haben,
Verbindungen der Formel (II) 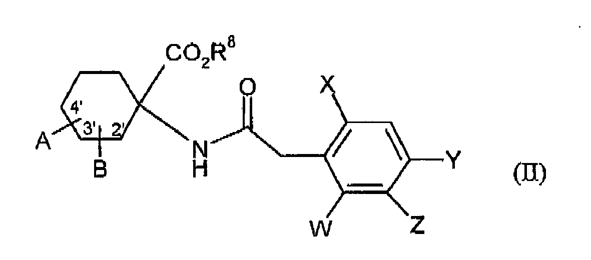 in welcher
A, B, W, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formeln (I-b) 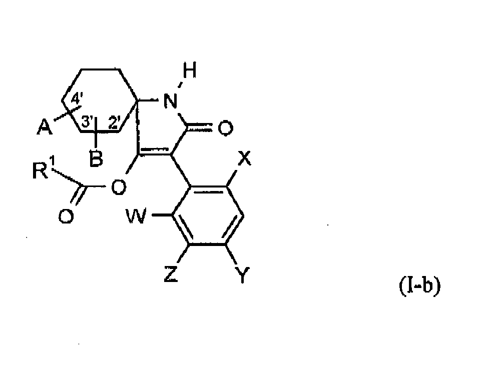 in welchen R¹, A, B, W, X, Y und Z die oben angegebenen Bedeutungen haben, Verbindungen der oben gezeigten Formeln (I-a), in welchen A, B, W, X, Y und Z die oben angegebenen Bedeutungen haben, jeweils
α) mit Verbindungen der Formel (III) 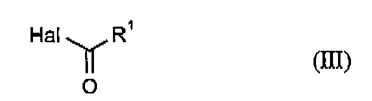 in welcher
R¹ die oben angegebene Bedeutung hat und
Hal für Halogen steht
oder
β) mit Carbansäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels umsetzt;
(C) Verbindungen der Formeln (I-c) 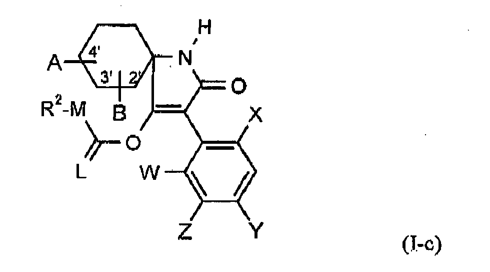 , in welchen R², A, B, M, W, X, Y und Z die oben angegebenen Bedeutungen haben und L für Sauerstoff steht, Verbindungen der oben gezeigten Formeln (I-a), in welchen A, B, W, X, Y und Z die oben angegebenen Bedeutungen haben, jeweils
mit Chlorameisensäureestern oder Chlorameisensäurethioestern der Formel (V)
R²-M-CO-Cl (V)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(D) Verbindungen der oben gezeigten Formeln (I-c), in welchen R², A, B, M, W, X, Y und Z die oben angegebenen Bedeutungen haben und L für Schwefel steht, Verbindungen der oben gezeigten Formeln (I-a), in welchen A, B, W, X, Y und Z die oben angegebenen Bedeutungen haben, jeweils
mit Chlormonothioameisensäureestem oder Chlordithioameisensäureestern der Formel (VII) 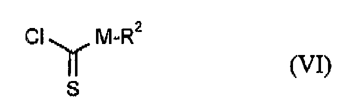 in welcher
M und R² die oben angegebenen Bedeutungen haben,
gegebenenfalls in Gegenwart eines Verdünnungsmittels und gegebenenfalls in Gegenwart eines Säurebindemittels umsetzt,
(E) Verbindungen der Formeln (I-d) 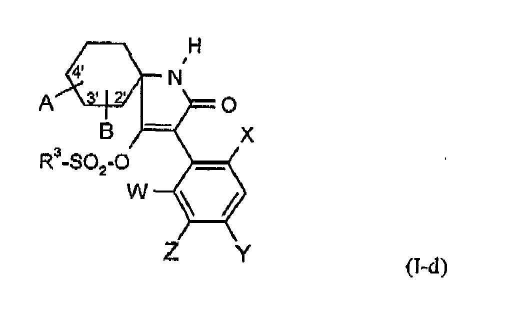 in welchen R³, A, B, W, X, Y und Z die oben angegebenen Bedeutungen haben, Verbindungen der oben gezeigten Formeln (I-a), in welchen A, B, W, X, Y und Z die oben angegebenen Bedeutungen haben, jeweils
mit Sulfonsäurechloriden der Formel (VII)
R³-SO₂-Cl (VII)
in welcher
R³ die oben angegebene Bedeutung hat,
gegebenenfalls in Gegenwart: eines Verdünnungsmittels und gegebenenfalls in Gegenwart eines Säurebindemittels umsetzt,
(F) Verbindungen der Formeln (I-e) 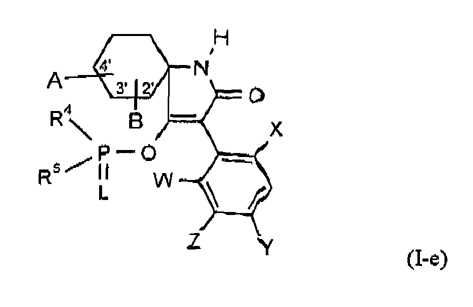 in welchen L, R⁴, R⁵, A, B, W, X, Y und Z die oben angegebenen Bedeutungen haben, Verbindungen der oben gezeigten Formeln (I-a), in welchen A, B, W, X, Y und Z die oben angegebenen Bedeutungen haben, jeweils
mit Phosphorverbindungen der Formel (VIII) 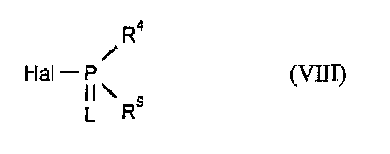 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(G) Verbindungen der Formeln (I-f) 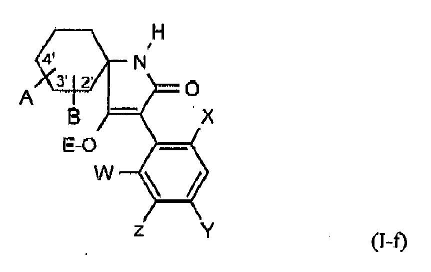 in welchen E, A, B, W, X, Y und Z die oben angegebenen Bedeutungen haben, Verbindungen der Formeln (I-a), in welchen A, B, W, X, Y und Z die oben angegebenen Bedeutungen haben, jeweils
mit Metallverbindungen oder Aminen der Formeln (IX) oder (X)
Me(OR¹⁰)ₜ (IX) 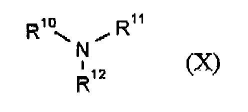
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(H) Verbindungen der Formeln (I-g) 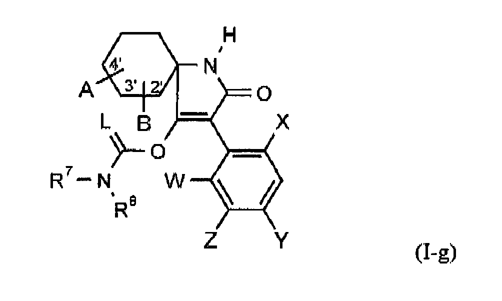 in welchen L, R⁶, R⁷, A, B, W, X, Y und Z die oben angegebenen Bedeutungen haben, Verbindungen der oben gezeigten Formeln (I-a), in welchen A, B, W, X, Y und Z die oben angegebenen Bedeutungen haben, jeweils
α) mit Isocyanaten oder Isothiocyanaten der Formel (XI)
R⁶-N=C=L (XI)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
β) mit Carbamidsäurechloriden oder Thiocarbamidsäurechloriden der Formel (XII) 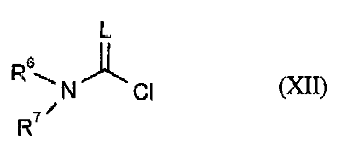 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt,
(Iα) Verbindungen der oben gezeigten Formeln (I-a) bis (I-g), in welchen A, B, G, W, X, Y und Z die oben angegebene Bedeutung haben, Verbindungen der Formeln (I-a') bis (I-g'), in welchen, B, G, W, X und Y die oben genannte Bedeutung haben und Z' bevorzugt für Brom oder Iod steht 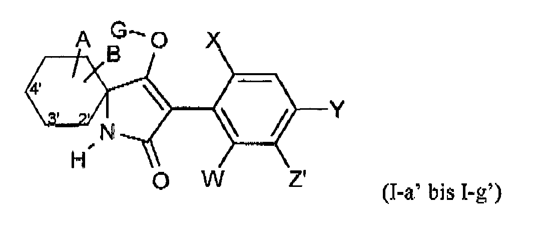 und
(Iβ) dass man Verbindungen der oben gezeigten Formeln (I-a) bis (I-g), in welchen A, B, G, W, X, Y und Z die oben angegebene Bedeutung haben, Verbindungen der Formeln (I-a") his (I-g"), in welchen A, B, G, W, X und Z die oben genannte Bedeutung haben und Y' bevorzugt für Brom oder Iod steht 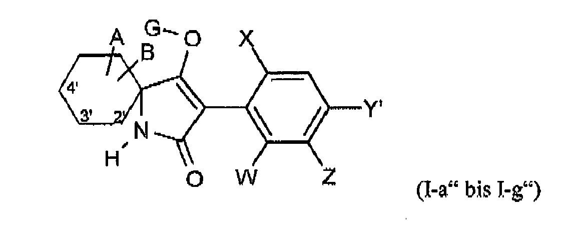 mit kupplungsfähigen (Het)-arylderivaten, z.B. Phenylboronsäuren der Formeln (XIIIα) und (XIIIβ) 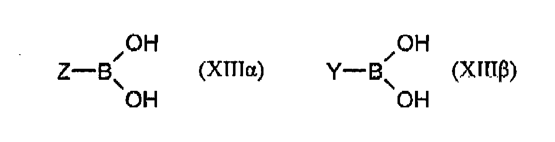 oder deren Ester in Gegenwart eines Lösungsmittels, in Gegenwart eines Katalysators und in Gegenwart einer Base kuppelt.

7. Mittel zur Bekämpfung von Schädlingen und/oder unerwünschten Pflanzenwuchs, **gekennzeichnet durch** einen Gehalt an mindestens einer Verbindung der Formel (I) gemäß Anspruch 1.

8. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge, unerwünschtem Pflanzenwuchs und/oder ihren Lebensraum einwirken lässt.

9. Verwendung von Verbindungen der Formel (I) gemäß Anspruch 1 zur Bekämpfung von tierischen Schädlingen und/oder unerwünschtem Pflanzenbewuchs.

10. Verfahren zur Herstellung von Mitteln zur Bekämpfung von Schädlingen und/oder unerwünschtem Pflanzenbewuchs, **dadurch gekennzeichnet, dass** man Verbindungen der Formel (I) gemäß Anspruch 1 mit Streckmitteln und/oder oberflächenaktiven Stoffen vermischt.

11. Verwendung von Verbindungen der Formel (I) gemäß Anspruch 1 zur Herstellung von Mitteln zur Bekämpfung von Schädlingen und/oder unerwünschtem Pflanzenwuchs.

12. Mittel enthaltend einen wirksamen Gehalt an einer Wirkstoffkombination umfassend als Komponenten
a') mindestens eine Verbindung der Formel (I) gemäß Anspruch 1, in welcher A, B, G, W, X, Y und Z die oben angegebene Bedeutung haben
und
(b') zumindest eine die Kulturpflanzen-Verträglichkeit verbessernde Verbindung aus der folgenden Gruppe von Verbindungen: S1, S2, S3, S4, S5, S6, S7, S8, S9, S10, S11, S12, S13, S14.

13. Mittel gemäß Anspruch 12, bei denen die Kulturpflanzen-Verträglichkeit verbessernde Vebindung Cloquintocct-mexyl ist.

14. Mittel gemäß Anspruch 12, bei denen die Kulturpflanzen-Verträglichkeit verbessernde Verbindung Mefenpyr-diethyl ist.

15. Verfahren zum Bekämpfen von unerwünschtem Pflanzenwuchs, **dadurch gekennzeichnet, dass** man ein Mittel gemäß Anspruch 12 auf die Pflanzen oder ihre Umgebung einwirken lässt.

16. Verwendung eines Mittels gemäß Anspruch 12 zum Bekämpfen von unerwünschten Pflanzenwuchs.

17. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 12 in zeitlich naher Abfolge getrennt auf die Pflanzen oder ihre Umgebung einwirken lässt.

18. Verbindungen der Formel (II) 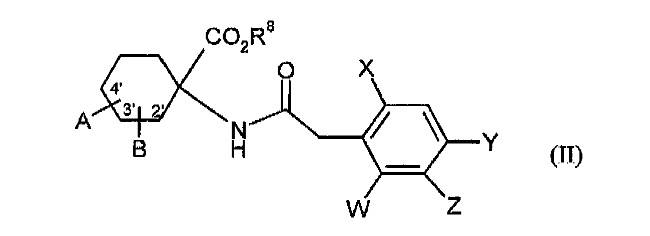 in welcher
A, B, W, X, Y, Z und R⁸ die oben angegebenen Bedeutungen haben.

19. Verbindungen der Formel (XIV) 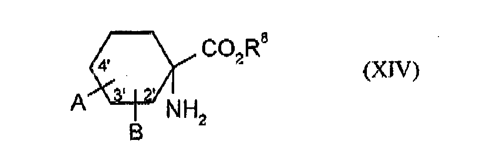 in welcher
A, B und R⁸ die oben angegebene Bedeutung haben.

20. Verbindungen der Formel (XVI) 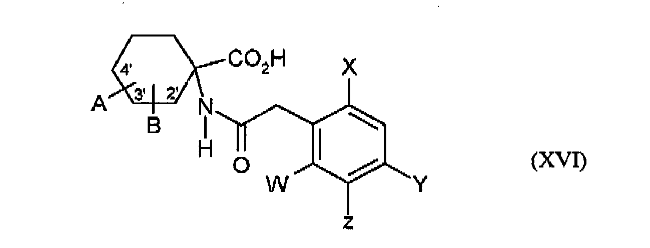 in welcher
A, B, W, X, Y und Z die oben angegebenen Bedeutungen haben.

21. Verbindungen der Formel (XVII) 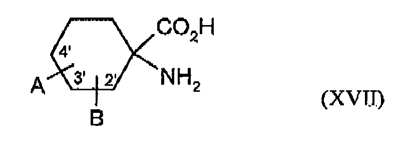 in welcher
A und B die oben angegebenen Bedeutungen haben.

22. Verbindungen der Formel (XVIII) 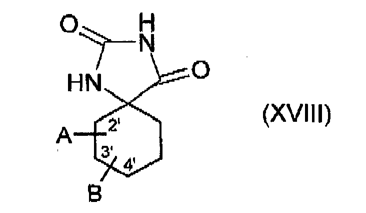 in welcher A und B die oben angegebenen Bedeutungen haben.

23. Verbindungen der Formel (XX) 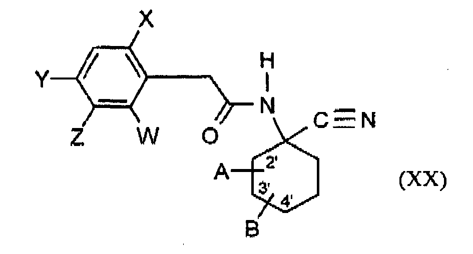 in welcher
A, B, W, X, Y und Z die oben angegebenen Bedeutungen haben.

24. Verbindungen der Formel (XIX) 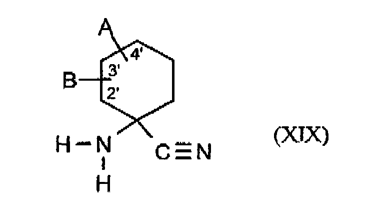 in welcher
A und B die oben angegebenen Bedeutungen haben.

25. Zusammensetzung umfassend
- mindestens eine Verbindung der Formel (I) gemäß Anspruch 1 oder ein Mittel gemäß Anspruch 12 und
- mindestens ein Salz der Formel (III') 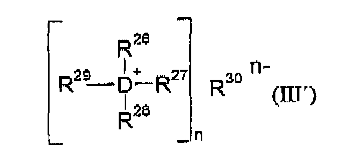 in welcher
D für Stickstoff oder Phosphor steht,
R²⁶, R²⁷, R²⁸ und R²⁹ unabhängig voneinander für Wasserstoff oder jeweils gegebenenfalls substituiertes C₁-C₈-Alkyl oder einfach oder mehrfach, ungesättigtes, gegebenenfalls substituiertes C₁-C₈-Alkylen stehen, wobei die Substituenten aus Halogen, Nitro und Cyano ausgewählt sein können,
n für 1, 2, 3 oder 4 steht,
R³⁰ für ein anorganisches oder organisches Anion steht.

26. Zusammensetzung gemäß Anspruch 25, **dadurch gekennzeichnet, dass** sie mindestens einen Penetrationsförderer enthält.

27. Verfahren zur Steigerung der Wirkung von Schädlingsbekämpfungsmitteln und/oder Herbiziden enthaltend einen Wirkstoff der Formel (I) gemäß Anspruch 1 oder ein Mittel gemäß Anspruch 12, **dadurch gekennzeichnet, dass** das anwendungsfertige Mittel (Spritzbrühe) unter Einsatz eines Salzes der Formel (III') gemäß Anspruch 25 zubereitet wird.

28. Verfahren gemäß Anspruch 27, **dadurch gekennzeichnet, dass** die Spritzbrühe unter Einsatz eines Penetrationsförderers zubereitet wird.

## Claims

1. Compounds of the formula (I) 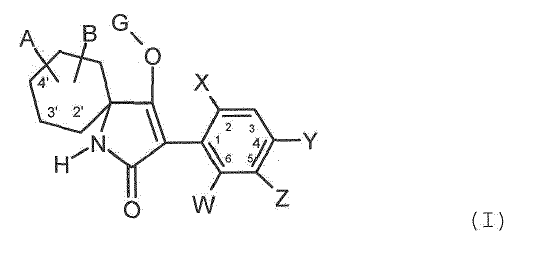 in which
W represents hydrogen, halogen, alkyl, alkenyl, alkynyl, optionally substituted cycloalkyl, alkoxy, alkenyloxy, haloalkyl, haloalkoxy or cyano,
X represents halogen, alkyl, alkenyl, alkynyl, optionally substituted cycloalkyl, alkoxy, alkenyloxy, alkylthio, alkylsulphinyl, alkylsulphonyl, haloalkyl, haloalkoxy, haloalkenyloxy, nitro or cyano,
Y and Z independently of one another represent hydrogen, alkyl, alkenyl, alkynyl, optionally substituted cycloalkyl, alkoxy, halogen, haloalkyl, haloalkoxy, cyano, nitro or in each case optionally substituted aryl or hetaryl,
A represents alkoxy,
B represents alkyl, where
A and B are attached to the same carbon atom,
G represents hydrogen (a) or one of the groups 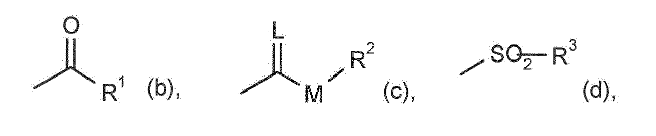 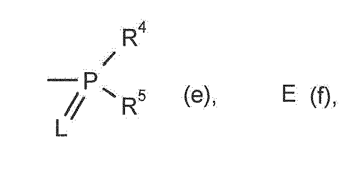
or 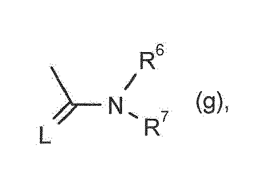 in which
E represents a metal ion or an ammonium ion,
L represents oxygen or sulphur,
M represents oxygen or sulphur,
R1 represents in each case optionally halogen- or cyano-substituted alkyl, alkenyl, alkoxyalkyl, alkylthioalkyl or polyalkoxyalkyl or represents in each case optionally halogen-, alkyl- or alkoxy-substituted cycloalkyl or heterocyclyl or represents in each case optionally substituted phenyl, phenylalkyl, hetaryl, phenoxyalkyl or hetaryloxyalkyl,
R2 represents in each case optionally halogen- or cyano-substituted alkyl, alkenyl, alkoxyalkyl or polyalkoxyalkyl or represents in each case optionally substituted cycloalkyl, phenyl or benzyl,
R3, R4 and R5 independently of one another represent in each case optionally halogen-substituted alkyl, alkoxy, alkylamino, dialkylamino, alkylthio, alkenylthio or cycloalkylthio or represent in each case optionally substituted phenyl, benzyl, phenoxy or phenylthio,
R6 and R7 independently of one another represent hydrogen, represent in each case optionally halogen- or cyano-substituted alkyl, cycloalkyl, alkenyl, alkoxy, alkoxyalkyl, represent in each case optionally substituted phenyl or benzyl, or together with the N atom to which they are attached form an optionally substituted cycle which optionally contains oxygen or sulphur.

2. Compounds of the formula (I) according to Claim 1, in which
W represents hydrogen, halogen, C₁-C₆-alkyl, C₂-C₆-alkenyl, C₂-C₆-alkynyl, represents C₃-C₆-cycloalkyl which is optionally mono- or disubstituted by C₁-C₂-alkyl, C₁-C₂-alkoxy, fluorine, chlorine, trifluoromethyl or C₃-C₆-cycloalkyl, represents C₁-C₆-alkoxy, C₁-C₄-haloalkyl, C₁-C₄-haloalkoxy or cyano,
X represents halogen, C1-C6-alkyl, C2-C6-alkenyl, C2-C6-alkynyl, represents C3-C6-cycloalkyl which is optionally mono- or disubstituted by C1-C2-alkyl, C1-C2-alkoxy, fluorine, chlorine, trifluoromethyl or C3-C6-cycloalkyl, represents C1-C6-haloalkyl, C1-C6-alkoxy, C3-C6-alkenyloxy, C1-C6-alkylthio, C1-C6-alkylsulphinyl, C1-C6-alkylsulphonyl, C1-C6-haloalkoxy, C3-C6-haloalkenyloxy, nitro or cyano,
Y and Z independently of one another represent hydrogen, halogen, C1-C6-alkyl, C2-C6-alkenyl, C2-C6-alkynyl, represent C₃-C₆-cycloalkyl which is optionally mono- or disubstituted by C₁-C₂-alkyl, C₁-C₂-alkoxy, fluorine, chlorine, trifluoromethyl or C₃-C₆-cycloalkyl, represent C1-C6-alkoxy, C1-C6-haloalkyl, C1-C6-haloalkoxy, cyano, C2-C6-alkenyl, C2-C6-alkynyl or represent one of the (het)aryl radicals 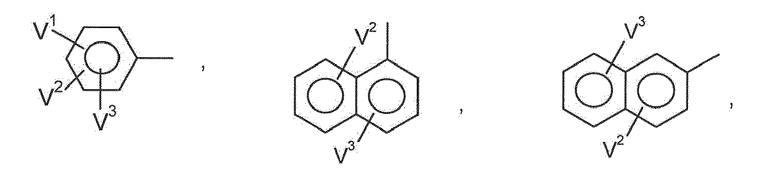 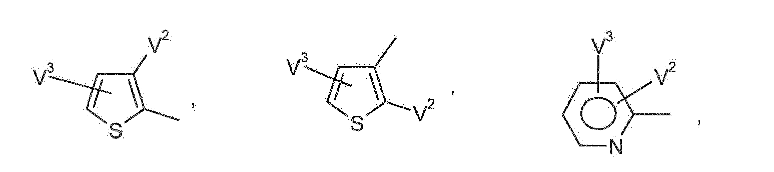 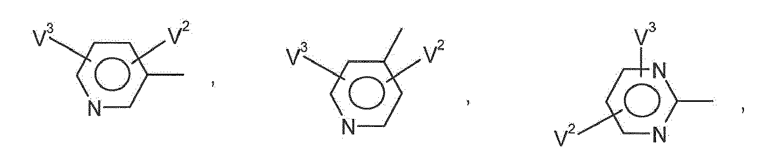 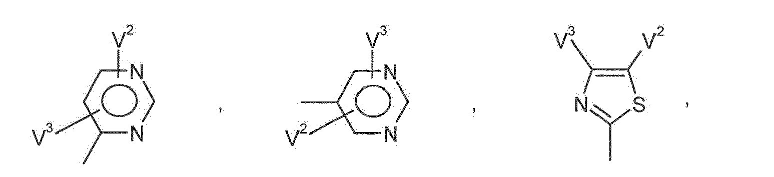 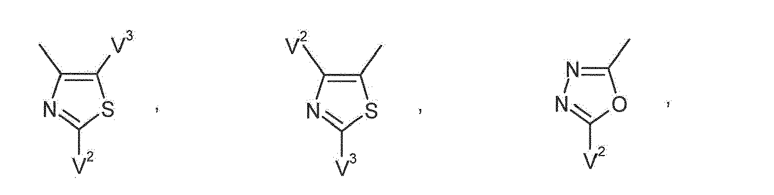 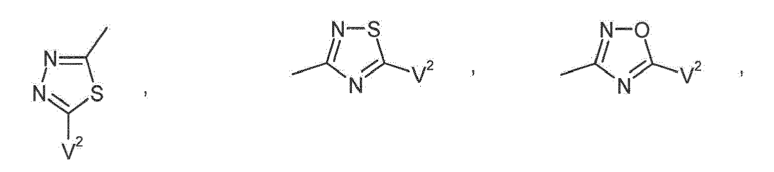 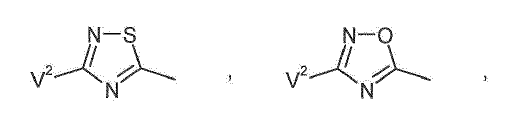
where in the case of (het)aryl only one of the radicals Y or Z may represent (het)aryl,
V1 represents hydrogen, halogen, C1-C12-alkyl, C1-C6-alkoxy, C1-C6-alkylthio, C1-C6-alkylsulphinyl, C1-C6-alkylsulphonyl, C1-C4-halogenoalkyl, C1-C4-halogenoalkoxy, nitro, cyano, or represents phenyl, phenoxy, phenoxy-C1-C4-alkyl, phenyl-C1-C4-alkoxy, phenylthio-C1-C4-alkyl or phenyl-C1-C4-alkylthio, each of which is optionally monosubstituted or polysubstituted by halogen, C1-C6-alkyl, C1-C6-alkoxy, C1-C4-halogenoalkyl, C1-C4-halogenoalkoxy, nitro or cyano,
V2 and V3 independently of one another represent hydrogen, halogen, C1-C6-alkyl, C1-C6-alkoxy, C1-C4-haloalkyl or C1-C4-haloalkoxy,
A represents C1-C₆-alkoxy,
B represents C₁-C₆-alkyl
where A and B are attached to the same carbon atom,
G represents hydrogen (a) or one of the groups 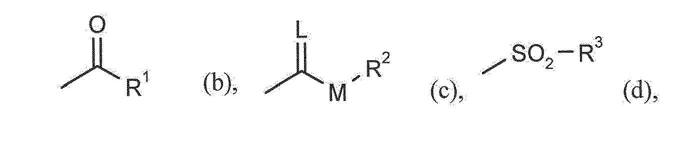 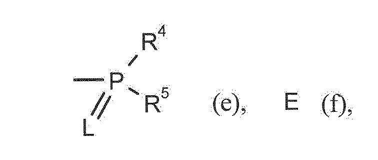
or 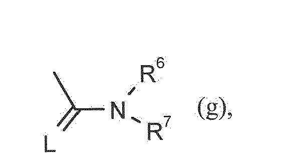 in which
E represents a metal ion or an ammonium ion,
L represents oxygen or sulphur and
M represents oxygen or sulphur,
R1 represents in each case optionally halogen- or cyano-substituted C1-C20-alkyl, C2-C20-alkenyl, C1-C8-alkoxy-C1-C8-alkyl, C1-C8-alkylthio-C1-C8-alkyl or poly-C1-C8-alkoxy-C1-C8-alkyl or represents optionally halogen-, C1-C6-alkyl- or C1-C6-alkoxy-substituted C3-C8-cycloalkyl in which optionally one or two not directly adjacent methylene groups are replaced by oxygen and/or sulphur,
represents phenyl which is optionally substituted by halogen, cyano, nitro, C1-C6-alkyl, C1-C6-alkoxy, C1-C6-haloalkyl, C1-C6-haloalkoxy, C1-C6-alkylthio or C1-C6-alkylsulphonyl,
or represents phenyl-C1-C6-alkyl which is optionally substituted by halogen, nitro, cyano, C1-C6-alkyl, C1-C6-alkoxy, C1-C6-halogenoalkyl or C1-C6-halogenoalkoxy,
represents optionally halogen- or C1-C6-alkyl-substituted 5- or 6-membered hetaryl having one or two heteroatoms from the group consisting of oxygen, sulphur and nitrogen,
represents phenoxy-C1-C6-alkyl which is optionally substituted by halogen or C1-C6-alkyl,
represents optionally halogen-, amino- or C1-C6-alkyl-substituted 5- or 6-membered hetaryloxy-C1-C6-alkyl having one or two heteroatoms from the group consisting of oxygen, sulphur and nitrogen,
R2 represents in each case optionally halogen- or cyano-substituted C1-C20-alkyl, C2-C20-alkenyl, C1-C8-alkoxy-C2-C8-alkyl or poly-C1-C8-alkoxy-C2-C8-alkyl,
or represents C3-C8-cycloalkyl which is optionally substituted by halogen, C1-C6-alkyl or C1-C6-alkoxy,
or represents phenyl or benzyl, each of which is optionally substituted by halogen, cyano, nitro, C1-C6-alkyl, C1-C6-alkoxy, C1-C6-halogenoalkyl or C1-C6-halogenalkoxy,
R3 represents optionally halogen-substituted C1-C8-alkyl or in each case optionally halogen-, C1-C6-alkyl-, C1-C6-alkoxy-, C1-C4-haloalkyl-, C1-C4-haloalkoxy-, cyano- or nitro-substituted phenyl or benzyl,
R4 and R5 independently of one another represent in each case optionally halogen-substituted C1-C8-alkyl, C1-C8-alkoxy, C1-C8-alkylamino, di(C1-C8-alkyl)amino, C1-C8-alkylthio or C3-C8-alkenylthio or represent in each case optionally halogen-, nitro-, cyano-, C1-C4-alkoxy-, C1-C4-haloalkoxy-, C1-C4-alkylthio-, C1-C4-haloalkylthio-, C1-C4-alkyl- or C1-C4-haloalkyl-substituted phenyl, phenoxy or phenylthio,
R6 and R7 independently of one another represent hydrogen, represent in each case optionally halogen- or cyano-substituted C1-C8-alkyl, C3-C8-cycloalkyl, C1-C8-alkoxy, C3-C8-alkenyl or C1-C8-alkoxy-C2-C8-alkyl, represent in each case optionally halogen-, C1-C8-alkyl-, C1-C8-haloalkyl- or C1-C8-alkoxy-substituted phenyl or benzyl or together represent an optionally C1-C6-alkyl-substituted C3-C6-alkylene radical in which optionally one methylene group is replaced by oxygen or sulphur.

3. Compounds of the formula (I) according to Claim 1, in which
W represents hydrogen, chlorine, bromine, C1-C4-alkyl, C2-C4-alkenyl, C2-C4-alkynyl, represents C3-C6-cycloalkyl which is optionally monosubstituted by methyl, ethyl, methoxy, fluorine, chlorine, trifluoromethyl or cyclopropyl, represents C1-C4-alkoxy, C₁-C₂-haloalkyl or C₁-C₂-haloalkoxy,
X represents chlorine, bromine, iodine, C₁-C₄-alkyl, C₂-C₄-alkenyl, C₂-C₄-alkynyl, represents C3-C6-cycloalkyl which is optionally monosubstituted by methyl, ethyl, methoxy, fluorine, chlorine, trifluoromethyl or cyclopropyl, represents C₁-C₄-alkoxy, C₁-C₄-haloalkyl, C₁-C₄-haloalkoxy or cyano,
Y and Z independently of one another represent hydrogen, fluorine, chlorine, bromine, iodine, C₁-C₄-alkyl, C2-C4-alkenyl, C2-C4-alkynyl, represent C₃-C₆-cycloalkyl which is optionally monosubstituted by methyl, ethyl, methoxy, fluorine, chlorine, trifluoromethyl or cyclopropyl, represents C₁-C₆-alkoxy, C₁-C₄-haloalkyl, C₁-C₄-haloalkoxy, cyano, C₂-C₄-alkenyl, C₂-C₄-alkynyl or represent one of the (het)aryl radicals, 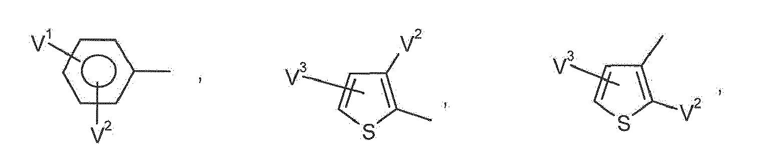 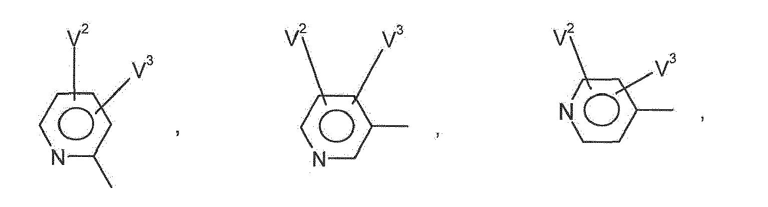 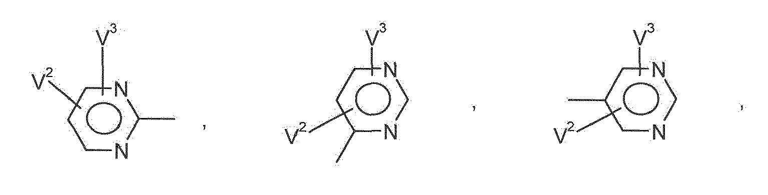
where in the case of (het)aryl only one of the radicals Y or Z may represent (het)aryl,
V1 represents hydrogen, fluorine, chlorine, bromine, C₁-C₆-alkyl, C₁-C₄-alkoxy, C₁-C₂- haloalkyl, C₁-C₂-haloalkoxy, nitro, cyano or represents phenyl which is optionally mono- or disubstituted by fluorine, chlorine, bromine, C₁-C₄-alkyl, C₁-C₄-alkoxy, C₁-C₂-haloalkyl, C₁-C₂-haloalkoxy, nitro or cyano,
V2 and V3 independently of one another represent hydrogen, fluorine, chlorine, bromine, C1-C4-alkyl, C1-C4-alkoxy, C1-C2-haloalkyl or C1-C2-haloalkoxy,
A represents C1-C₄-alkoxy,
B represents C1-C4-alkyl
where A and B are attached to the same carbon atom,
G represents hydrogen (a) or one of the groups 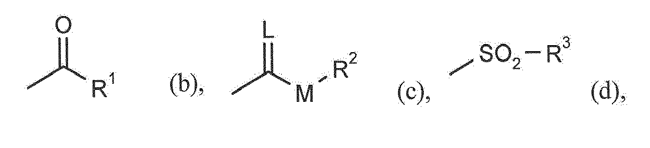 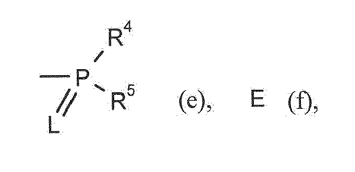
or 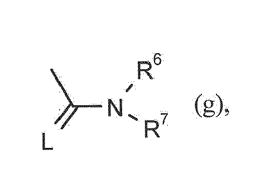 in which
E represents a metal ion or an ammonium ion,
L represents oxygen or sulphur and
M represents oxygen or sulphur,
R1 represents C1-C16-alkyl, C2-C16-alkenyl, C1-C6-alkoxy-C1-C4-alkyl, C1-C6-alkylthio-C1-C4-alkyl or poly-C1-C6-alkoxy-C1-C4-alkyl, each of which is optionally mono- to trisubstituted by fluorine or chlorine, or represents C3-C7-cycloalkyl which is optionally mono- or disubstituted by fluorine, chlorine, C1-C5-alkyl or C1-C5-alkoxy and in which optionally one or two not directly adjacent methylene groups are replaced by oxygen and/or sulphur,
represents phenyl which is optionally mono- to trisubstituted by fluorine, chlorine, bromine, cyano, nitro, C1-C4-alkyl, C1-C4-alkoxy, C1-C3-haloalkyl, C1-C3-haloalkoxy, C1-C4-alkylthio or C1-C4-alkylsulphonyl,
represents phenyl-C1-C4-alkyl which is optionally mono- or disubstituted by fluorine, chlorine, bromine, C1-C4-alkyl, C1-C4-alkoxy, C1-C3-haloalkyl or C1-C3-haloalkoxy,
represents pyrazolyl, thiazolyl, pyridyl, pyrimidyl, furanyl or thienyl, each of which is optionally mono- or disubstituted by fluorine, chlorine, bromine or C1-C4-alkyl,
represents phenoxy-C1-C5-alkyl which is optionally mono- or disubstituted by fluorine, chlorine, bromine or C1-C4-alkyl or
represents pyridyloxy-C1-C5-alkyl, pyrimidyloxy-C1-C5-alkyl or thiazolyloxy-C1-C5-alkyl, each of which is optionally mono- or disubstituted by fluorine, chlorine, bromine, amino or C1-C4-alkyl,
R2 represents C₁-C₁₆-alkyl, C₂-C₁₆-alkenyl, C₁-C₆-alkoxy-C₂-C₆-alkyl or poly-C₁-C₆-alkoxy-C₂-C₆-alkyl, each of which is optionally mono- to trisubstituted by fluorine or chlorine,
represents C3-C7-cycloalkyl which is optionally mono- or disubstituted by fluorine, chlorine, C1-C4-alkyl or C1-C4-alkoxy or
represents phenyl or benzyl, each of which is optionally mono- to trisubstituted by fluorine, chlorine, bromine, cyano, nitro, C1-C4-alkyl, C1-C3-alkoxy, C1-C3-haloalkyl or C1-C3-haloalkoxy,
R3 represents C1-C6-alkyl which is optionally mono-to trisubstituted by fluorine or chlorine or represents phenyl or benzyl, each of which is optionally mono- or disubstituted by fluorine, chlorine, bromine, C1-C4-alkyl, C1-C4-alkoxy, C1-C2-haloalkoxy, C1-C2-haloalkyl, cyano or nitro,
R⁴ and R⁵ independently of one another represent C₁-C₆-alkyl, C₁-C₆-alkoxy, C₁-C₆-alkylamino, di-(C₁-C₆-alkyl)amino, C₁-C₆-alkylthio or C₃-C₄-alkenylthio, each of which is optionally mono- to trisubstituted by fluorine or chlorine, or represent phenyl, phenoxy or phenylthio, each of which is optionally mono- or disubstituted by fluorine, chlorine, bromine, nitro, cyano, C₁-C₃-alkoxy, C₁-C₃-haloalkoxy, C₁-C₃-alkylthio, C₁-C₃-haloalkylthio, C₁-C₃-alkyl or C₁-C₃-haloalkyl,
R6 and R7 independently of one another represent hydrogen, represent C1-C6-alkyl, C3-C6-cycloalkyl, C1-C6-alkoxy, C3-C6-alkenyl or C1-C6-alkoxy-C2-C6-alkyl, each of which is optionally mono- to trisubstituted by fluorine or chlorine, represent phenyl or benzyl, each of which is optionally mono- to trisubstituted by fluorine, chlorine, bromine, C1-C5-haloalkyl, C1-C5-alkyl or C1-C5-alkoxy, or together represent an optionally C1-C4-alkyl-substituted C3-C6-alkylene radical in which optionally one methylene group is replaced by oxygen or sulphur.

4. Compounds of the formula (I) according to Claim 1, in which
W represents hydrogen, chlorine, bromine, methyl, ethyl, vinyl, ethynyl, propynyl, cyclopropyl, methoxy, ethoxy or trifluoromethyl,
X represents chlorine, bromine, methyl, ethyl, propyl, isopropyl, vinyl, ethynyl, propynyl, cyclopropyl, methoxy, ethoxy, trifluoromethyl, difluoromethoxy, trifluoromethoxy or cyano,
Y and Z independently of one another represent hydrogen, fluorine, chlorine, bromine, iodine, methyl, ethyl, vinyl, ethynyl, propynyl, cyclopropyl, methoxy, trifluoromethyl, trifluoromethoxy, cyano or a phenyl radical 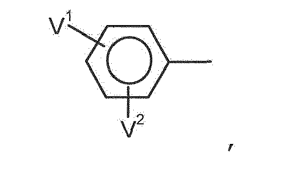
where in the case of phenyl only one of the radicals Y or Z may represent phenyl,
V1 represents hydrogen, fluorine or chlorine,
V2 represents hydrogen, fluorine, chlorine, methyl, ethyl, n-propyl, isopropyl, methoxy, ethoxy or trifluoromethyl,
A represents methoxy, ethoxy or propoxy,
B represents methyl, ethyl or propyl,
where A and B are attached to the same carbon atom,
G represents hydrogen (a) or one of the groups 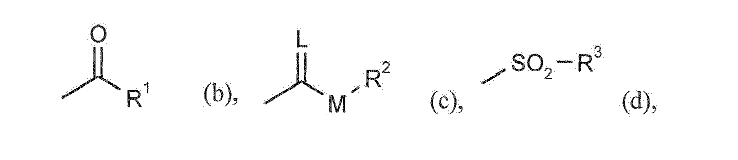 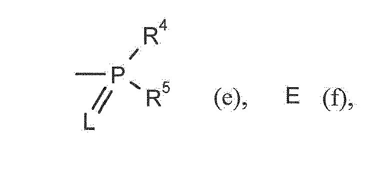
or 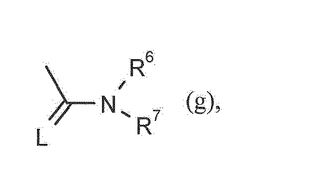 in which
E represents a metal ion or an ammonium ion,
L represents oxygen or sulphur and
M represents oxygen or sulphur,
R1 represents C1-C10-alkyl, C2-C10-alkenyl, C1-C4-alkoxy-C1-C2-alkyl, C1-C4-alkylthio-C1-C2-alkyl, each of which is optionally mono- to trisubstituted by fluorine or chlorine, or represents C3-C6-cycloalkyl which is optionally monosubstituted by fluorine, chlorine, methyl, ethyl or methoxy,
represents phenyl which is optionally mono- or disubstituted by fluorine, chlorine, bromine, cyano, nitro, methyl, ethyl, n-propyl, isopropyl, methoxy, ethoxy, trifluoromethyl or trifluoromethoxy,
represents furanyl, thienyl or pyridyl, each of which is optionally monosubstituted by chlorine, bromine or methyl,
R2 represents C1-C10-alkyl, C2-C10-alkenyl or C1-C4-alkoxy-C2-C4-alkyl, each of which is optionally mono- to trisubstituted by fluorine or chlorine,
represents cyclopentyl or cyclohexyl
or represents phenyl or benzyl, each of which is optionally mono- or disubstituted by fluorine, chlorine, cyano, nitro, methyl, ethyl, methoxy, trifluoromethyl or trifluoromethoxy,
R3 represents methyl, ethyl, propyl or isopropyl, each of which is optionally mono- to trisubstituted by fluorine or chlorine, or represents phenyl which is optionally monosubstituted by fluorine, chlorine, bromine, methyl, ethyl, isopropyl, tert-butyl, methoxy, ethoxy, isopropoxy, trifluoromethyl, trifluoromethoxy, cyano or nitro,
R4 and R5 independently of one another represent C1-C4-alkoxy or C1-C4-alkylthio or represent phenyl, phenoxy or phenylthio, each of which is optionally monosubstituted by fluorine, chlorine, bromine, nitro, cyano, methyl, methoxy, trifluoromethyl or trifluoromethoxy,
R⁶ and R⁷ independently of one another represent hydrogen, represent C₁-C₄-alkyl, C₃-C₆-cycloalkyl, C₁-C₄-alkoxy, C₃-C₄-alkenyl or C₁-C₄-alkoxy-C₂-C₄-alkyl, represent phenyl which is optionally mono-or disubstituted by fluorine, chlorine, bromine, methyl, methoxy or trifluoromethyl, or together represent a C₅-C₆-alkylene radical in which optionally one methylene group is replaced by oxygen or sulphur.

5. Compounds of the formula (I) according to Claim 1, in which
W represents hydrogen, chlorine, bromine, methyl, ethyl or methoxy,
X represents chlorine, bromine, methyl, ethyl, methoxy or ethoxy,
Y and Z independently of one another represent hydrogen, chlorine, bromine, methyl or represent the radical 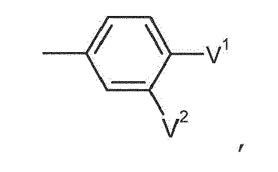
where in this case only one of the radicals Y or Z may represent 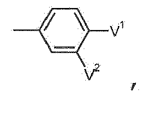
V¹ represents fluorine or chlorine,
V² represents hydrogen, fluorine or chlorine,
A represents methoxy or ethoxy,
B represents methyl, ethyl or propyl,
where A and B are attached to the same carbon atom in the 4'-position,
G represents hydrogen (a) or one of the groups 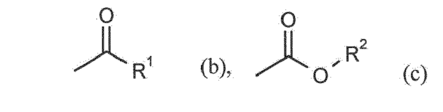
or E(f).
in which
E represents a metal ion or an ammonium ion,
R1 represents C1-C10-alkyl, C1-C4-alkoxy-C1-C2-alkyl, C3-C6-cycloalkyl,
represents phenyl which is optionally monosubstituted by chlorine, or represents thienyl,
R2 represents C₁-C₁₀-alkyl, C₂-C₁₀-alkenyl, or represents benzyl.

6. Process for the preparation of compounds of the formula (I) according to Claim 1, **characterized in that**, to obtain
(A) compounds of the formula (I-a) 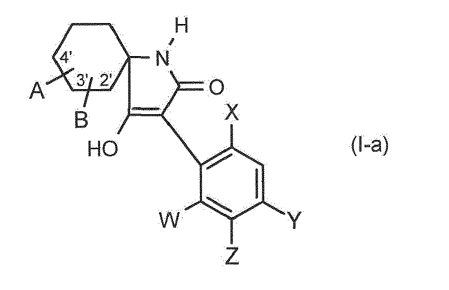 in which
A, B, W, X, Y and Z have the meanings given above, compounds of the formula (II) 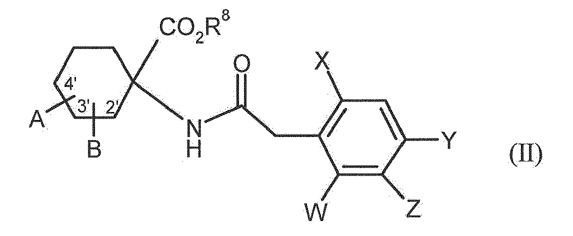 in which
A, B, W, X, Y and Z have the meanings given above,
and
R8 represents alkyl,
are subjected to an intramolecular condensation reaction in the presence of a diluent and in the presence of a base,
(B) compounds of the formula (I-b) 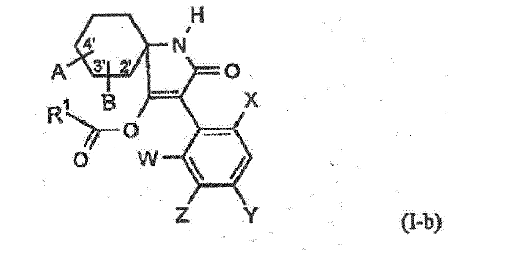 in which R¹, A, B, W, X, Y and Z have the meanings given above are obtained when compounds of the formula (I-a) shown above in which A, B, W, X, Y and Z have the meanings given above are in each case
α) reacted with compounds of the formula (III) 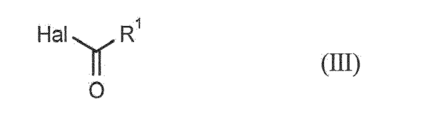
in which
R1 has the meaning given above and
Hal represents halogen
or
ß) with carboxylic anhydrides of the formula (IV)
R1-CO-O-CO-R1 (IV)
in which
R1 has the meaning given above,
if appropriate in the presence of a diluent and if appropriate in the presence of an acid binder;
(C) compounds of the formula (I-c) 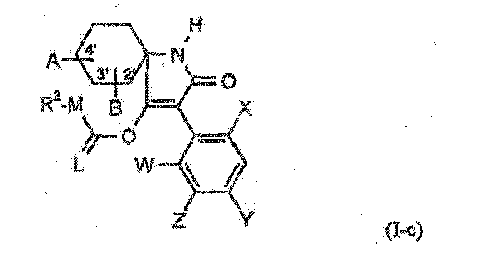 in which R2, A, B, M, W, X, Y and Z have the meanings given above and L represents oxygen are obtained when compounds of the formula (I-a) shown above in which A, B, W, X, Y and Z have the meanings given above are in each case
reacted with chloroformic esters or chloroformic thioesters of the formula (V)
R2-M-CO-Cl (V)
in which
R2 and M have the meanings given above,
if appropriate in the presence of a diluent and if appropriate in the presence of an acid binder;
(D) compounds of the formula (I-c) shown above in which R2, A, B, M, W, X, Y and Z have the meanings given above and L represents sulphur are obtained when compounds of the formula (I-a) shown above in which A, B, W, X, Y and Z have the meanings given above are in each case
reacted with chloromonothioformic esters or chlorodithioformic esters of the formula (VI) 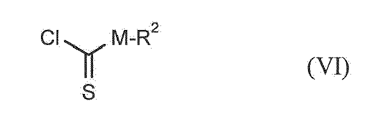 in which
M and R2 have the meanings given above,
if appropriate in the presence of a diluent and if appropriate in the presence of an acid binder,
(E) compounds of the formula (I-d) 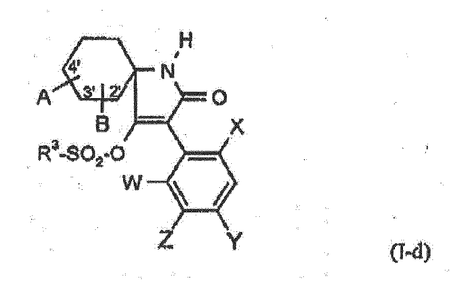 in which R³, A, B, W, X, Y and Z have the meanings given above are obtained when compounds of the formula (I-a) shown above in which A, B, W, X, Y and Z have the meanings given above are in each case
reacted with sulphonyl chlorides of the formula (VII)
R3-SO2-Cl (VII)
in which
R3 has the meaning given above,
if appropriate in the presence of a diluent and if appropriate in the presence of an acid binder,
(F) compounds of the formula (I-e) 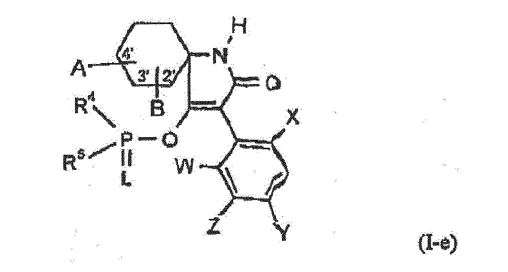 in which L, R4, R5, A, B, W, X, Y and Z have the meanings given above are obtained when compounds of the formula (I-a) shown above in which A, B, W, X, Y and Z have the meanings given above are in each case
reacted with phosphorus compounds of the formula (VIII) 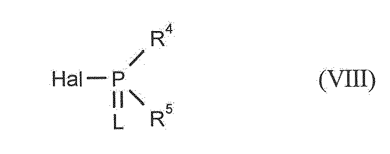 in which
L, R4 and R5 have the meanings given above and
Hal represents halogen
if appropriate in the presence of a diluent and if appropriate in the presence of an acid binder,
(G) compounds of the formula (I-f) 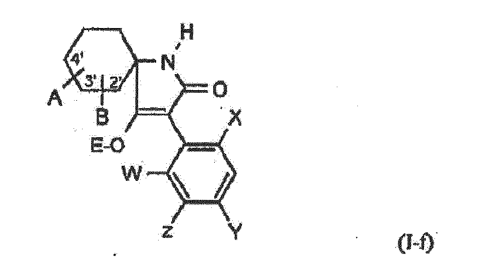 in which E, A, B, W, X, Y and Z have the meanings given above are obtained when compounds of the formula (I-a) in which A, B, W, X, Y and Z have the meanings given above are in each case
reacted with metal compounds or amines of the formula (IX) or (X)
Me(OR¹⁰)ₜ (IX) 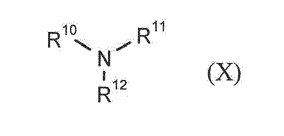
in which
Me represents a monovalent or divalent metal,
t represents the number 1 or 2 and
R10, R11, R12 independently of one another represent hydrogen or alkyl,
if appropriate in the presence of a diluent,
(H) compounds of the formula (I-g) 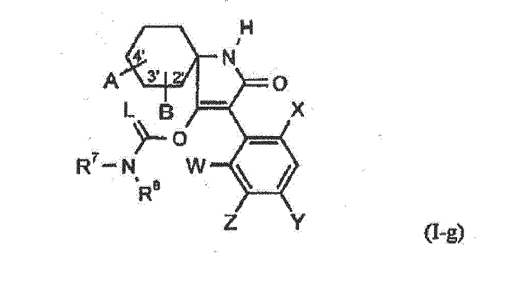 in which L, R6, R7, A, B, W, X, Y and Z have the meanings given above are obtained when compounds of the formula (I-a) shown above in which A, B, W, X, Y and Z have the meanings given above are in each case
α) reacted with isocyanates or isothiocyanates of the formula (XI)
R6-N=C=L (XI)
in which
R6 and L have the meanings given above,
if appropriate in the presence of a diluent and if appropriate in the presence of a catalyst, or
ß) reacted with carbamoyl chlorides or thiocarbamoyl chlorides of the formula (XII) 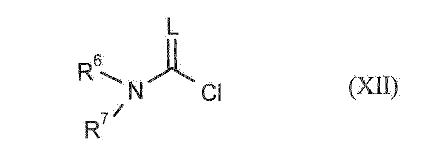 in which
L, R6 and R7 have the meanings given above,
if appropriate in the presence of a diluent and if appropriate in the presence of an acid binder,
(Iα) compounds of the formulae (I-a) to (I-g) shown above in which A, B, G, W, X, Y and Z have the meaning given above are obtained when compounds of the formulae (I-a') to (I-g') in which A, B, G, W, X and Y have the meaning given above and Z' preferably represents bromine or iodine 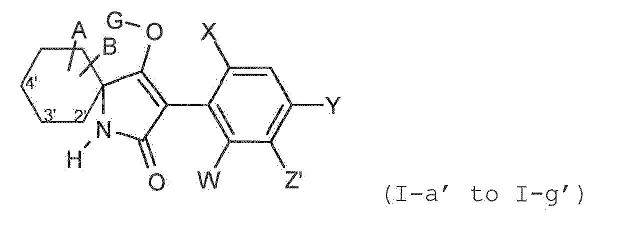 and
(Iβ) that compounds of the formulae (I-a) to (I-g) shown above in which A, B, G, W, X, Y and Z have the meaning given above are obtained when compounds of the formulae (I-a") to (I-g") in which A, B, G, W, X and Y have the meaning given above and Y' preferably represents bromine or iodine 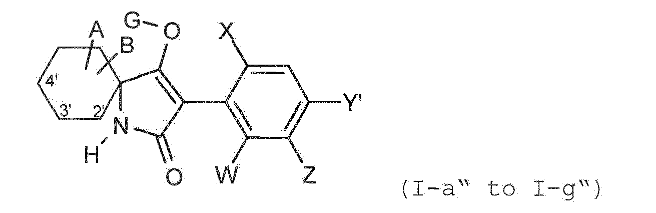 are coupled with (het)aryl derivatives capable of coupling, for example phenylboronic acids of the formulae (XIIIα) and (XIIIβ) 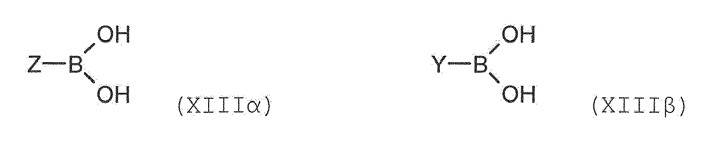 or esters thereof, in the presence of a solvent, in the presence of a catalyst and in the presence of a base.

7. Compositions for controlling pests and/or unwanted vegetation, **characterized in that** they comprise at least one compound of the formula (I) according to Claim 1.

8. Method of controlling animal pests and/or unwanted vegetation, **characterized in that** compounds of the formula (I) according to Claim 1 are allowed to act on pests, unwanted vegetation and/or their habitat.

9. Use of compounds of the formula (I) according to Claim 1 for controlling animal pests and/or unwanted vegetation.

10. Process for preparing compositions for controlling pests and/or unwanted vegetation, **characterized in that** compounds of the formula (I) according to Claim 1 are mixed with extenders and/or surfactants.

11. Use of compounds of the formula (I) according to Claim 1 for preparing compositions for controlling pests and/or unwanted vegetation.

12. Compositions, comprising an effective amount of an active compound combination comprising, as components,
a') at least one compound of the formula (I) according to Claim 1 in which A, B, G, W, X, Y and Z have the meaning given above
and
(b') at least one crop plant compatibility-improving compound from the following group of compounds: S1, S2, S3, S4, S5, S6, S7, S8, S9, S10, S11, S12, S13, S14.

13. Compositions according to Claim 12 where the crop plant compatibility-improving compound is cloquintocet-mexyl.

14. Compositions according to Claim 12 where the crop plant compatibility-improving compound is mefenpyr-diethyl.

15. Method for controlling unwanted vegetation, **characterized in that** a composition according to Claim 12 is allowed to act on the plants or their surroundings.

16. Use of a composition according to Claim 12 for controlling unwanted vegetation.

17. Method for controlling unwanted vegetation, **characterized in that** a compound of the formula (I) according to Claim 1 and the crop plant compatibility-improving compound as set forth in Claim 12 are allowed to act separately, in close temporal succession on the plants or their surroundings.

18. Compounds of the formula (II) 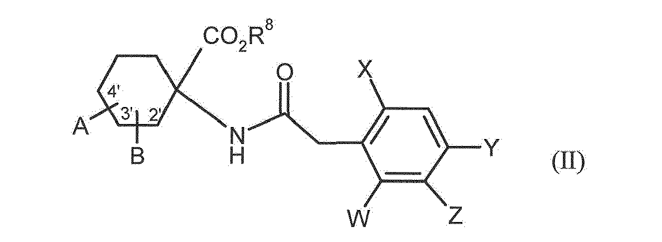 in which
A, B, W, X, Y, Z and R8 have the meanings given above.

19. Compounds of the formula (XIV) 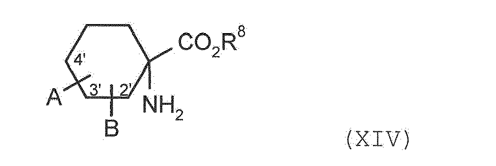 in which
A, B and R8 have the meaning given above.

20. Compounds of the formula (XVI) 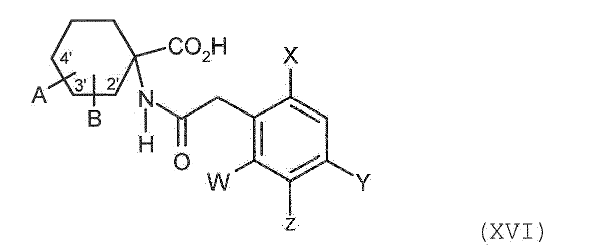 in which
A, B, W, X, Y and Z have the meanings given above.

21. Compounds of the formula (XVII) 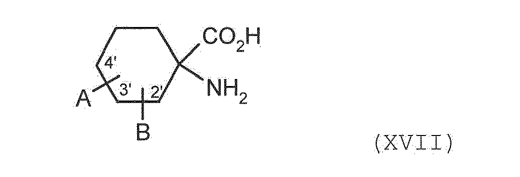 in which
A and B have the meanings given above.

22. Compounds of the formula (XVIII) 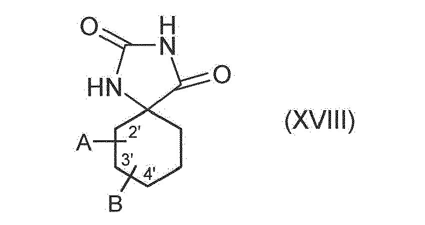 in which A and B have the meanings given above.

23. Compounds of the formula (XX) 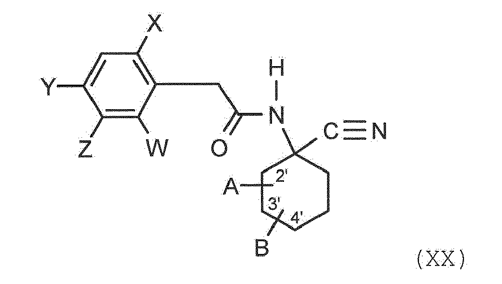 in which A, B, W, X, Y and Z have the meanings given above.

24. Compounds of the formula (XIX) 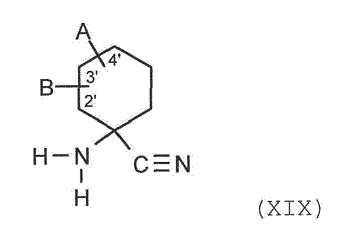 in which
A and B have the meanings given above.

25. Composition, comprising
- at least one compound of the formula (I) according to Claim 1 or a composition according to Claim 12 and
- at least one salt of the formula (III') 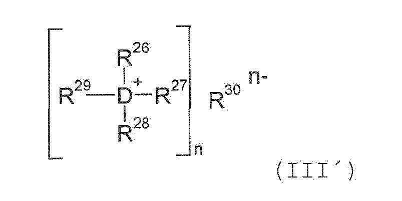 in which
D represents nitrogen or phosphorus,
R26, R27, R28 and R29 independently of one another represent hydrogen or in each case optionally substituted C₁-C₈-alkyl or mono- or polyunsaturated, optionally substituted C₁-C₈-alkylene, the substituents being selectable from halogen, nitro and cyano,
n represents 1, 2, 3 or 4,
R30 represents an inorganic or organic anion.

26. Composition according to Claim 25, **characterized in that** it comprises at least one penetrant.

27. Method of increasing the activity of pesticides and/or herbicides comprising an active compound of the formula (I) according to Claim 1 or a composition according to Claim 12, **characterized in that** the ready-to-use material (spray liquor) is prepared using a salt of the formula (III') according to Claim 25.

28. Method according to Claim 27, **characterized in that** the spray liquor is prepared using a penetrant.

## Revendications

1. Composés de formule (I) 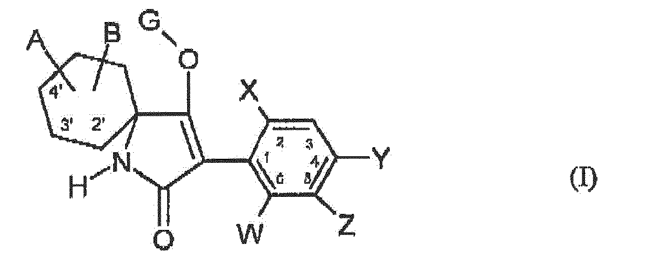 dans laquelle
W représente un atome d'hydrogène ou d'halogène, un groupe alkyle, alcényle, alcynyle, cycloalkyle éventuellement substitué, alcoxy, alcényloxy, halogénoalkyle, halogénoalcoxy ou cyano,
X représente un atome d'halogène, un groupe alkyle, alcényle, alcynyle, cycloalkyle éventuellement substitué, alcoxy, alcényloxy, alkylthio, alkylsulfinyle, alkylsulfonyle, halogénoalkyle, halogénoalcoxy, halogénoalcényloxy, nitro ou cyano,
Y et Z représentent indépendamment l'un de l'autre un atome d'hydrogène, un groupe alkyle, alcényle, alcynyle, cycloalkyle éventuellement substitué, alcoxy, un atome d'halogène, un groupe halogénoalkyle, halogénoalcoxy, cyano, nitro ou un groupe aryle ou hétéroaryle chacun éventuellement substitué,
A représente un groupe alcoxy,
B représente un groupe alkyle,
A et B étant liés au même atome de carbone,
G représente un atome d'hydrogène (a) ou l'un des groupes 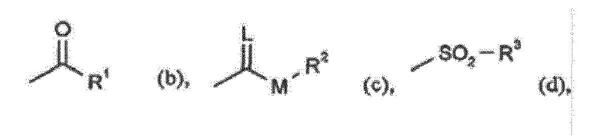 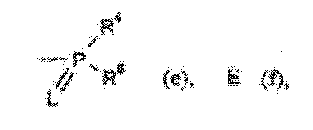
ou 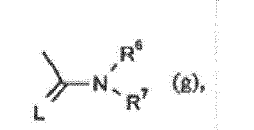 où
E représente un ion métallique ou un ion ammonium,
L représente un atome d'oxygène ou de soufre,
M représente un atome d'oxygène ou de soufre,
R¹ représente un groupe alkyle, alcényle, alcoxyalkyle, alkylthioalkyle ou polyalcoxyalkyle chacun éventuellement substitué par halogéno ou cyano, ou représente un groupe cycloalkyle ou hétérocyclyle chacun éventuellement substitué par halogéno, alkyle ou alcoxy ou représente un groupe phényle, phénylalkyle, hétéroaryle, phénoxyalkyle ou hétéroaryloxyalkyle chacun éventuellement substitué,
R² représente un groupe alkyle, alcényle, alcoxyalkyle ou polyalcoxyalkyle chacun éventuellement substitué par halogéno ou cyano ou représente un groupe cycloalkyle, phényle ou benzyle chacun éventuellement substitué,
R³, R⁴ et R⁵ chacun indépendamment représentent un groupe alkyle, alcoxy, alkylamino, dialkylamino, alkylthio, alcénylthio ou cycloalkylthio chacun éventuellement substitué par halogéno ou représentent un groupe phényle, benzyle, phénoxy ou phénylthio chacun éventuellement substitué,
R⁶ et R⁷ indépendamment l'un de l'autre représentent un atome d'hydrogène, représentent un groupe alkyle, cycloalkyle, alcényle, alcoxy, alcoxyalkyle, chacun éventuellement substitué par halogéno ou cyano, représentent un groupe phényle ou benzyle chacun éventuellement substitué, ou forment ensemble avec l'atome d'azote, auquel ils sont liés, un cycle contenant éventuellement des atomes d'oxygène ou de soufre et éventuellement substitué.

2. Composés de formule (I) selon la revendication 1, dans lesquels
W représente un atome d'hydrogène ou d'halogène, un groupe alkyle en C₁-C₆, alcényle en C₂-C₆, alcynyle en C₂-C₆, un groupe cycloalkyle en C₃-C₆ éventuellement une ou deux fois substitué par alkyle en C₁-C₂, alcoxy en C₁-C₂, fluoro, chloro, trifluorométhyle ou cycloalkyle en C₃-C₆, un groupe alcoxy en C₁-C₆, halogénoalkyle(C₁-C₄), halogénoalcoxy(C₁-C₄) ou cyano,
X représente un atome d'halogène, un groupe alkyle en C₁-C₆, alcényle en C₂-C₆, alcynyle en C₂-C₆, un groupe cycloalkyle en C₃-C₆ éventuellement une ou deux fois substitué par alkyle en C₁-C₂, alcoxy en C₁-C₂, fluoro, chloro, trifluorométhyle ou cycloalkyle en C₃-C₆, un groupe halogénoalkyle(C₁-C₆), alcoxy en C₁-C₆, alcényloxy(C₃-C₆) alkyl(C₁-C₆)thio, alkyl(C₁-C₆)sulfinyle, alkyl(C₁-C₆)sulfonyle, halogénoalcoxy(C₁-C₆), halogénoalcényloxy(C₃-C₆), nitro ou cyano,
Y et Z représentent indépendamment l'un de l'autre un atome d'hydrogène ou d'halogène, un groupe alkyle en C₁-C₆, alcényle en C₂-C₆, alcynyle en C₂-C₆, un groupe cycloalkyle en C₃-C₆ éventuellement une ou deux fois substitué par alkyle en C₁-C₂, alcoxy en C₁-C₂, fluoro, chloro, trifluorométhyle ou cycloalkyle en C₃-C₆, un groupe alcoxy en C₁-C₆, halogénoalkyle(C₁-C₆), halogénoalcoxy(C₁-C₆), cyano, alcényle en C₂-C₆, alcynyle en C₂-C₆ ou représentent l'un des radicaux (hétéro)aryle 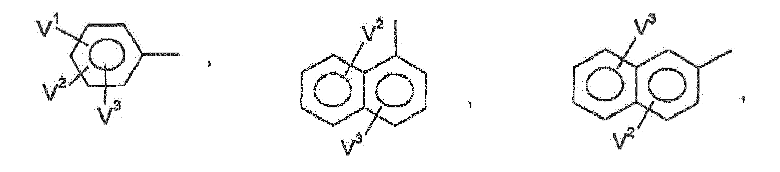 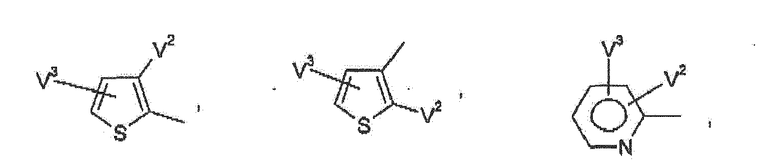 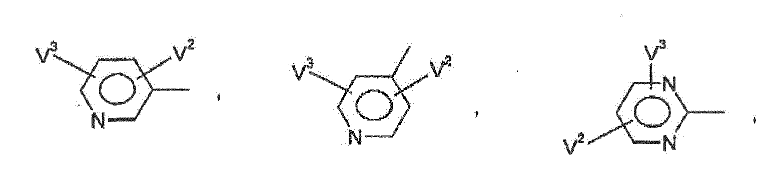 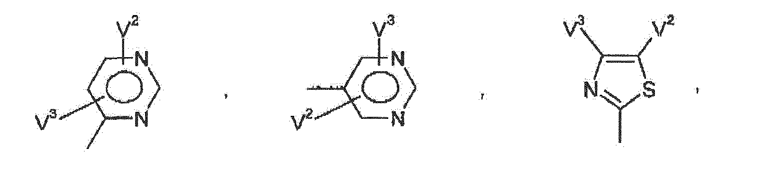 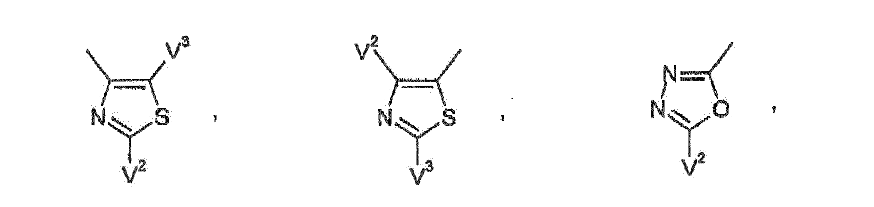 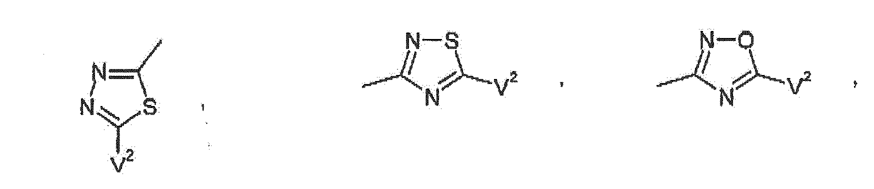 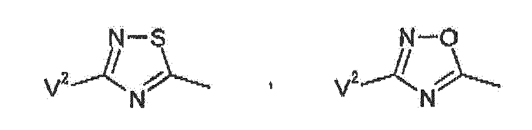
dans le cas du radical (hétéro)-aryle un seul des radicaux Y ou Z pouvant représenter un radical (hétéro)-aryle,
V¹ représente un atome d'hydrogène ou d'halogène, un groupe alkyle en C₁-C₁₂, alcoxy en C₁-C₆, alkyl(C₁-C₆)thio, alkyl (C₁-C₆) sulfinyl, alkyl(C₁-C₆)sulfonyle, halogénoalkyle(C₁-C₄), halogénoalcoxy(C₁-C₄), nitro, cyano ou un groupe phényle, phénoxy, phénoxy-alkyle(C₁-C₄), phényl-alcoxy(C₁-C₄), phénylthioalkyle(C₁-C₄) ou phényl-alkyl(C₁-C₄)thio chacun éventuellement une ou plusieurs fois substitué par halogéno, alkyle en C₁-C₆, alcoxy en C₁-C₆, halogénoalkyle(C₁-C₄), halogénoalcoxy(C₁-C₄), nitro ou cyano,
V² et V³ représentent indépendamment l'un de l'autre un atome d'hydrogène ou d'halogène, un groupe alkyle en C₁-C₆, alcoxy en C₁-C₆, halogénoalkyle(C₁-C₄) ou halogénoalcoxy(C₁-C₄),
A représente un groupe alcoxy en C₁-C₆,
B représente un groupe alkyle en C₁-C₆
A et B étant liés au même atome de carbone,
G représente un atome d'hydrogène (a) ou l'un des groupes 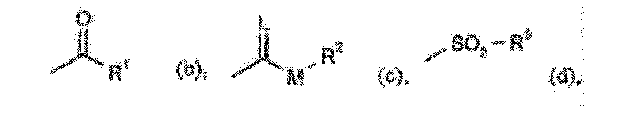 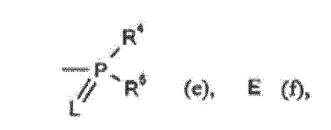
ou 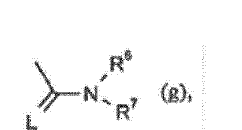 dans lesquels
E représente un ion métallique ou un ion ammonium,
L représente un atome d'oxygène ou de soufre,
M représente un atome d'oxygène ou de soufre,
R¹ représente un groupe alkyle en C₁-C₂₀, alcényle en C₂-C₂₀, alcoxy(C₁-C₈)-alkyle(C₁-C₈), alkyl(C₁-C₈)-thio-alkyle(C₁-C₈) ou polyalcoxy(C₁-C₈)-alkyle(C₁-C₈) chacun éventuellement substitué par halogéno ou cyano, ou représente un groupe cycloalkyle en C₃-C₈ éventuellement substitué par halogéno, alkyle en C₁-C₆ ou alcoxy en C₁-C₆, dans lequel éventuellement un ou deux groupes méthylène non directement voisins sont remplacés par des atomes d'oxygène ou de soufre,
représente un groupe phényle éventuellement substitué par halogéno, cyano, nitro, alkyle en C₁-C₆, alcoxy en C₁-C₆, halogénoalkyle(C₁-C₆), halogénoalcoxy(C₁-C₆), alkyl(C₁-C₆)thio ou alkyl (C₁-C₆) sulfonyle,
représente un groupe phényl-alkyle(C₁-C₆) éventuellement substitué par halogéno, nitro, cyano, alkyle en C₁-C₆, alcoxy en C₁-C₆, halogéno-alkyle(C₁-C₆) ou halogénoalcoxy(C₁-C₆),
représente un groupe hétéroaryle à 5 ou 6 chaînons, comportant un ou deux hétéroatomes choisis dans la série des atomes d'oxygène, de soufre ou d'azote, éventuellement substitué par halogéno ou alkyle en C₁-C₆,
représente un groupe phénoxy-alkyle(C₁-C₆) éventuellement substitué par halogéno ou alkyle en C₁-C₆ ou
représente un groupe hétéroaryloxy(à 5 ou 6 chaînons)-alkyle(C₁-C₆) comportant un ou deux hétéroatomes choisis dans la série des atomes d'oxygène, de soufre ou d'azote, éventuellement substitué par halogéno, amino ou alkyle en C₁-C₆,
R² représente un groupe alkyle en C₁-C₂₀, alcényle en C₂-C₂₀, alcoxy(C₁-C₈)-alkyle (C₂-C₈) ou poly-alcoxy(C₁-C₈)-alkyle(C₂-C₈), chacun éventuellement substitué par halogéno ou cyano,
représente un groupe cycloalkyle en C₃-C₈ éventuellement substitué par halogéno, alkyle en C₁-C₆ ou alcoxy en C₁-C₆ ou
représente un groupe phényle ou benzyle chacun éventuellement substitué par halogéno, cyano, nitro, alkyle en C₁-C₆, alcoxy en C₁-C₆, halogénoalkyle(C₁-C₆) ou halogénoalcoxy(C₁-C₆),
R³ représente un groupe alkyle en C₁-C₈ éventuellement substitué par halogéno, ou un groupe phényle ou benzyle chacun éventuellement substitué par halogéno, alkyle en C₁-C₆, alcoxy en C₁-C₆, halogénoalkyle(C₁-C₄), halogénoalcoxy(C₁-C₄), cyano ou nitro,
R⁴ et R⁵ indépendamment l'un de l'autre représentent un groupe alkyle en C₁-C₈, alcoxy en C₁-C₈, alkyl (C₁-C₈) amino, di-(alkyl(C₁-C₈) ) amino, alkyl(C₁-C₈)thio ou alcényl(C₃-C₈)thio, chacun éventuellement substitué par halogéno ou représentent un groupe phényle, phénoxy ou phénylthio chacun éventuellement substitué par halogéno, nitro, cyano, alcoxy en C₁-C₄, halogéno-alcoxy(C₁-C₄), alkyl(C₁-C₄)thio, halogéno-alkyl(C₁-C₄)thio, alkyle en C₁-C₄ ou halogénoalkyle(C₁-C₄),
R⁶ et R⁷ indépendamment l'un de l'autre représentent un atome d'hydrogène, représentent un groupe alkyle en C₁-C₈, cycloalkyle en C₃-C₈, alcoxy en C₁-C₈, alcényle en C₃-C₈ ou alcoxy(C₁-C₈)-alkyle(C₂-C₈), chacun éventuellement substitué par halogéno ou cyano, représentent un groupe phényle ou benzyle chacun éventuellement substitué par halogéno, alkyle en C₁-C₈, halogénoalkyle(C₁-C₈) ou alcoxy en C₁-C₈, ou représentent ensemble un radical alkylène en C₃-C₆ éventuellement substitué par alkyle en C₁-C₆, dans lequel éventuellement un groupe méthylène est remplacé par un atome d'oxygène ou de soufre.

3. Composés de formule (I) selon la revendication 1, dans lesquels
W représente un atome d'hydrogène, de chlore, de brome, un groupe alkyle en C₁-C₄, alcényle en C₂-C₄, alcynyle en C₂-C₄, un groupe cycloalkyle en C₃-C₆ éventuellement une fois substitué par méthyle, éthyle, méthoxy, fluoro, chloro, trifluorométhyle ou cyclopropyle, un groupe alcoxy en C₁-C₄, halogénoalkyle(C₁-C₂) ou halogéno-alcoxy(C₁-C₂),
X représente un atome de chlore, brome, iode, un groupe alkyle en C₁-C₄, alcényle en C₂-C₄, alcynyle en C₂-C₄, un groupe cycloalkyle en C₃-C₆ éventuellement une fois substitué par méthyle, éthyle, méthoxy, fluoro, chloro, trifluorométhyle ou cyclopropyle, un groupe alcoxy en C₁-C₄, halogénoalkyle(C₁-C₄), halogénoalcoxy(C₁-C₄) ou cyano,
Y et Z représentent indépendamment l'un de l'autre un atome d'hydrogène, de fluor, chlore, brome, iode, un groupe alkyle en C₁-C₄, alcényle en C₂-C₄, alcynyle en C₂-C₄, un groupe cycloalkyle en C₃-C₆ éventuellement une fois substitué par méthyle, éthyle, méthoxy, fluoro, chloro, trifluorométhyle ou cyclopropyle, un groupe alcoxy en C₁-C₆, halogénoalkyle(C₁-C₄), halogénoalcoxy(C₁-C₄), cyano, alcényle en C₂-C₄, alcynyle en C₂-C₄ ou représentent l'un des radicaux (hétéro)aryle 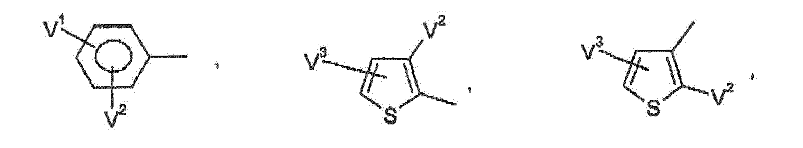 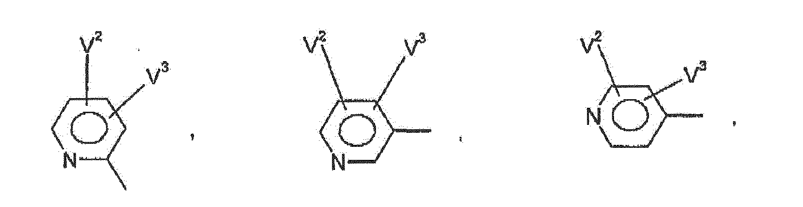 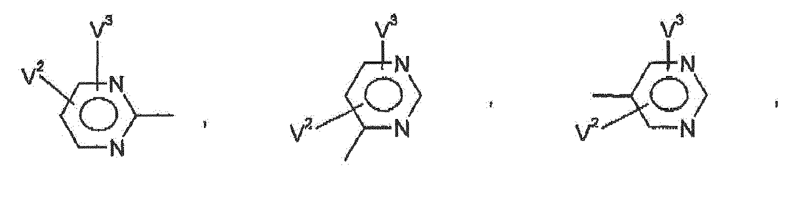
dans le cas du radical (hétéro)-aryle un seul des radicaux Y ou Z pouvant représenter un radical (hétéro)-aryle,
V¹ représente un atome d'hydrogène, de fluor, chlore, brome, un groupe alkyle en C₁-C₆, alcoxy en C₁-C₄, halogénoalkyle(C₁-C₂), halogénoalcoxy(C₁-C₂), nitro, cyano ou un groupe phényle éventuellement une ou deux fois substitué par fluoro, chloro, bromo, alkyle en C₁-C₄, alcoxy en C₁-C₄, halogénoalkyle(C₁-C₂), halogénoalcoxy(C₁-C₂), nitro ou cyano,
V² et V³ représentent indépendamment l'un de l'autre un atome d'hydrogène, de fluor, chlore, brome, un groupe alkyle en C₁-C₄, alcoxy en C₁-C₄, halogénoalkyle(C₁-C₂) ou halogénoalcoxy(C₁-C₂),
A représente un groupe alcoxy en C₁-C₄,
B représente un groupe alkyle en C₁-C₄,
A et B étant liés au même atome de carbone,
G représente un atome d'hydrogène (a) ou l'un des groupes 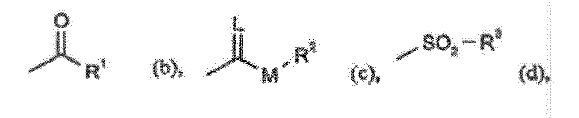 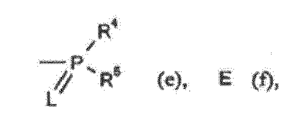
ou 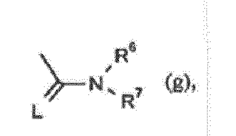 dans lesquels
E représente un ion métallique ou un ion ammonium,
L représente un atome d'oxygène ou de soufre,
M représente un atome d'oxygène ou de soufre,
R¹ représente un groupe alkyle en C₁-C₁₆, alcényle en C₂-C₁₆, alcoxy (C₁-C₆) -alkyle (C₁-C₄), alkyl (C₁-C₆)-thio-alkyle (C₁-C₄) ou polyalcoxy (C₁-C₆)-alkyle(C₁-C₄) chacun éventuellement une à trois fois substitué par fluoro ou chloro, ou représente un groupe cycloalkyle en C₃-C₇ éventuellement une ou deux fois substitué par fluoro, chloro, alkyle en C₁-C₅ ou alcoxy en C₁-C₅, dans lequel éventuellement un ou deux groupes méthylène non directement voisins sont remplacés par des atomes d'oxygène et/ou de soufre,
représente un groupe phényle éventuellement une à trois fois substitué par fluoro, chloro, bromo, cyano, nitro, alkyle en C₁-C₄, alcoxy en C₁-C₄, halogénoalkyle(C₁-C₃), halogénoalcoxy(C₁-C₃), alkyl(C₁-C₄)thio ou alkyl(C₁-C₄)sulfonyle,
représente un groupe phényl-alkyle(C₁-C₄) éventuellement une ou deux fois substitué par fluoro, chloro, bromo, alkyle(C₁-C₄), alcoxy(C₁-C₄), halogénoalkyle(C₁-C₃) ou halogénoalcoxy(C₁-C₃),
représente un groupe pyrazolyle, thiazolyle, pyridyle, pyrimidyle, furanyle ou thiényle, chacun éventuellement une ou deux fois substitué par fluoro, chloro, bromo ou alkyle en C₁-C₄,
représente un groupe phénoxyalkyle(C₁-C₅) éventuellement une ou deux fois substitué par fluoro, chloro, bromo ou alkyle en C₁-C₄ ou
représente un groupe pyridyloxy-alkyle(C₁-C₅), pyrimidyloxy-alkyle(C₁-C₅) ou thiazolyloxy-alkyle(C₁-C₅), chacun éventuellement une ou deux fois substitué par fluoro, chloro, bromo, amino ou alkyle en C₁-C₄,
R² représente un groupe alkyle en C₁-C₁₆, alcényle en C₂-C₁₆, alcoxy(C₁-C₆)-alkyle(C₂-C₆) ou poly-alcoxy(C₁-C₆)-alkyle(C₂-C₆), chacun éventuellement une à trois fois substitué par fluoro ou chloro,
représente un groupe cycloalkyle en C₃-C₇ éventuellement une ou deux fois substitué par fluoro, chloro, alkyle en C₁-C₄ ou alcoxy en C₁-C₄ ou
représente un groupe phényle ou benzyle chacun éventuellement une à trois fois substitué par fluoro, chloro, bromo, cyano, nitro, alkyle en C₁-C₄, alcoxy en C₁-C₃, halogénoalkyle(C₁-C₃) ou halogénoalcoxy(C₁-C₃),
R³ représente un groupe alkyle en C₁-C₆ éventuellement une à trois fois substitué par fluor ou chloro ou un groupe phényle ou benzyle chacun éventuellement une ou deux fois substitué par fluoro, chloro, bromo, alkyle en C₁-C₄, alcoxy en C₁-C₄, halogénoalkyle(C₁-C₂), halogénoalcoxy(C₁-C₂), cyano ou nitro,
R⁴ et R⁵ indépendamment l'un de l'autre représentent chacun un groupe alkyle en C₁-C₆, alcoxy en C₁-C₆, alkyl (C₁-C₆) amino, di-(alkyl(C₁-C₆))amino, alkyl(C₁-C₆)thio ou alcényl(C₃-C₄)thio, chacun éventuellement une à trois fois substitué par fluoro ou chloro, ou représentent chacun un groupe phényle, phénoxy ou phénylthio chacun éventuellement une ou deux fois substitué par fluoro, chloro, bromo, nitro, cyano, alcoxy en C₁-C₃, halogénoalcoxy(C₁-C₃), alkyl (C₁-C₃) thio, halogénoalkyl(C₁-C₃)thio, alkyle en C₁-C₃ ou halogénoalkyle(C₁-C₃),
R⁶ et R⁷ indépendamment l'un de l'autre représentent un atome d'hydrogène, représentent un groupe alkyle en C₁-C₆, cycloalkyle en C₃-C₆, alcoxy en C₁-C₆, alcényle en C₃-C₆ ou alcoxy(C₁-C₆)-alkyle(C₂-C₆), chacun éventuellement une à trois fois substitué par fluoro ou chloro, représentent un groupe phényle ou benzyle chacun éventuellement une à trois fois substitué par fluoro, chloro, bromo, halogénoalkyle(C₁-C₅), alkyle en C₁-C₅, ou alcoxy en C₁-C₅, ou représentent ensemble un radical alkylène en C₃-C₆ éventuellement substitué par alkyle en C₁-C₄, dans lequel éventuellement un groupe méthylène est remplacé par un atome d'oxygène ou de soufre.

4. Composés de formule (I) selon la revendication 1, dans lesquels
W représente un atome d'hydrogène, de chlore, de brome, le groupe méthyle, éthyle, vinyle, éthynyle, propynyle, cyclopropyle, méthoxy, éthoxy ou trifluorométhyle,
X représente un atome de chlore, brome, le groupe méthyle, éthyle, propyle, isopropyle, vinyle, éthynyle, propynyle, cyclopropyle, méthoxy, éthoxy, trifluorométhyle, difluorométhoxy, trifluorométhoxy ou cyano,
Y et Z représentent indépendamment l'un de l'autre un atome d'hydrogène, de fluor, chlore, brome, iode, le groupe méthyle, éthyle, vinyle, éthynyle, propynyle, cyclopropyle, méthoxy, trifluorométhyle, trifluorométhoxy, cyano ou un radical phényle 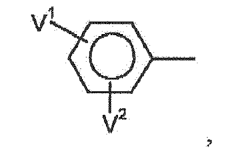
dans le cas du radical phényle un seul des radicaux Y ou Z pouvant représenter un radical phényle,
V¹ représente un atome d'hydrogène, de fluor ou de chlore,
V² représente un atome d'hydrogène, de fluor, chlore, le groupe méthyle, éthyle, n-propyle, isopropyle, méthoxy, éthoxy ou trifluorométhyle,
A représente le groupe méthoxy, éthoxy ou propoxy,
B représente le groupe méthyle, éthyle ou propyle,
A et B étant liés au même atome de carbone,
G représente un atome d'hydrogène (a) ou l'un des groupes 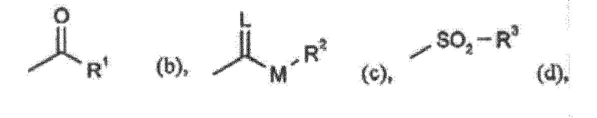 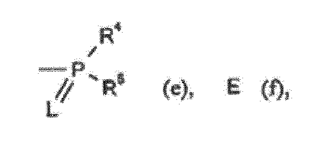
ou 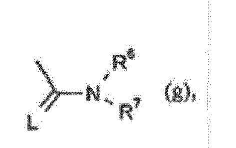 dans lesquels
E représente un ion métallique ou un ion ammonium,
L représente un atome d'oxygène ou de soufre,
M représente un atome d'oxygène ou de soufre,
R¹ représente un groupe alkyle en C₁-C₁₀, alcényle en C₂-C₁₀, alcoxy (C₁-C₄) -alkyle (C₁-C₂), alkyl (C₁-C₄)-thio-alkyle(C₁-C₂), chacun éventuellement une à trois fois substitué par fluoro ou chloro, ou représente un groupe cycloalkyle en C₃-C₆ éventuellement une fois substitué par fluoro, chloro, méthyle, éthyle ou méthoxy,
représente un groupe phényle éventuellement une ou deux fois substitué par fluoro, chloro, bromo, cyano, nitro, méthyle, éthyle, n-propyle, isopropyle, méthoxy, éthoxy, trifluorométhyle ou trifluorométhoxy,
représente un groupe furanyle, thiényle ou pyridyle chacun éventuellement une fois substitué par chloro, bromo ou méthyle,
R² représente un groupe alkyle en C₁-C₁₀, alcényle en C₂-C₁₀ ou alcoxy (C₁-C₄)-alkyle (C₂-C₄), chacun éventuellement une à trois fois substitué par fluoro ou chloro,
représente le groupe cyclopentyle ou cyclohexyle
ou représente un groupe phényle ou benzyle chacun éventuellement une ou deux fois substitué par fluoro, chloro, cyano, nitro, méthyle, éthyle, méthoxy, trifluorométhyle ou trifluorométhoxy,
R³ représente un groupe méthyle, éthyle, propyle ou isopropyle, chacun éventuellement une à trois fois substitué par fluor ou chloro, ou un groupe phényle éventuellement une fois substitué par fluoro, chloro, bromo, méthyle, éthyle, isopropyle, tert-butyle, méthoxy, éthoxy, isopropoxy, trifluorométhyle, trifluorométhoxy, cyano ou nitro,
R⁴ et R⁵ indépendamment l'un de l'autre représentent un groupe alcoxy en C₁-C₄ ou alkyl (C₁-C₄) thio ou représentent un groupe phényle, phénoxy ou phénylthio chacun éventuellement une fois substitué par fluoro, chloro, bromo, nitro, cyano, méthyle, méthoxy, trifluorométhyle ou trifluorométhoxy,
R⁶ et R⁷ indépendamment l'un de l'autre représentent un atome d'hydrogène, représentent un groupe alkyle en C₁-C₄, cycloalkyle en C₃-C₆, alcoxy en C₁-C₄, alcényle en C₃-C₄ ou alcoxy(C₁-C₄)-alkyle(C₂-C₄), représentent un groupe phényle éventuellement une ou deux fois substitué par fluoro, chloro, bromo, méthyle, méthoxy ou trifluorométhyle, ou représentent ensemble un radical alkylène en C₅-C₆, dans lequel éventuellement un groupe méthylène est remplacé par un atome d'oxygène ou de soufre.

5. Composés de formule (I) selon la revendication 1, dans lesquels
W représente un atome d'hydrogène, de chlore, de brome, le groupe méthyle, éthyle ou méthoxy,
X représente un atome de chlore, brome, le groupe méthyle, éthyle, méthoxy ou éthoxy,
Y et Z représentent indépendamment l'un de l'autre un atome d'hydrogène, de chlore, brome, le groupe méthyle ou le radical 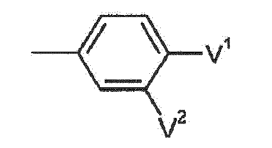
en ce cas un seul des radicaux Y ou Z pouvant représenter 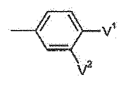
V¹ représente un atome de fluor ou de chlore,
V² représente un atome d'hydrogène, de fluor ou de chlore,
A représente le groupe méthoxy ou éthoxy,
B représente le groupe méthyle, éthyle ou propyle,
A et B étant liés au même atome de carbone à la position 4',
G représente un atome d'hydrogène (a) ou l'un des groupes 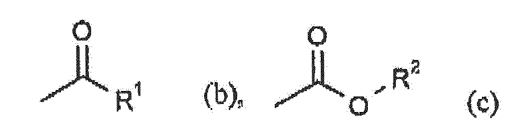
ou E(f)
dans lesquels
E représente un ion métallique ou un ion ammonium,
R¹ représente un groupe alkyle en C₁-C₁₀, alcoxy (C₁-C₄) -alkyle (C₁-C₂), cycloalkyle en C₃-C₆,
représente un groupe phényle éventuellement une fois substitué par chloro, ou représente le groupe thiényle,
R² représente un groupe alkyle en C₁-C₁₀, alcényle en C₂-C₁₀ ou représente le groupe benzyle.

6. Procédé pour la préparation de composés de formule (I) selon la revendication 1, **caractérisé en ce que**, pour l'obtention
(A) de composés de formule (I-a) 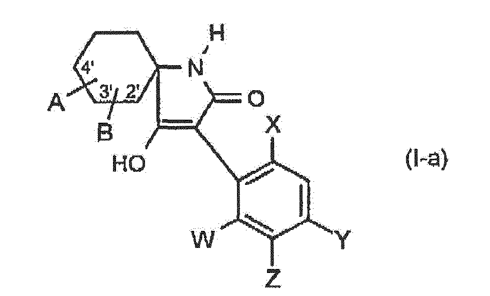 dans laquelle
A, B, W, X, Y et Z ont les significations données
plus haut,
on soumet à une condensation intramoléculaire des composés de formule (II) 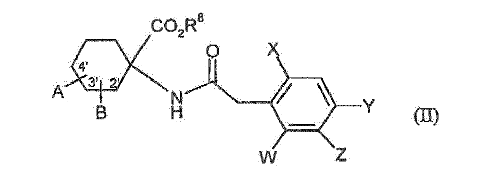 dans laquelle
A, B, W, X, Y et Z ont les significations données plus haut,
et
R⁸ représente un groupe alkyle,
en présence d'un diluant et en présence d'une base,
(B) de composés de formule (I-b), 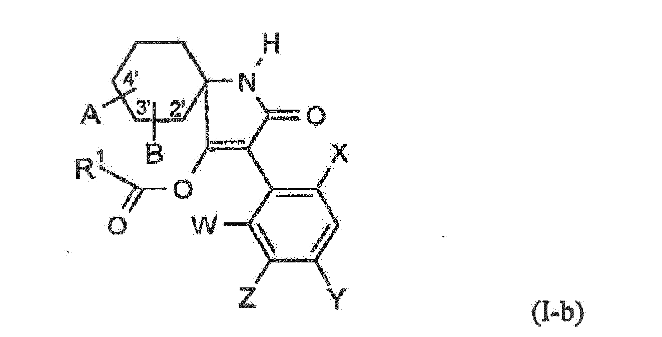 dans laquelle R¹, A, B, W, X, Y et Z ont les significations données plus haut, on fait réagir des composés de formule (I-a) indiquée plus haut, dans laquelle A, B, W, X, Y et Z ont les significations indiquées plus haut, respectivement
α) avec des composés de formule (III) 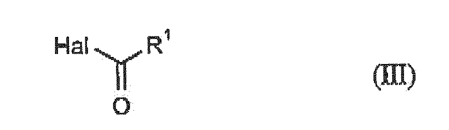 dans laquelle
R¹ a la signification indiquée plus haut et
Hal représente un halogène
ou
β) avec des anhydrides d'acides carboxyliques de formule (IV)
R¹-CO-O-CO-R¹ (IV)
dans laquelle
R¹ a la signification indiquée plus haut,
éventuellement en présence d'un diluant et éventuellement en présence d'un capteur d'acide ;
(C) de composés de formule (I-c), 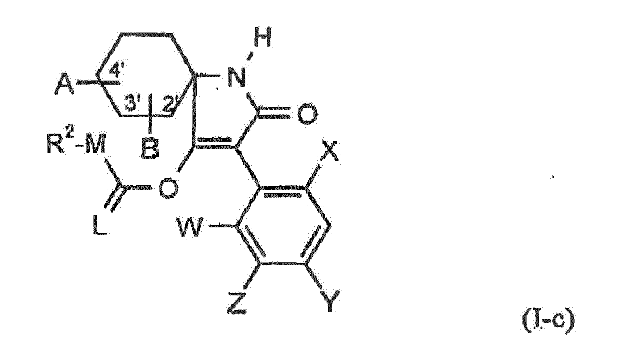 dans laquelle R², A, B, M, W, X, Y et Z ont les significations indiquées plus haut et L représente un atome d'oxygène, on fait réagir des composés de formule (I-a) indiquée plus haut, dans laquelle A, B, W, X, Y et Z ont les significations indiquées plus haut, respectivement
avec des esters d'acide chloroformique ou des thioesters d'acide chloroformique de formule (V)
R²-M-CO-C1 (V)
dans laquelle
R² et M ont les significations indiquées plus haut,
éventuellement en présence d'un diluant et éventuellement en présence d'un capteur d'acide ;
(D) de composés de formule (I-c) indiquée plus haut, dans laquelle R², A, B, M, X, Y et Z ont les significations indiquées plus haut et L représente un atome de soufre,
on fait réagir des composés de formule (I-a) indiquée plus haut, dans laquelle A, B, W, X, Y et Z ont les significations indiquées plus haut, respectivement
avec des esters d'acide chloromonothioformique ou des esters d'acide chlorodithioformique de formule (VI) 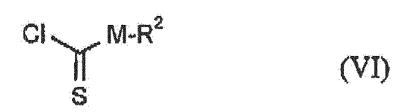 dans laquelle
M et R² ont les significations indiquées plus haut,
éventuellement en présence d'un diluant et éventuellement en présence d'un capteur d'acide ;
(E) de composés de formule (I-d), 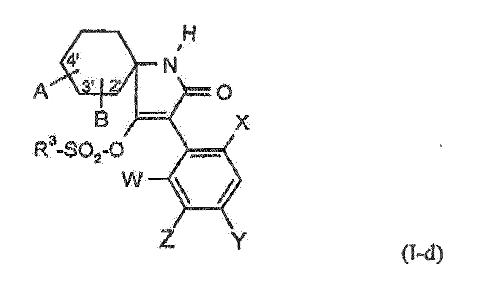 dans laquelle R³, A, B, W, X, Y et Z ont les significations indiquées plus haut, on fait réagir des composés de formule (I-a) indiquée plus haut, dans laquelle A, B, W, X, Y et Z ont les significations indiquées plus haut, chaque fois
avec des chlorures de sulfonyle de formule (VII)
R³-SO₂-Cl (VII)
dans laquelle
R³ a la signification indiquée plus haut,
éventuellement en présence d'un diluant et éventuellement en présence d'un capteur d'acide,
(F) de composés de formule (I-e) 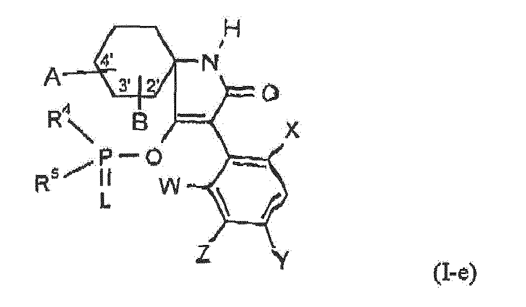 dans laquelle L, R⁴, R⁵, A, B, W, X, Y et Z ont les significations indiquées plus haut, on fait réagir des composés de formule (I-a) indiquée plus haut, dans laquelle A, B, W, X, Y et Z ont les significations indiquées plus haut, chaque fois
avec des composés phosphorés de formule (VIII) 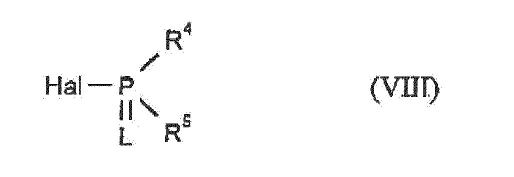 dans laquelle
L, R⁴ et R⁵ ont les significations indiquées plus
haut et
Hal représente un halogène,
éventuellement en présence d'un diluant et éventuellement en présence d'un capteur d'acide,
(G) de composés de formule (I-f), 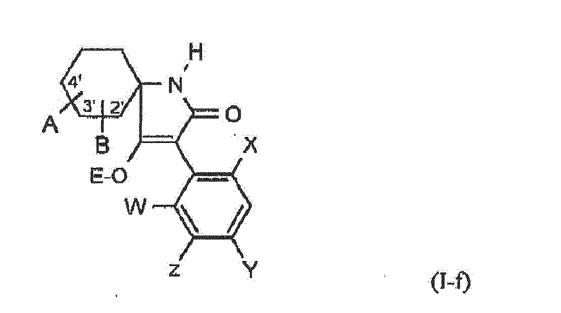 dans laquelle E, A, B, W, X, Y et Z ont les significations indiquées plus haut, on fait réagir des composés de formule (I-a), dans laquelle A, B, W, X, Y et Z ont les significations indiquées plus haut, respectivement
avec des composés contenant un métal ou des amines de formules (IX) ou (X)
Me(OR¹⁰)ₜ (IX) 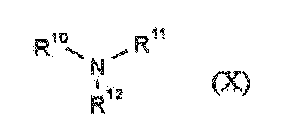 dans lesquelles
Me représente un métal mono- ou divalent,
t représente le nombre 1 ou 2 et
R¹⁰, R¹¹, R¹² représentent chacun indépendamment un atome d'hydrogène ou un groupe alkyle,
éventuellement en présence d'un diluant,
(H) de composés de formule (I-g), 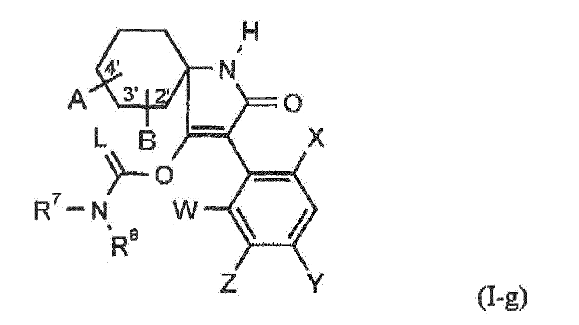 dans laquelle L, R⁶, R⁷, A, B, W, X, Y et Z ont les significations indiquées plus haut, on fait réagir des composés de formule (I-a) indiquée plus haut, dans laquelle A, B, W, X, Y et Z ont les significations données plus haut, respectivement
α) avec des isocyanates ou des isothiocyanates de formule (XI)
R⁶-N=C=L (XI)
dans laquelle
R⁶ et L ont les significations indiquées plus haut,
éventuellement en présence d'un diluant et éventuellement en présence d'un catalyseur ou
β) avec des chlorures d'acide carbamique ou des chlorures d'acide thiocarbamique de formule (XII) 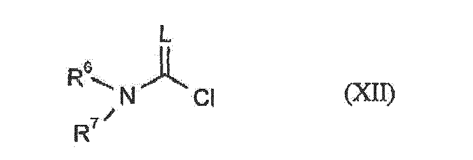 dans laquelle
L, R⁶ et R⁷ ont les significations indiquées plus haut,
éventuellement en présence d'un diluant et éventuellement en présence d'un capteur d'acide,
(Iα) on combine des composés de formules (I-a) à (I-g) indiquées plus haut, dans lesquelles A, B, G, W, X, Y et Z ont les significations indiquées plus haut, des composés de formules (I-a') à (I-g'), 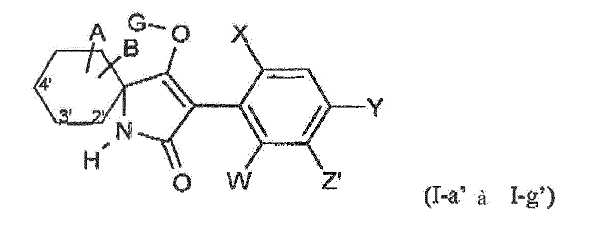 dans lesquelles A, B, G, W, X et Y ont les significations indiquées plus haut et Z' représente de préférence un atome de brome ou d'iode
et
(Iβ) on combine des composés de formules (I-a) à (I-g) indiquées plus haut, dans lesquelles A, B, G, W, X, Y et Z ont les significations indiquées plus haut, des composés de formules (I-a") à (I-g"), 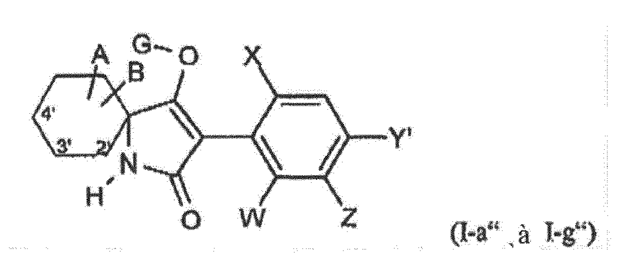 dans lesquelles A, B, G, W, X et Z ont les significations indiquées plus haut et Y' représente de préférence un atome de brome ou d'iode,
avec des dérivés (hétéro)aryle conjugables, par exemple des acides phénylboroniques de formules (XIIIα) et (XIIIβ) 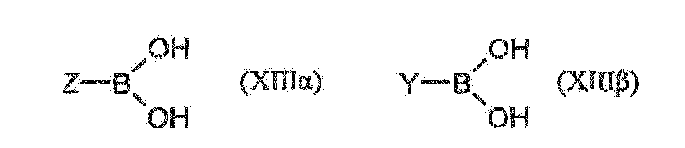 ou leurs esters, en présence d'un solvant, en présence d'un catalyseur et en présence d'une base.

7. Agent destiné à la lutte contre des ravageurs et/ou une végétation indésirable, **caractérisé par** une teneur en au moins un composé de formule (I) selon la revendication 1.

8. Procédé pour la lutte contre des ravageurs animaux et/ou une végétation indésirable, **caractérisé en ce qu'**on fait agir sur les ravageurs, la végétation indésirable et/ou leur habitat des composés de formule (I) selon la revendication 1.

9. Utilisation de composés de formule (I) selon la revendication 1, pour la lutte contre des ravageurs animaux et/ou une végétation indésirable.

10. Procédé pour la préparation d'agents destinés à la lutte contre des ravageurs et/ou une végétation indésirable, **caractérisé en ce qu'**on mélange des composés de formule (I) selon la revendication 1 avec des excipients et/ou des substances tensioactives.

11. Utilisation de composés de formule (I) selon la revendication 1, pour la préparation d'agents destinés à la lutte contre des ravageurs et/ou une végétation indésirable.

12. Agent ayant une teneur efficace en une association de substances actives, comprenant comme composants
a') au moins un composé de formule (I) selon la revendication 1, dans laquelle A, B, G, W, X, Y et Z ont la signification indiquée plus haut
et
b') au moins un composé améliorant la tolérance par des plantes cultivées, choisi parmi les groupes suivants de composés : S1, S2, S3, S4, S5, S6, S7, S8, S9, S10, S11, S12, S13, S14.

13. Agents selon la revendication 12, dans lesquels le composé améliorant la tolérance par des plantes cultivées est le cloquintocet-mexyl.

14. Agents selon la revendication 12, dans lesquels le composé améliorant la tolérance par des plantes cultivées est le méfenpyr-diéthyl.

15. Procédé pour la lutte contre une végétation indésirable, **caractérisé en ce qu'**on fait agir sur la plantes ou les plantes ou leur environnement un agent selon la revendication 12.

16. Utilisation d'un agent selon la revendication 12, pour la lutte contre une végétation indésirable.

17. Procédé pour la lutte contre une végétation indésirable, **caractérisé en ce qu'**on fait agir sur les plantes ou leur environnement, en une succession rapprochée dans le temps, un composé de formule (I) selon la revendication 1 et le composé améliorant la tolérance par des plantes cultivées selon la revendication 12.

18. Composés de formule (II) 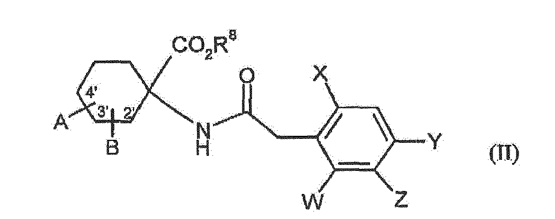 dans laquelle
A, B, W, X, Y, Z et R⁸ ont les significations indiquées plus haut.

19. Composés de formule (XIV) 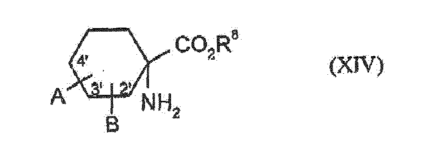 dans laquelle A, B et R⁸ ont les significations indiquées plus haut.

20. Composés de formule (XVI) 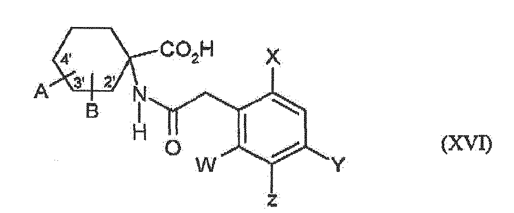 dans laquelle
A, B, W, X, Y et Z ont les significations indiquées plus haut.

21. Composés de formule (XVII) 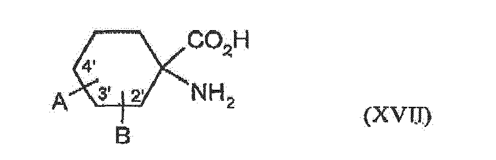 dans laquelle
A et B ont les significations indiquées plus haut.

22. Composés de formule (XVIII) 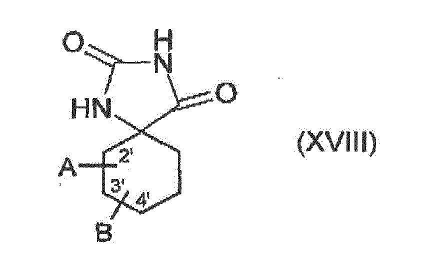 dans laquelle A et B ont les significations indiquées plus haut.

23. Composés de formule (XX) 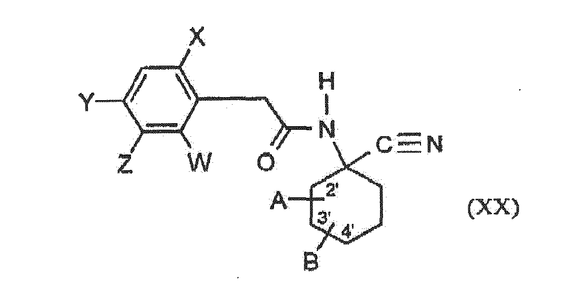 dans laquelle
A, B, W, X, Y et Z ont les significations indiquées plus haut.

24. Composés de formule (XIX) 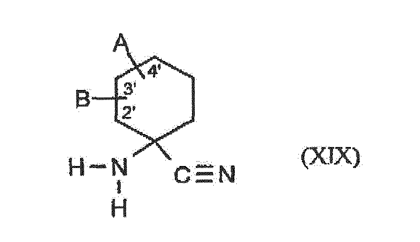 dans laquelle
A et B ont les significations indiquées plus haut.

25. Composition comprenant
- au moins un composé de formule (I) selon la revendication 1 ou un agent selon la revendication 12 et
- au moins un sel de formule (III') 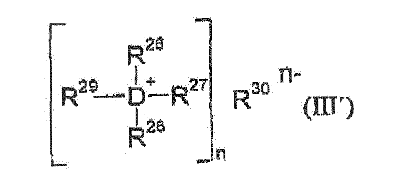 dans laquelle
D représente un atome d'azote ou de phosphore,
R²⁶, R²⁷, R²⁸ et R²⁹ représentent indépendamment les uns des autres un atome d'hydrogène ou un groupe alkyle en C₁-C₈ chaque fois éventuellement substitué ou un groupe alkylène en C₁-C₈ une ou plusieurs fois insaturé, éventuellement substitué, les substituants pouvant être choisis parmi halogéno, nitro et cyano,
n représente 1, 2, 3 ou 4,
R³⁰ représente un anion organique ou inorganique.

26. Composition selon la revendication 25, **caractérisée en ce qu'**elle contient au moins un agent favorisant la pénétration.

27. Procédé pour augmenter l'action de pesticides et/ou d'herbicides contenant une substance active de formule (I) selon la revendication 1 ou un agent selon la revendication 12, **caractérisé en ce qu'**on prépare le produit prêt à l'emploi (bouillie pulvérisable) en utilisant un sel de formule (III') selon la revendication 25.

28. Procédé selon la revendication 27, **caractérisé en ce qu'**on prépare la bouillie pulvérisable en utilisant un agent favorisant la pénétration.
